# EUROPEAN PATENT APPLICATION

(11) **EP 4 431 110 A2**
(43) Date of publication of application: **18.09.2024**
(21) Application number: 24187471.8
(22) Date of filing: 19.01.2020
(51) Int. Cl.: A61K 39/00

(54) **ENHANCED RECEPTOR FOR IMPROVING IMMUNE CELL FUNCTION**

(62) Divisional of application: 20914237.1
(71) Applicant: Chineo Medical Technology Co., Ltd., Beijing 101111 (CN)
(72) Inventor: GU, Weiyue, BEIJING, 101111 (CN)
(74) Representative: Lavoix

(57) **Abstract**

The present invention provides an enhanced receptor for improving the function of an immune cell, and a composition and a cell related thereto. The enhanced receptor is a transmembrane protein, which is a fusion protein consisting of an extracellular domain and an intracellular domain, wherein the extracellular domain is capable of binding to a target cell and activating the signaling function of the intracellular domain, thereby increasing the activation level of the immune cell, and overcoming the inhibition of the target cell microenvironment on the immune cell, thus enhancing the effect of immunotherapy.

## Description

### Technical Field

The present invention belongs to the technical field of biomedicine, in particular to the field of immune cell therapy.

### Background Art

Traditional immunotherapies have limitations, which are mainly reflected in that the activation levels of immune cells are insufficient, and the microenvironment of target cells (such as cancer cells) can inhibit immune cells, thereby affecting the effects of immune cells in killing target cells. The existing monoclonal antibody drugs against immune checkpoints can abolish the inhibitory effects of the microenvironment to a certain extent, but their limitations are also obvious. Firstly, such antibodies indiscriminately abolish the inhibition on all T cells, including T cells that attack their own hosts, which will bring hidden dangers of autoimmune diseases. Secondly, even if the inhibition is abolished, the functions of T cells will only return to the "normal" levels, which are often not enough to beat advanced cancers.

### Summary of the Invention

In view of the above background, there is an urgent need for methods, systems, and products to improve the function of immune cells, and the present invention meets this need. The present invention provides an enhanced receptor for an immune cell, and a composition and a cell related thereto. The receptor is a fusion protein consisting of an extracellular domain (ECD) and an intracellular domain (ICD), wherein the extracellular domain is capable of binding to a target cell and activating the signaling function of the intracellular domain, thereby increasing the activation level of the immune cell, and overcoming the inhibition of the target cell microenvironment on the immune cell, thus enhancing the effect of immunotherapy.

The practice of some methods disclosed herein employ, unless otherwise indicated, conventional techniques of immunology, biochemistry, chemistry, molecular biology, microbiology, cell biology, genomics and recombinant DNA, which are within the skill of the art. See for example Sambrook and Green, Molecular Cloning: A Laboratory Manual, 4th Edition (2012); the series Current Protocols in Molecular Biology (F. M. Ausubel, et al. eds.); the series Methods In Enzymology (Academic Press, Inc.), PCR 2: A Practical Approach (M.J. MacPherson, B.D. Hames and G.R. Taylor eds. (1995)), Harlow and Lane, eds. (1988) Antibodies, A Laboratory Manual, and Culture of Animal Cells: A Manual of Basic Technique and Specialized Applications, 6th Edition (R.I. Freshney, ed. (2010)).

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated, the term "about" meaning within an acceptable error range for the particular value should be assumed.

As used herein, a "cell" can generally refer to a biological cell. A cell can be the basic structural, functional and/or biological unit of an organism. A cell can originate from any organism having one or more cells. Some non-limiting examples include: a prokaryotic cell, a eukaryotic cell, a bacterial cell, an archaeal cell, a cell of a single-cell eukaryotic organism, a protozoa cell, a cell from a plant (e.g., cells from plant crops, fruits, vegetables, grains, soy bean, corn, maize, wheat, seeds, tomatoes, rice, cassava, sugarcane, pumpkin, hay, potatoes, cotton, cannabis, tobacco, flowering plants, conifers, gymnosperms, ferns, clubmosses, hornworts, fiverworts and mosses), an algal cell (e.g., Botryococcus braunii, Chlamydomonas reinhardtii, Nannochloropsis gaditana, Chlorella pyrenoidosa, Sargassum patens C. Agardh, etc.), seaweeds (e.g., kelp), a fungal cell (e.g., a yeast cell, and a cell from a mushroom), an animal cell, a cell from an invertebrate animal (e.g., fruit fly, cnidarian, echinoderm, nematode, etc.), a cell from a vertebrate animal (e.g., fish, amphibian, reptile, bird and mammal), a cell from a mammal (e.g., a pig, a cow, a goat, a sheep, a rodent, a rat, a mouse, a non-human primate, a human, etc.), and etcetera. Sometimes a cell is not originating from a natural organism (e.g., a cell can be a synthetically made, sometimes termed an artificial cell).

The term "antigen," as used herein, refers to a molecule or fragment thereof capable of being bound by a selective binding agent. As an example, an antigen can be a ligand that can be bound by a selective binding agent such as a receptor. As another example, an antigen can be an antigenic molecule that can be bound by a selective binding agent such as an immunological protein (e.g., an antibody). An antigen can also refer to a molecule or fragment thereof capable of being used in an animal to produce antibodies capable of binding to that antigen.

The term "neoantigen," as used herein, generally refers to a tumor-specific antigen arising from mutations in a gene. The resulting mutated proteins or fragments thereof can trigger an antitumor T cell response.

The term "gene," as used herein, refers to a nucleic acid (e.g., DNA, such as genomic DNA and cDNA) and its corresponding nucleotide sequence that encodes an RNA transcript. The term as used herein with reference to genomic DNA includes intervening, non-coding regions as well as regulatory regions, and can include 5' and 3' ends. In some uses, the term encompasses the transcribed sequences, including 5' and 3' untranslated regions (5'-UTR and 3'-UTR), exons and introns. In some genes, the transcribed region will contain "open reading frames" that encode polypeptides. In some uses of the term, a "gene" comprises only the coding sequence (e.g., an "open reading frame" or "coding region") necessary for encoding a polypeptide. In some cases, genes do not encode polypeptides, for example, ribosomal RNA genes (rRNA) and transfer RNA (tRNA) genes. In some cases, the term "gene" includes not only the transcribed sequences, but also includes non-transcribed regions, including upstream and downstream regulatory regions, enhancers and promoters. A gene can refer to an "endogenous gene" or a native gene in its natural location in the genome of an organism. A gene can refer to an "exogenous gene" or a non-native gene. A non-native gene can refer to a gene not normally found in the host organism but which is introduced into the host organism by gene transfer. A non-native gene can also refer to a gene not in its natural location in the genome of an organism. A non-native gene can also refer to a naturally occurring nucleic acid or polypeptide sequence that comprises mutations, insertions and/or deletions (e.g., non-native sequences).

The term "antibody," as used herein, refers to a proteinaceous binding molecule with immunoglobulin-like functions. The term antibody includes antibodies (e.g., monoclonal and polyclonal antibodies), as well as derivatives, variants and fragments thereof. Antibodies include, but are not limited to, immunoglobulins (Ig's) of different classes (i.e., IgA, IgG, IgM, IgD and IgE) and subclasses (such as IgG1, IgG2, etc.). A derivative, variant or fragment thereof can refer to a functional derivative or fragment which retains the binding specificity (e.g., complete and/or partial) of the corresponding antibody. Antigen-binding fragments include Fab, Fab', F(ab')2, variable fragments (Fvs), single chain variable fragments (scFvs), minibodies, diabodies, and single-domain antibodies ("sdAb" or "nanobodies" or "camelids"). The term antibody includes antibodies and antigen-binding fragments of antibodies that have been optimized, engineered or chemically conjugated. Examples of antibodies that have been optimized include affinity-matured antibodies. Examples of antibodies that have been engineered include Fc optimized antibodies (e.g., antibodies optimized in the fragment crystallizable region) and multispecific antibodies (e.g., bispecific antibodies).

The term "gene," as used herein, refers to a nucleic acid (e.g., DNA, such as genomic DNA and cDNA) and its corresponding nucleotide sequence that encodes an RNA transcript. The term as used herein with reference to genomic DNA includes intervening, non-coding regions as well as regulatory regions, and can include 5' and 3' ends. In some uses, the term encompasses the transcribed sequences, including 5' and 3' untranslated regions (5'-UTR and 3'-UTR), exons and introns. In some genes, the transcribed region will contain "open reading frames" that encode polypeptides. In some uses of the term, a "gene" comprises only the coding sequence (e.g., an "open reading frame" or "coding region") necessary for encoding a polypeptide. In some cases, genes do not encode polypeptides, for example, ribosomal RNA genes (rRNA) and transfer RNA (tRNA) genes. In some cases, the term "gene" includes not only the transcribed sequences, but also includes non-transcribed regions, including upstream and downstream regulatory regions, enhancers and promoters. A gene can refer to an "endogenous gene" or a native gene in its natural location in the genome of an organism. A gene can refer to an "exogenous gene" or a non-native gene. A non-native gene can refer to a gene not normally found in the host organism but which is introduced into the host organism by gene transfer. A non-native gene can also refer to a gene not in its natural location in the genome of an organism. A non-native gene can also refer to a naturally occurring nucleic acid or polypeptide sequence that comprises mutations, insertions and/or deletions (e.g., non-native sequences).

The term "antibody," as used herein, refers to a proteinaceous binding molecule with immunoglobulin-like functions. The term antibody includes antibodies (e.g., monoclonal and polyclonal antibodies), as well as derivatives, variants and fragments thereof. Antibodies include, but are not limited to, immunoglobulins (Ig's) of different classes (i.e., IgA, IgG, IgM, IgD and IgE) and subclasses (such as IgG1, IgG2, etc.). A derivative, variant or fragment thereof can refer to a functional derivative or fragment which retains the binding specificity (e.g., complete and/or partial) of the corresponding antibody. Antigen-binding fragments include Fab, Fab', F(ab')2, variable fragments (Fvs), single chain variable fragments (scFvs), minibodies, diabodies, and single-domain antibodies ("sdAb" or "nanobodies" or "camelids"). The term antibody includes antibodies and antigen-binding fragments of antibodies that have been optimized, engineered or chemically conjugated. Examples of antibodies that have been optimized include affinity-matured antibodies. Examples of antibodies that have been engineered include Fc optimized antibodies (e.g., antibodies optimized in the fragment crystallizable region) and multispecific antibodies (e.g., bispecific antibodies).

The term "nucleotide," as used herein, generally refers to a base-sugar-phosphate combination. A nucleotide can include synthetic nucleotides. A nucleotide can include synthetic nucleotide analogs. Nucleotides can be monomeric units of a nucleic acid sequence (e.g., deoxyribonucleic acid (DNA) and ribonucleic acid (RNA)). The term nucleotide can include ribonucleoside triphosphates adenosine triphosphate (ATP), uridine triphosphate (UTP), cytosine triphosphate (CTP), guanosine triphosphate (GTP) and deoxyribonucleoside triphosphates such as dATP, dCTP, dITP, dUTP, dGTP, dTTP, or derivatives thereof. Such derivatives can include, for example, [αS]dATP, 7-deaza-dGTP and 7-deaza-dATP, and nucleotide derivatives that confer nuclease resistance on the nucleic acid molecule containing them. The term nucleotide as used herein can refer to dideoxyribonucleoside triphosphates (ddNTPs) and their derivatives. Illustrative examples of dideoxyribonucleoside triphosphates can include, but are not limited to, ddATP, ddCTP, ddGTP, ddITP and ddTTP. A nucleotide can be unlabeled or detectably labeled by well-known techniques. Labeling can also be carried out with quantum dots. Detectable labels can include, for example, radioactive isotopes, fluorescent labels, chemiluminescent labels, bioluminescent labels and enzyme labels.

The terms "polynucleotide," "oligonucleotide," and "nucleic acid" are used interchangeably to refer to a polymeric form of nucleotides of any length, either deoxyribonucleotides or ribonucleotides, or analogs thereof, either in single-, double- or multi-stranded form. A polynucleotide can be exogenous or endogenous to a cell. A polynucleotide can exist in a cell-free environment. A polynucleotide can be a gene or fragment thereof. A polynucleotide can be DNA. A polynucleotide can be RNA. A polynucleotide can have any three dimensional structure, and can perform any function, known or unknown. A polynucleotide can comprise one or more analogs (e.g., altered backbone, sugar or nucleobase).

The term "expression" refers to one or more processes by which a polynucleotide is transcribed from a DNA template (such as into an mRNA or other RNA transcripts) and/or by which a transcribed mRNA is subsequently translated into peptides, polypeptides or proteins. Transcripts and encoded polypeptides can be collectively referred to as "gene products." If the polynucleotide is derived from genomic DNA, expression can include splicing of the mRNA in a eukaryotic cell. "Up-regulated," with reference to expression, generally refers to an increased expression level of a polynucleotide (e.g., RNA such as mRNA) and/or polypeptide sequence relative to its expression level in a wild-type state, while "down-regulated" generally refers to a decreased expression level of a polynucleotide (e.g., RNA such as mRNA) and/or polypeptide sequence relative to its expression in a wild-type state.

The term "regulating" with reference to expression or activity, as used herein, refers to altering the level of expression or activity. Regulation can occur at the transcription level and/or translation level.

The terms "peptide," "polypeptide," and "protein" are used interchangeably herein to refer to a polymer of at least two amino acid residues joined by peptide bond(s). The terms do not connote a specific length of polymer, nor are they intended to imply or distinguish whether the peptide is produced using recombinant techniques, chemical or enzymatic synthesis, or is naturally occurring. The terms apply to naturally occurring amino acid polymers as well as amino acid polymers comprising at least one modified amino acid. In some cases, the polymers can be interrupted by non-amino acids. The terms include amino acid chains of any length, including full length proteins, and proteins with or without secondary and/or tertiary structure (e.g., domains). The terms also encompass amino acid polymers that have been modified, for example, by disulfide bond formation, glycosylation, lipidation, acetylation, phosphorylation, oxidation, and any other manipulations such as conjugation with a labeling component. The terms "amino acid" and "amino acids," as used herein, generally refer to natural and non-natural amino acids, including, but not limited to, modified amino acids and amino acid analogs. Modified amino acids can include natural amino acids and non-natural amino acids, which have been chemically modified to include a group or a chemical moiety not naturally present on the amino acid. Amino acid analogs can refer to amino acid derivatives. The term "amino acid" includes both D-amino acids and L-amino acids.

The terms "derivative," "variant," and "fragment," when used herein with reference to a polypeptide, refer to a polypeptide related to a wild type polypeptide, for example either by amino acid sequence, structure (e.g., secondary and/or tertiary), activity (e.g., enzymatic activity) and/or function. Derivatives, variants and fragments of a polypeptide can comprise one or more amino acid variations (e.g., mutations, insertions and deletions), truncations, modifications, or combinations thereof compared to a wild type polypeptide.

As used herein, "fusion" can refer to a protein and/or nucleic acid comprising one or more non-native sequences (e.g., moieties). A fusion can comprise one or more identical non-native sequences. A fusion can comprise one or more different non-native sequences. A fusion can be a chimera. A fusion can comprise a nucleic acid affinity tag. A fusion can comprise a barcode. A fusion can comprise a peptide affinity tag. A fusion can provide for subcellular localization of the site-directed polypeptide (e.g., a nuclear localization signal (NLS) for targeting to the nucleus, a mitochondrial localization signal for targeting to the mitochondria, a chloroplast localization signal for targeting to a chloroplast, an endoplasmic reticulum (ER) retention signal, etc.). A fusion can provide a non-native sequence (e.g., affinity tag) that can be used to track or purify. A fusion can be a small molecule such as biotin or a dye such as Alexa fluor dyes, Cyanine3 dye and Cyanine5 dye.

The phrase "artificial TCR", as used herein, which can be understood as "exogenous T cell receptor (TCR) complex", refers to a TCR complex in which one or more chains of the TCR are introduced into the genome of an immune cell that may or may not endogenously express the TCR. In some cases, an exogenous TCR complex can refer to a TCR complex in which one or more chains of an endogenous TCR complex have one or more mutated sequences, for example at either the nucleic acid or amino acid level. Expression of an exogenous TCR on an immune cell can confer binding specificity for an epitope or antigen (e.g., an epitope or antigen preferentially present on the surface of a cancer cell or other disease-causing cells or particles). An exogenous TCR complex can comprise a TCR-α chain, a TCR-β chain, a CD3-γ chain, a CD3-δ chain, a CD3-ζ chain, or any combination thereof, which is introduced into the genome. In some cases, the chain introduced into the genome may replace the endogenous chain.

The terms "subject," "individual," and "patient" are used interchangeably herein to refer to a vertebrate, preferably a mammal such as a human. Mammals include, but are not limited to, murines, simians, humans, farm animals, sport animals and pets. Tissues, cells and their progenies of a biological entity obtained *in vivo* or cultured *in vitro* are also encompassed.

The terms "treatment" and "treating," as used herein, refer to an approach for obtaining beneficial or desired results including but not limited to a therapeutic benefit and/or a prophylactic benefit. For example, treatment can comprise administering a system or population of cells disclosed herein. By therapeutic benefit is meant any therapeutically relevant improvement in or effect on one or more diseases, conditions or symptoms under treatment. For prophylactic benefit, a composition can be administered to a subject at risk of developing a particular disease, condition or symptom, or to a subject reporting one or more physiological symptoms of a disease, even though the disease, condition or symptom may not have yet been manifested.

The term "effective amount" or "therapeutically effective amount" refers to the quantity of a composition, for example a composition comprising immune cells such as lymphocytes (e.g., T lymphocytes and/or NK cells) of the present disclosure, that is sufficient to result in a desired activity upon administration to a subject in need thereof. Within the context of the present disclosure, the term "therapeutically effective" refers to that quantity of a composition that is sufficient to delay the manifestation, arrest the progression, relieve or alleviate at least one symptom of a disorder treated by the methods of the present disclosure.

The term "genetic profile," as used herein, refers to information about specific genes, including variations and gene expression in an individual or in a certain type of tissue. A genetic profile can be used for neoantigen selection. The term "somatic mutation profile," as used herein, refers to information about specific genes associated with somatic mutations, including but not limited to specific genes resulted from somatic mutations. A somatic mutation profile can be used for neoantigen selection.

In an aspect, the present disclosure provides a modified tumor-infiltrating lymphocyte (TIL) that specifically binds to a tumor-associated antigen, including but not limited to a neoantigen. The modified TIL can comprise a chimeric stimulating molecule. The chimeric stimulating molecule can comprise a polypeptide extracellular domain (PED) that binds to the neoantigen. The PED can be fused to an intracellular domain (ICD) of a costimulatory molecule that mediates an immune cell activation signal. Binding of the chimeric stimulating molecule to the neoantigen can yield the immune cell activation signal in the modified TIL. In some embodiments, the PED can be an extracellular domain of a surface protein of an unmodified TIL. In some embodiments, examples of the PED include antibodies, as well as derivatives, variants, and fragments thereof.

In an aspect, the present disclosure provides a modified tumor-infiltrating lymphocyte (TIL) that specifically binds to a neoantigen, the modified TIL comprising an enhanced receptor. The enhanced receptor can comprise an extracellular domain (ECD) of a protein. The ECD can be fused to an intracellular domain (ICD) of a costimulatory molecule that mediates an immune cell activation signal. Binding of the enhanced receptor to its ligand can yield the immune cell activation signal in the modified TIL instead of an immune cell inactivation signal.

The TIL can be any cell obtained from a tumor. For example, the TIL can be a cell that has migrated to a tumor. The TIL can be a cell that has infiltrated a tumor. In some embodiments, the TIL is a white blood cell that has migrated into a tumor from the bloodstream of a subject. The TIL can be, for example, a T cell, B cell, monocyte or natural killer (NK) cell. In some cases, a modified TIL comprises a CD8+ cytotoxic T cell (lymphocyte), Th1 and Th17 CD4+ T cell, a natural killer cell, a dendritic cell, or an M1 macrophage. A population of immune cells comprising TILs can be a mixed population of cells. A population of TILs can comprise cells of different phenotypes, cells of different degrees of differentiation, cells of different lineages, or any combination thereof. TILs can generally be defined either biochemically, using cell surface markers, or functionally, by their ability to infiltrate tumors and effect treatment. TILs can be categorized based on expression one or more of the following biomarkers: CD4, CD8, TCRαβ, CD25, CD27, CD28, CD39, CD56, CD137, CCR7, CD45Ra, CD95, PD-1 and TIM-3. In some embodiments, the modified TIL expresses at least one of PD-1, CD39, CD137 and TIM-3. In some cases, TILs can be functionally defined by their ability to infiltrate solid tumors upon reintroduction into a patient. In some cases, the modified TIL comprises a "primary TIL," referring to a TIL that is obtained from a patient tissue sample. In some cases, the modified TIL comprises a "secondary TIL," referring to a TIL that has been expanded or proliferated. A TIL can exhibit specific binding to a neoantigen. In some cases, the TCR complex of the TIL confers the antigen binding specificity (e.g., neoantigen binding).

In an aspect, the present disclosure provides a modified T cell that specifically binds to a neoantigen, the modified T cell comprising an enhanced receptor. The enhanced receptor can comprise an extracellular domain (ECD) of a protein. The ECD can be fused to an intracellular domain (ICD) of a costimulatory molecule that mediates an immune cell activation signal. Binding of the enhanced receptor to its ligand can yield the immune cell activation signal in the modified T cell instead of an immune cell inactivation signal.

The modified T cell can comprise a T cell receptor (TCR) complex which exhibits specific binding to the neoantigen. In some embodiments, the TCR complex is an endogenous TCR complex. In some embodiments, the TCR is an exogenous TCR complex. The TCR complex, e.g., endogenous or exogenous, of the modified immune cell can confer the antigen binding specificity (e.g., neoantigen binding) of the immune cell. In some embodiments, the present disclosure provides a modified T cell comprising an endogenous TCR complex that specifically binds to a neoantigen, said modified T cell comprising a chimeric stimulating molecule, wherein said chimeric stimulating molecule comprises: a polypeptide extracellular domain (PED) that binds to a membrane protein on a cell including but not limited to a tumor cell, wherein said PED is fused to an intracellular domain (ICD) of a costimulatory molecule that mediates an immune cell activation signal, wherein binding of said chimeric stimulating molecule to said membrane protein yields said immune cell activating signal in said modified T cell.

Binding of a modified immune cell, such as the modified T cell or the modified TIL provided herein, to a neoantigen can activate the immune cell. The enhanced receptor of the modified cell can be used to provide further control over immune cell activities, such as but not limited to, immune cell activation and expansion. Binding of the enhanced receptor to its ligand in the modified immune cell, such as a modified T cell or a modified TIL, can elicit an immune cell activation signal in the modified immune cell instead of an immune cell inactivation signal. Eliciting the immune cell activation signal in the modified immune cell instead of the immune cell inactivation signal may minimize an immune-suppressive effect in the immune cell. Minimizing an immune-suppressive effect in the immune cell can increase the effectiveness of the immune cell in an immune response, for example by increasing immune cell cytotoxicity against a target cell, such as a tumor cell.

The enhanced receptor can comprise an extracellular domain (ECD) of a protein that, in an unmodified immune cell, elicits an immune cell signal upon binding to its ligand, which can be an inactivation signal, an activating signal, or neither an activating signal nor an inactivation signal. The protein can be a signaling receptor, or any functional fragment, derivative or variant thereof. In some cases, the signaling receptor can be a membrane-bound receptor. A signaling receptor can, in response to ligand binding, induce one or more signaling pathways in a cell. In some cases, the signaling receptor can be a non-membrane-bound receptor. The enhanced receptor can comprise a fragment, for example an extracellular domain, of a receptor selected from a G-protein coupled receptor (GPCR); an integrin receptor; a cadherin receptor; a catalytic receptor (e.g., kinases); a death receptor; a checkpoint receptor; a cytokine receptor; a chemokine receptor; a growth factor receptor; a hormone receptor; and an immune receptor.

In some embodiments, the enhanced receptor comprises a fragment of an immune checkpoint receptor, which may be involved in regulation of the immune system. Non-limiting examples of such receptors include, but are not limited to, programmed cell death 1 (PD-1 or PD1), cytotoxic T-lymphocyte associated protein 4 (CTLA-4), B and T lymphocyte attenuator (BTLA), a killer immunoglobulin-like receptor (KIR), indoleamine 2,3-dioxygenase (IDO), lymphocyte activation gene-3 (LAG3), T cell immunoglobulin mucin 3 (TIM-3), T-cell immunoreceptor with Ig and ITIM domains (TIGIT), SIRPα and NKG2D.

In some embodiments, the enhanced receptor comprises at least an extracellular fragment of a TCR, which may be involved in recognizing a neoantigen of a target cell (e.g., a cancer cell antigen or a tumor antigen). In some examples, the enhanced receptor can comprise the extracellular variable regions of the TCRα and/or β chains.

The enhanced receptor may comprise an immune checkpoint receptor, or any derivative, variant or fragment thereof. The enhanced receptor can bind an antigen comprising any suitable immune checkpoint receptor ligand, or any derivative, variant or fragment thereof. Non-limiting examples of such ligands include, but are not limited to, B7-1, B7-H3, B7-H4, HVEM (Herpesvirus Entry Mediator), AP2M1, CD80, CD86, SHP-2, PPP2R5A, MHC (e.g., class I and class II), CD47, CD70, PD-L1 (or PDL1) and PD-L2. The regions where the enhanced receptor bind to such ligands can be natural receptors for such ligands or monoclonal antibodies to such ligands.

In some embodiments, the enhanced receptor comprises a fragment of a cytokine receptor. Cytokine receptors can serve a variety of functions, non-limiting examples of which include immune cell regulation and mediating inflammation. In some embodiments, the enhanced receptor comprises a cytokine receptor, for example a type I cytokine receptor or a type II cytokine receptor, or any derivative, variant or fragment thereof. In some embodiments, the enhanced receptor comprises an interleukin receptor (e.g., IL-2R, IL-3R, IL-4R, IL-5R, IL-6R, IL-7R, IL-9R, IL-11R, IL-12R, IL-13R, IL-15R, IL-21R, IL-23R, IL-27R and IL-31R), a colony stimulating factor receptor (e.g., erythropoietin receptor, CSF-1R, CSF-2R, GM-CSFR and G-CSFR), a hormone receptor/neuropeptide receptor (e.g., growth hormone receptor, prolactin receptor and leptin receptor), or any derivative, variant or fragment thereof. In some embodiments, the enhanced receptor comprises a type II cytokine receptor, or any derivative, variant or fragment thereof. In some embodiments, the enhanced receptor comprises an interferon receptor (e.g., IFNAR1, IFNAR2 and IFNGR), an interleukin receptor (e.g., IL-10R, IL-20R, IL-22R and IL-28R), a tissue factor receptor (also known as platelet tissue factor), or any derivative, variant or fragment thereof.

In some embodiments, the extracellular binding domain of the enhanced receptor can be any antibody or antibody fragment, and the antigen to which the antibody binds can be any membrane protein widely expressed by human cells, or a membrane protein expressed by tumor cells, including a class I RTK (e.g., the epidermal growth factor (EGF) receptor family including EGFR; and the ErbB family including ErbB-2, ErbB-3 and ErbB-4), a class II RTK (e.g., the insulin receptor family including INSR, IGF-1R and IRR), a class III RTK (e.g., the platelet-derived growth factor (PDGF) receptor family including PDGFR-α, PDGFR-β, CSF-1R, KIT/SCFR and FLK2/FLT3), a class IV RTK (e.g., the fibroblast growth factor (FGF) receptor family including FGFR-1, FGFR-2, FGFR-3 and FGFR-4), a class V RTK (e.g., the vascular endothelial growth factor (VEGF) receptor family including VEGFR1, VEGFR2 and VEGFR3), a class VI RTK (e.g., the hepatocyte growth factor (HGF) receptor family including hepatocyte growth factor receptor (HGFR/MET) and RON), a class VII RTK (e.g., the tropomyosin receptor kinase (Trk) receptor family including TRKA, TRKB and TRKC), a class VIII RTK (e.g., the ephrin (Eph) receptor family including EPHA1, EPHA2, EPHA3, EPHA4, EPHA5, EPHA6, EPHA7, EPHA8, EPHB1, EPHB2, EPHB3, EPHB4, EPHB5 and EPHB6), a class IX RTK (e.g., AXL receptor family such as AXL, MER and TRYO3), a class X RTK (e.g., LTK receptor family such as LTK and ALK), a class XI RTK (e.g., TIE receptor family such as TIE and TEK), a class XII RTK (e.g., ROR receptor family ROR1 and ROR2), a class XIII RTK (e.g., the discoidin domain receptor (DDR) family such as DDR1 and DDR2), a class XIV RTK (e.g., RET receptor family such as RET), a class XV RTK (e.g., KLG receptor family including PTK7), a class XVI RTK (e.g., RYK receptor family including Ryk), a class XVII RTK (e.g., MuSK receptor family such as MuSK), CD47, CD70, NKG2D, or any derivative, variant or fragment thereof.

In some embodiments, the enhanced receptor can comprise at least an extracellular region (e.g., ligand binding domain) of a catalytic receptor such as a receptor tyrosine kinase (RTK), or any derivative, variant or fragment thereof, or comprises fragments of the variable regions of antibodies with these fragments as antigens. In some embodiments, the enhanced receptor comprises a class I RTK (e.g., the epidermal growth factor (EGF) receptor family including EGFR; and the ErbB family including ErbB-2, ErbB-3 and ErbB-4), a class II RTK (e.g., the insulin receptor family including INSR, IGF-1R and IRR), a class III RTK (e.g., the platelet-derived growth factor (PDGF) receptor family including PDGFR-α, PDGFR-β, CSF-1R, KIT/SCFR and FLK2/FLT3), a class IV RTK (e.g., the fibroblast growth factor (FGF) receptor family including FGFR-1, FGFR-2, FGFR-3 and FGFR-4), a class V RTK (e.g., the vascular endothelial growth factor (VEGF) receptor family including VEGFR1, VEGFR2 and VEGFR3), a class VI RTK (e.g., the hepatocyte growth factor (HGF) receptor family including hepatocyte growth factor receptor (HGFR/MET) and RON), a class VII RTK (e.g., the tropomyosin receptor kinase (Trk) receptor family including TRKA, TRKB and TRKC), a class VIII RTK (e.g., the ephrin (Eph) receptor family including EPHA1, EPHA2, EPHA3, EPHA4, EPHA5, EPHA6, EPHA7, EPHA8, EPHB1, EPHB2, EPHB3, EPHB4, EPHB5 and EPHB6), a class IX RTK (e.g., AXL receptor family such as AXL, MER and TRYO3), a class X RTK (e.g., LTK receptor family such as LTK and ALK), a class XI RTK (e.g., TIE receptor family such as TIE and TEK), a class XII RTK (e.g., ROR receptor family ROR1 and ROR2), a class XIII RTK (e.g., the discoidin domain receptor (DDR) family such as DDR1 and DDR2), a class XIV RTK (e.g., RET receptor family such as RET), a class XV RTK (e.g., KLG receptor family including PTK7), a class XVI RTK (e.g., RYK receptor family including Ryk), a class XVII RTK (e.g., MuSK receptor family such as MuSK), CD47, CD70, NKG2D, or any derivative, variant or fragment thereof.

The enhanced receptor comprises RTK, or any derivative, variant or fragment thereof. The enhanced receptor can bind an antigen comprising any suitable RTK ligand, or any derivative, variant or fragment thereof, or comprises fragments of the variable regions of antibodies with these fragments as antigens. Non limiting examples of RTK ligands include growth factors, cytokines and hormones. Growth factors include, for example, members of the epidermal growth factor family (e.g., epidermal growth factor or EGF, heparin-binding EGF-like growth factor or HB-EGF, transforming growth factor-α or TGF-α, amphiregulin or AR, epiregulin or EPR, epigen, betacellulin or BTC, neuregulin-1 or NRG1, neuregulin-2 or NRG2, neuregulin-3 or NRG3, and neuregulin-4 or NRG4), the fibroblast growth factor family (e.g., FGF1, FGF2, FGF3, FGF4, FGF5, FGF6, FGF7, FGF8, FGF9, FGF10, FGF11, FGF12, FGF13, FGF14, FGF15/19, FGF16, FGF17, FGF18, FGF20, FGF21 and FGF23), the vascular endothelial growth factor family (e.g., VEGF-A, VEGF-B, VEGF-C, VEGF-D and PIGF), and the platelet-derived growth factor family (e.g., PDGFA, PDGFB, PDGFC and PDGFD). Hormones include, for example, members of the insulin/IGF/relaxin family (e.g., insulin, insulin-like growth factors, relaxin family peptides including relaxin1, relaxin2, relaxin3, Leydig cell-specific insulin-like peptide (gene INSL3), early placenta insulin-like peptide (FLIP) (gene INSL4), insulin-like peptide 5 (gene INSL5) and insulin-like peptide 6).

In some embodiments, the enhanced receptor comprises at least an extracellular region (e.g., ligand binding domain) of a catalytic receptor such as a receptor threonine/serine kinase (RTSK), or any derivative, variant or fragment thereof, or comprises fragments of the variable regions of antibodies with these fragments as antigens. The enhanced receptor can comprise a type I RTSK, type II RTSK, or any derivative, variant or fragment thereof. The enhanced receptor can comprise a type I receptor, or any derivative, variant or fragment thereof, selected from the group consisting of: ALK1 (ACVRL1), ALK2 (ACVR1A), ALK3 (BMPR1A), ALK4 (ACVR1B), ALK5 (TGFβR1), ALK6 (BMPR1B) and ALK7 (ACVR1C). The enhanced receptor can comprise a type II receptor, or any derivative, variant or fragment thereof, selected from the group consisting of: TGFβR2, BMPR2, ACVR2A, ACVR2B and AMHR2 (AMHR). In some embodiments, the enhanced receptor comprises a TGF-β receptor, or any derivative, variant or fragment thereof.

The enhanced receptor comprising RTSK, or any derivative, variant or fragment thereof, can bind an antigen comprising any suitable RTSK ligand, or any derivative, variant or fragment thereof, or comprises fragments of the variable regions of antibodies with these fragments as antigens.

The enhanced receptor can comprise an intracellular domain (ICD) of a costimulatory molecule that elicits an immune cell activating signal. The costimulatory molecule may bind a ligand. In some cases, the costimulatory molecule may be activated by a ligand responsive protein. In some embodiments, the costimulatory molecule can be used to regulate a proliferative and/or survival signal in the immune cell. In some embodiments, the ICD is an intracellular domain of a costimulatory molecule selected from an MHC class I protein, an MHC class II protein, a TNF receptor protein, an immunoglobulin-like protein, a cytokine receptor, an integrin, a signaling lymphocytic activation molecule (SLAM protein), an activating NK cell receptor, BTLA or a Toll ligand receptor. In some embodiments, the costimulatory molecule or costimulatory domain comprises a signaling domain of a molecule selected from the group consisting of: 2B4/CD244/SLAMF4, 4-1BB/TNFSF9/CD137, B7-1/CD80, B7-2/CD86, B7-H1/PD-L1, B7-H2, B7-H3, B7-H4, B7-H6, B7-H7, BAFF R/TNFRSF13C, BAFF/BLyS/TNFSF13B, BLAME/SLAMF8, BTLA/CD272, CD100 (SEMA4D), CD103, CD11a, CD11b, CD11c, CD11d, CD150, CD160 (BY55), CD18, CD19, CD2, CD200, CD229/SLAMF3, CD27 Ligand/TNFSF7, CD27/TNFRSF7, CD28, CD29, CD2F-10/SLAMF9, CD3, CD30 Ligand/TNFSF8, CD30/TNFRSF8, CD300a/LMIR1, CD4, CD40 Ligand/TNFSF5, CD40/TNFRSF5, CD48/SLAMF2, CD49a, CD49D, CD49f, CD5, CD53, CD58/LFA-3, CD69, CD7, CD8α, CD8β, CD82/Kai-1, CD84/SLAMF5, CD90/Thy1, CD96, CDS, CEACAM1, CRACC/SLAMF7, CRTAM, CTLA-4, DAP12, Dectin-1/CLEC7A, DNAM1 (CD226), DPPIV/CD26, DR3/TNFRSF25, EphB6, GADS, Gi24/VISTA/B7-H5, GITR Ligand/TNFSF18, GITR/TNFRSF18, HLA Class I, HLA-DR, HVEM/TNFRSF14, IA4, ICAM-1, ICOS/CD278, Ikaros, IL2Rβ, IL2Rγ, IL7Rα, IL-12R, Integrin α4/CD49d, Integrin α4β1, Integrin α4β7/LPAM-1, IPO-3, ITGA4, ITGA6, ITGAD, ITGAE, ITGAL, ITGAM, ITGAX, ITGB1, ITGB2, ITGB7, KIRDS2, LAG-3, LAT, LIGHT/TNFSF14, LTBR, Ly108, Ly9 (CD229), lymphocyte function associated antigen-1 (LFA-1), Lymphotoxin-α/TNF-β, NKG2C, NKG2D, NKp30, NKp44, NKp46, NKp80 (KLRF1), NTB-A/SLAMF6, OX40 Ligand/TNFSF4, OX40/TNFRSF4, PAG/Cbp, PD-1, PDCD6, PD-L2/B7-DC, PSGL1, RELT/TNFRSF19L, SELPLG (CD162), SLAM (SLAMF1), SLAM/CD150, SLAMF4 (CD244), SLAMF6 (NTB-A), SLAMF7, SLP-76, TACI/TNFRSF13B, TCL1A, TCL1B, TIM-1/KIM-1/HAVCR, TIM-4, TL1A/TNFSF15, TNF RII/TNFRSF1B, TNF-α, TRANCE/RANKL, TSLP, TSLP R, VLA1 and VLA-6.

The ECD and the ICD of the enhanced receptor can be joined by a transmembrane domain, for example by a transmembrane segment. In some embodiments, the transmembrane segment comprises a polypeptide. The transmembrane polypeptide can have any suitable polypeptide sequence. In some cases, the transmembrane polypeptide comprises a polypeptide sequence of a transmembrane portion of an endogenous or wild-type transmembrane protein. In some embodiments, the transmembrane polypeptide comprises a polypeptide sequence having at least 1 (e.g., at least 2, 3, 4, 5, 6, 7, 8, 9, 10 or more) amino acid substitutions, deletions and insertions compared to a transmembrane portion of an endogenous or wild-type transmembrane protein. In some embodiments, the transmembrane polypeptide comprises a non-natural polypeptide sequence, such as the sequence of a polypeptide linker. The polypeptide linker may be flexible or rigid. The polypeptide linker can be structured or unstructured. In some embodiments, the transmembrane polypeptide transmits a signal from the ECD to the ICD, for example a signal indicating ligand-binding. In some embodiments, the ECD comprises a transmembrane domain. In some embodiments, the ICD comprises a transmembrane domain.

In some embodiments, the ICD may mediate the generation of an immune cell activation signal (or called immune cell activating signal) in the immune cell. In some embodiments, the immune cell activation signal is mediated by an activation factor. The activation factor can be an immunomodulating molecule. The activation factor may bind, activate, or stimulate T cells or other immune cells to modulate their activity. In some embodiments, the activation factor can be secreted from the immune cell. The activation factor can be, for example, a soluble cytokine, a soluble chemokine, or a growth factor molecule. Non-limiting examples of activation factors which can mediate the immune cell activation include soluble cytokines, such as IL-1, IL-2, IL-6, IL-7, IL-8, IL-10, IL-12, IL-15, IL-21, tumor necrosis factor (TNF), transforming growth factor (TGF), interferon (IFN), or any functional fragment or variant thereof.

The immune cell activating signal can comprise or result in clonal expansion of the modified immune cell (e.g., modified TIL or modified T cell); cytokine release by the modified immune cell (e.g., modified TIL or modified T cell); cytotoxicity of the modified immune cell (e.g., modified TIL or modified T cell); proliferation of the modified immune cell (e.g., modified TIL or modified T cell); differentiation, dedifferentiation or transdifferentiation of the modified immune cell (e.g., modified TIL or modified T cell); movement and/or trafficking of the modified immune cell (e.g., modified TIL or modified T cell); exhaustion and/or reactivation of the modified immune cell (e.g., modified TIL or modified T cell); and release of other intercellular molecules, metabolites, chemical compounds, or combinations thereof by the modified immune cell (e.g., modified TIL or modified T cell).

In some embodiments, the immune cell activation signal comprises or results in clonal expansion of the immune cell. Clonal expansion can comprise the generation of daughter cells arising from the immune cell. The daughter cells resulting from clonal expansion can comprise the enhanced receptor. Clonal expansion of the modified immune cell can be greater than that of a comparable immune cell lacking the enhanced receptor. Clonal expansion of the modified immune cell can be about 5 fold to about 10 fold, about 10 fold to about 20 fold, about 20 fold to about 30 fold, about 30 fold to about 40 fold, about 40 fold to about 50 fold, about 50 fold to about 60 fold, about 60 fold to about 70 fold, about 70 fold to about 80 fold, about 80 fold to about 90 fold, about 90 fold to about 100 fold, about 100 fold to about 200 fold, about 200 fold to about 300 fold, about 300 fold to about 400 fold, about 400 fold to about 500 fold, about 500 fold to about 600 fold, or about 600 fold to about 700 fold greater than a comparable immune cell lacking the enhanced receptor. In some embodiments, determining clonal expansion can comprise quantifying a number of immune cells, for example with and without enhanced receptors and after ligand binding to the enhanced receptors. Quantifying a number of immune cells can be achieved by a variety of techniques, non-limiting examples of which include flow cytometry, Trypan Blue exclusion and hemocytometry.

In some embodiments, the immune cell activation signal comprises or results in cytokine release by the immune cell. In some embodiments, the immune cell activity comprises or results in the release of intercellular molecules, metabolites, chemical compounds or combinations thereof. Cytokine release by the modified immune cell can comprise the release of IL-1, IL-2, IL-4, IL-5, IL-6, IL-13, IL-17, IL-21, IL-22, IFNγ, TNFα, CSF, TGFβ, granzyme, etc. In some embodiments, cytokine release may be quantified using enzyme-linked immunosorbent assay (ELISA), flow cytometry, western blot, etc. Cytokine release by the modified immune cell can be greater than that of a comparable immune cell lacking the enhanced receptor. The modified immune cell provided herein can generate about 1 fold, 2 fold, 3 fold, 4 fold, 5 fold, 6 fold, 7 fold, 8 fold, 9 fold, 10 fold, 11 fold, 12 fold, 13 fold, 14 fold, 15 fold, 20 fold, 30 fold, 40 fold, 50 fold, 60 fold, 70 fold, 80 fold, 90 fold, 100 fold, 150 fold, 200 fold, 250 fold, or over 300 fold greater cytokine release as compared to a comparable immune cell lacking the enhanced receptor. The modified immune cell can exhibit increased cytokine secretion as compared to a comparable immune cell lacking the enhanced receptor (e.g., unmodified), when the enhanced receptor binds to its ligand and the modified immune cell binds to the neoantigen present on a target cell. In some embodiments, the cytokine secreted is IFNγ or IL-2. In some embodiments, cytokine release can be quantified *in vitro* or *in vivo.*

In some embodiments, the immune cell activation signal comprises or results in cytotoxicity of the immune cell. In some cases, cytotoxicity of the modified immune cell provided herein can be used for killing a target cell. An immune cell or population of immune cells expressing an enhanced receptor can induce death of a target cell. Killing of a target cell can be useful for a variety of applications, including, but not limited to, treating a disease or disorder in which a population of cells is desired to be eliminated or its proliferation is desired to be inhibited. Cytotoxicity can also refer to the release of cytotoxic cytokines, for example IFNγ or granzyme, by the immune cell. In some cases, the modified immune cell provided herein may have altered (i) release of cytotoxins such as perforin, granzyme and granulysin, and/or (ii) induction of apoptosis via Fas-Fas ligand interaction between the T cells and target cells. In some embodiments, cytotoxicity can be quantified by a cytotoxicity assay, including a co-culture assay, ELISPOT, chromium release cytotoxicity assay, etc. Cytotoxicity of the modified immune cell provided herein can be greater than that of a comparable immune cell lacking the enhanced receptor. The modified immune cell can exhibit increased cytotoxicity against a target cell as compared to a comparable immune cell lacking the enhanced receptor (e.g., unmodified), when the enhanced receptor binds to its ligand and the modified immune cell binds to the neoantigen present on the target cell. The modified immune cell of the present invention can be about 5%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, or 200% more cytotoxic to target cells as compared to a comparable immune cell lacking the enhanced receptor. The modified immune cell of the present invention can induce death of target cells that is at least 5%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, or 200% greater than that of a comparable immune cell lacking the switch molecule. In some embodiments, the immune cell provided herein can induce apoptosis in target cells displaying target epitopes (e.g., neoantigens) on their surface. In some embodiments, cytotoxicity can be determined *in vitro* or *in vivo.* In some embodiments, determining cytotoxicity can comprise determining a level of disease after administration of the modified immune cells provided herein as compared to a level of disease prior to the administration. In some embodiments, determining cytotoxicity can comprise determining a level of disease after administration of the modified immune cells provided herein and a level of disease after administration of comparable immune cells lacking the enhanced receptor.

In some embodiments, the immune cell activation signal comprises or results in proliferation of the immune cell. Proliferation of the immune cell can refer to expansion of the immune cell. Proliferation of the immune cell can refer to phenotypic changes of the immune cell. Proliferation of the modified immune cell of the present disclosure can be greater than that of a comparable immune cell lacking the enhanced receptor. Proliferation of the modified immune cell provided herein can be about 5 fold to about 10 fold, about 10 fold to about 20 fold, about 20 fold to about 30 fold, about 30 fold to about 40 fold, about 40 fold to about 50 fold, about 50 fold to about 60 fold, about 60 fold to about 70 fold, about 70 fold to about 80 fold, about 80 fold to about 90 fold, about 90 fold to about 100 fold, about 100 fold to about 200 fold, about 200 fold to about 300 fold, about 300 fold to about 400 fold, about 400 fold to about 500 fold, about 500 fold to about 600 fold, or about 600 fold to about 700 fold greater than the proliferation of a comparable immune cell lacking the enhanced receptor. In some embodiments, proliferation can be determined by quantifying a number of immune cells. Quantifying a number of immune cells can comprise flow cytometry, Trypan Blue exclusion and/or hemocytometry. Proliferation can also be determined by phenotypic analysis of the immune cells.

In some embodiments, the immune cell activation signal can comprise or result in differentiation, dedifferentiation, or transdifferentiation of the immune cell. Differentiation, dedifferentiation, or transdifferentation of the immune cell can be determined by evaluating phenotypic expression of markers of differentiation, dedifferentiation, or transdifferentation on the cell surface by flow cytometry. In some embodiments, the modified immune cell provided herein has increased differentiation ability as compared to a comparable immune cell lacking the enhanced receptor. In some embodiments, the modified immune cell provided herein has increased dedifferentiation ability as compared to a comparable immune cell lacking the enhanced receptor. In some embodiments, the modified immune cell provided herein has increased dedifferentiation ability as compared to a comparable immune cell lacking the enhanced receptor. In some embodiments, the modified immune cell provided herein has greater transdifferentiation ability as compared to a comparable immune cell lacking the enhanced receptor.

In some embodiments, the immune cell activation signal can comprise or result in movement and/or trafficking of the immune cell. In some embodiments, movement can be determined by quantifying localization of the immune cell to a target site. For example, the modified immune cells provided herein can be quantified at a target site after administration, for example at a site that is not the target site. Quantification can be performed by isolating a lesion and quantifying a number of immune cells, for example tumor-infiltrating lymphocytes, comprising the enhanced receptor. Movement and/or trafficking of an immune cell comprising an enhanced receptor can be greater than that of a comparable immune cell lacking the enhanced receptor. In some embodiments, the number of immune cells comprising the enhanced receptor at a target site, for example a tumor lesion, can be about 5X, 10X, 15X, 20X, 25X, 30X, 35X, or 40X that of comparable immune cells lacking the enhanced receptor. Trafficking can also be determined *in vitro* utilizing a transwell migration assay. In some embodiments, the number of immune cells comprising the enhanced receptor at a target site, for example in a transwell migration assay, can be about 5X, 10X, 15X, 20X, 25X, 30X, 35X, or 40X that of comparable immune cells lacking the enhanced receptor.

In some embodiments, the immune cell activation signal can comprise or result in exhaustion and/or activation of the immune cell. Exhaustion and/or activation of the immune cell can be determined by phenotypic analysis by flow cytometry or microscopic analysis. For example, expression levels of markers of exhaustion, for instance programmed cell death protein 1 (PD1), lymphocyte activation gene 3 protein (LAG3), 2B4, CD160, Tim3, and T cell immunoreceptor with immunoglobulin and ITIM domains (TIGIT), can be determined quantitatively and/or qualitatively. In some cases, immune cells, such as T cells, can lose effector functions in a hierarchical manner and become exhausted. As a result of exhaustion, functions such as IL-2 production and cytokine expression, as well as high proliferative capacity, can be lost. Exhaustion can also be followed by defects in the production of IFNγ, TNF and chemokines, as well as in degranulation. Exhaustion or activation of the modified immune cell provided herein can be greater than that of a comparable immune cell lacking the enhanced receptor. In some embodiments, the immune cell provided herein can undergo at least about a 1 fold, 2 fold, 3 fold, 4 fold, 5 fold, 6 fold, 7 fold, 8 fold, 9 fold, 10 fold, 11 fold, 12 fold, 13 fold, 14 fold, 15 fold, 20 fold, 30 fold, 40 fold, 50 fold, 60 fold, 70 fold, 80 fold, 90 fold, 100 fold, 150 fold, 200 fold, 250 fold, or over 300 fold increase in exhaustion or activation as compared to a comparable immune cell lacking the enhanced receptor. In some embodiments, the immune cell provided herein can undergo at least about a 1 fold, 2 fold, 3 fold, 4 fold, 5 fold, 6 fold, 7 fold, 8 fold, 9 fold, 10 fold, 11 fold, 12 fold, 13 fold, 14 fold, 15 fold, 20 fold, 30 fold, 40 fold, 50 fold, 60 fold, 70 fold, 80 fold, 90 fold, 100 fold, 150 fold, 200 fold, 250 fold, or over 300 fold decrease in exhaustion or activation as compared to a comparable immune cell lacking the enhanced receptor.

In some embodiments, binding of a target cell to the enhanced receptor can yield an immune cell activation signal in the immune cell modified with the enhanced receptor.

In some embodiments, upon binding of the enhanced receptor to its ligand, the immune cell modified with the enhanced receptor (e.g., modified TIL or modified T cell) exhibits enhanced neoantigen binding as compared to an immune cell lacking the enhanced receptor.

In some embodiments, the immune cell modified with the enhanced receptor (such as TIL, neoantigen-reactive T cell, CAR-T, TCR-T, NK, etc.), as compared to an immune cell modified without the enhanced receptor (corresponding to the aforementioned TIL, neoantigen-reactive T cell, CAR-T, TCR-T, NK, etc., respectively), can better overcome the inhibitory signal from the tumor microenvironment on the immune cell to a certain extent, which can be from a ligand or antigen of the extracellular domain of the enhanced receptor, or other inhibitory signals from the tumor microenvironment on the immune cell other than the ligand or antigen.

In some embodiments, the ECD of the enhanced receptor is an anti-PD1 or PDL1 monoclonal antibody, and the ICD is any kind of costimulatory molecule, the tumor inhibitory signal that can be effectively overcome by which to a certain extent is not only from PDL1, but also may include inhibitory signals from CD47, TIM-3 ligands such as Galectin-9, TIGIT ligands such as CD155 and CD122 or PVR, or CTLA-4 ligands such as B7 on the immune cell.

In some embodiments, the ECD of the enhanced receptor is an anti-SIRPα or CD47 monoclonal antibody, and the ICD is any kind of costimulatory molecule, the tumor inhibitory signal that can be effectively overcome by which to a certain extent is not only from CD47, but also may include inhibitory signals from PDL1, TIM-3 ligands such as Galectin-9, TIGIT ligands such as CD155 and CD122 or PVR, or CTLA-4 ligands such as B77 on the immune cell.

In some embodiments, the ECD of the enhanced receptor is an anti-TIM-3 ligand monoclonal antibody, such as an anti-Galectin-9 monoclonal antibody or an anti-TIM-3 monoclonal antibody, and the ICD is any kind of costimulatory molecule, the tumor inhibitory signal that can be effectively overcome by which to a certain extent is not only from TIM-3 ligands such as Galectin-9, but also may include inhibitory signals from PDL1, TIGIT ligands such as CD155 and CD122 or PVR, or CTLA-4 ligands such as B77 on the immune cell.

In some embodiments, the ECD of the enhanced receptor is a monoclonal antibody against TIGIT or its ligands, such as an anti-CD155 monoclonal antibody or an anti-CD122 monoclonal antibody or an anti-PVR monoclonal antibody, and the ICD is any kind of costimulatory molecule, the tumor inhibitory signal that can be effectively overcome by which to a certain extent is not only from TIGIT ligands such as CD155 and CD122 or PVR, but also may include inhibitory signals from PDL1, TIM-3 ligands such as Galectin-9, or CTLA-4 ligands such as B77 on the immune cell.

In some embodiments, the ECD of the enhanced receptor is VISTA, and the ICD is any kind of costimulatory molecule, the tumor inhibitory signal that can be effectively overcome by which to a certain extent is not only from VISTA ligands; or the ECD of the enhanced receptor is CTLA-4, the tumor inhibitory signal that can be effectively overcome by which to a certain extent is not only from CTLA-4 ligands such as B7, but also may include inhibitory signals from PDL1, CD47, TIM-3 ligands such as Galectin-9, TIGIT ligands such as CD155 and CD122 or PVR, or CTLA-4 ligands such as B77 on the immune cell.

In some embodiments, T cells are loaded with a T cell receptor (TCR) or a chimeric antigen receptor (CAR), wherein when the TCR or CAR can recognize target cells, the T cells modified with the enhanced receptor have stronger immune cell activation function than T cells modified without the enhanced receptor; and when neither the TCR nor the CAR can recognize target cells, the T cells modified with the enhanced receptor do not have stronger immune cell activation function than T cells modified without the enhanced receptor.

In an aspect, the present disclosure provides a modified immune cell comprising a chimeric antigen receptor (CAR) and a T cell receptor (TCR) complex which exhibits specific binding to a neoantigen. The CAR can comprise an antigen interacting domain capable of binding a B cell surface protein, a transmembrane domain, and an intracellular signaling domain.

The T cell receptor (TCR) complex which exhibits specific binding to the neoantigen can be an endogenous TCR complex or an exogenous TCR complex. The TCR complex, e.g., endogenous or exogenous, of the modified immune cell can confer the antigen binding specificity (e.g., neoantigen binding) of the immune cell.

In some embodiments, the immune cell is a tumor-infiltrating lymphocyte (TIL). The TIL can be, for example, a T cell, B cell, monocyte or natural killer (NK) cell. In some cases, the TIL comprises a CD8+ cytotoxic T cell (lymphocyte), Th1 and Th17 CD4+ T cell, a natural killer cell, a dendritic cell, or an M1 macrophage. In some embodiments, the TIL can express at least one of PD-1, CD137 and TIM-3. In some cases, the modified TIL comprises a "primary TIL," referring to a TIL that is obtained from a patient tissue sample. In some cases, the modified TIL comprises a "secondary TIL," referring to a TIL that has been expanded or proliferated. In some cases, the TIL can leave the tumor tissue and exist in the peripheral blood, which can be obtained by isolation of peripheral blood mononuclear cells (PBMCs) by apheresis, or by further screening and enrichment with one or more markers such as PD1, TIM3, CD137, CD39, etc.

The CAR can comprise an antigen interacting domain capable of binding a B cell surface protein. The B cell surface protein can be any protein that may be found on the surface of a B cell. Non-limiting examples include CD1d, CD5, CD10, CD11a, CD19, CD20, CD21, CD22, CD23, CD24, CD25, CD27, CD28, CD29, CD34, CD37, CD38, CD40, CD44, CD45, CD49b, CD69, CD72, CD74, CD80, CD83, CD84, CD86, CD93, CD95, CD117, CD127, CD138, CD147, CD148, CD185, CD270, CD284 and CD360. In some embodiments, the antigen interacting domain of the CAR can be capable of binding a surface protein on a non-B cell, as long as the binding to the surface protein does not significantly compromise the general health status or the immune system of the host. In some embodiments, the surface protein is a surfer protein on an immune cell. In some embodiments, the surface protein is a surface protein on a cell other than an immune cell. In some embodiments, the surface protein may be selected from, with the proviso that the binding to the surface protein does not significantly compromise the general health status or the immune system of the host, CD31, CD32, A, B, CD33, CD34, CD35, CD36, CD37, CD38, CD39, CD40, CD41, CD42, a, b, c, d, CD43, CD44, CD45, CD46, CD47, CD48, CD49 (a, b, c, d, e, f), CD50, CD51, CD52, CD53, CD54, CD55, CD56, CD57, CD58, CD59, CD61, CD62 (E, L, P), CD63, CD64 (A, B, C), CD66 (a, b, c, d, e, f), CD68, CD69, CD70, CD71, CD72, CD73, CD74, CD78, CD79 (a, b), CD80, CD81, CD82, CD83, CD84, CD85 (a, d, e, h, j, k), CD86, CD87, CD88, CD89, CD90, CD91, CD92, CD93, CD94, CD95, CD96, CD97, CD98, CD99, CD100, CD1 (a-c), 1A, 1D, 1E, CD2, CD3 (γ, δ, ε), CD4, CD5, CD6, CD7, CD8, a, CD9, CD10, CD11 (a, b, c, d), CD13, CD14, CD15, CD16, A, B, CD18, CD19, CD20, CD21, CD22, CD23, CD24, CD25, CD26, CD27, CD28, CD29, CD30, CD101, CD102, CD103, CD104, CD105, CD106, CD107 (a, b), CD108, CD109, CD110, CD111, CD112, CD113, CD114, CD115, CD116, CD117, CD118, CD119, CD120 (a, b), CD121 (a, b), CD122, CD123, CD124, CD125, CD126, CD127, CD129, CD130, CD131, CD132, CD133, CD134, CD135, CD136, CD137, CD138, CD140b, CD141, CD142, CD143, CD144, CD146, CD147, CD148, CD150, CD191, CD192, CD193, CD194, CD195, CD196, CD197, CDw198, CDw199, CD200, CD201, CD202b, CD204, CD205, CD206, CD207, CD208, CD209, CDw210 (a, b), CD212, CD213a (1, 2), CD217, CD218, (a, b), CD220, CD221, CD222, CD223, CD224, CD225, CD226, CD227, CD228, CD229, CD230, CD233, CD234, CD235 (a, b), CD236, CD238, CD239, CD240CE, CD240D, CD241, CD243, CD244, CD246, CD247, CD248, CD249, CD252, CD253, CD254, CD256, CD257, CD258, CD261, CD262, CD263, CD264, CD265, CD266, CD267, CD268, CD269, CD271, CD272, CD273, CD274, CD275, CD276, CD278, CD279, CD280, CD281, CD282, CD283, CD284, CD286, CD288, CD289, CD290, CD292, CDw293, CD294, CD295, CD297, CD298, CD299, CD300A, CD301, CD302, CD303, CD304, CD305, CD306, CD307, CD309, CD312, CD314, CD315, CD316, CD317, CD318, CD320, CD321, CD322, CD324, CD325, CD326, CD328, CD329, CD331, CD332, CD333, CD334, CD335, CD336, CD337, CD338, CD339, CD340, CD344, CD349, CD350, CD151, CD152, CD153, CD154, CD155, CD156 (a, b, c), CD157, CD158, (a, d, e, i, k), CD159 (a, c), CD160, CD161, CD162, CD163, CD164, CD166, CD167 (a, b), CD168, CD169, CD170, CD171, CD172 (a, b, g), CD174, CD177, CD178, CD179 (a, b), CD180, CD181, CD182, CD183, CD184, CD185 and CD186.

In some embodiments, the antigen interacting domain of the CAR can be capable of binding a B cell surface protein or fragment thereof on a dead B cell. B cell apoptosis can occur before or after development of an immune response (e.g., an immune response towards a tumor cell). Thus, a dead B cell or its debris can still have the B cell surface protein or fragment thereof presented on the surface. The ability of the CAR to target both live and dead B cells may increase the chance of the immune cell comprising the CAR to (i) bind the B cell surface protein and (i) initiate signaling of the intracellular signaling domain. In some cases, the signaling of the intracellular signaling domain may promote expansion (proliferation) of the immune cell comprising the CAR.

In some embodiments, the antigen interacting domain of the CAR can be capable of binding a B cell surface protein or fragment thereof that is coupled (e.g., via a covalent and/or non-covalent bond) to the surface of particles (e.g., nanoparticles). The particles may be any particulate materials comprising organic and/or inorganic material. The particles may have various shapes and sizes. The particles may be about 1 nanometer (nm) to about 50 micometers (µm) in at least one dimension. The particles may be at least about 1 nm, 5 nm, 10 nm, 50 nm, 100 nm, 500 nm, 1 µm, 5 µm, 10 µm, 50 µm or more in at least one dimension. The particles may be at most about 50 µm, 10 µm, 5 µm, 1 µm, 500 nm, 100 nm, 50 nm, 10 nm, 5 nm, 1 nm or less in at least one dimension. The particles may be nanoparticles, microparticles, nanospheres, micropsheres, nanorods, microrods, nanofibers, nanoribbons, etc. Examples of the particles include metal nanoparticles (e.g., gold nanoparticles, silver nanoparticles, and iron nanoparticles), intermetallic nanosemiconductor nanoparticles, core-shell nanoparticles, particles with an inorganic core with a polymer shell, particles with an organic core with a polymer shell, and mixtures thereof. Alternatively, the particles can be organic nanoparticles, such as crosslinked polymers, hydrogel polymers, biodegradable polymers, polylactide (PLA), polyglycolide (PGA), polycaprolactone (PCL), copolymers, polysaccharides, starch, cellulose, chitosan, polyhydroxyalkanoates (PHA), PHB, PHV, lipids, peptides, peptide amphiphiles, polypeptides (e.g., proteins), or combinations thereof. The particles presenting the B cell surface protein on the surface may be introduced *in vitro* to the immune cell comprising the CAR that binds the B cell surface protein. Alternatively or in addition to, the particles presenting the B cell surface protein may be introduced *in vivo* (e.g., by local or systemic injection) along with the immune cell comprising the CAR. Such particles may be used to expand a population of immune cells comprising the CAR *in vitro* or *in vivo.*

The antigen binding domain can comprise any protein or molecule capable of binding to an antigen, e.g., B cell surface protein. Non-limiting examples of the antigen binding domain include, but are not limited to, a monoclonal antibody, a polyclonal antibody, a recombinant antibody, a human antibody, a humanized antibody, a murine antibody, or a functional derivative, variant or fragment thereof, including, but not limited to, Fab, Fab', F(ab')2, Fv, a single-chain Fv (scFv), a minibody, a diabody, and a single-domain antibody such as a heavy chain variable domain (VH), a light chain variable domain (VL) and a variable domain (VHH) of camelid-derived nanobody. In some embodiments, the first antigen binding domain comprises at least one of Fab, Fab', F(ab')2, Fv and scFv. In some embodiments, the antigen binding domain comprises an antibody mimetic. Antibody mimetics refer to molecules which can bind a target molecule with an affinity comparable to an antibody, and include single-chain binding molecules, cytochrome b562-based binding molecules, fibronectin or fibronectin-like protein scaffolds (e.g., adnectins), lipocalin scaffolds, calixarene scaffolds, A-domains and other scaffolds. In some embodiments, the antigen binding domain comprises a transmembrane receptor, or any derivative, variant or fragment thereof. For example, the antigen binding domain can comprise at least a ligand binding domain of the transmembrane receptor.

In some embodiments, the antigen interacting domain of the CAR can be capable of binding a B cell surface protein or fragment thereof on a dead B cell. B cell apoptosis can occur before or after development of an immune response (e.g., an immune response towards a tumor cell). Thus, a dead B cell or its debris can still have the B cell surface protein or fragment thereof presented on the surface. The ability of the CAR to target both live and dead B cells may increase the chance of the immune cell comprising the CAR to (i) bind the B cell surface protein and (i) initiate signaling of the intracellular signaling domain. In some cases, the signaling of the intracellular signaling domain may promote expansion (proliferation) of the immune cell comprising the CAR.

In some embodiments, the antigen interacting domain of the CAR can be capable of binding a B cell surface protein or fragment thereof that is coupled (e.g., via a covalent and/or non-covalent bond) to the surface of particles (e.g., nanoparticles). The particles may be any particulate materials comprising organic and/or inorganic material. The particles may have various shapes and sizes. The particles may be about 1 nanometer (nm) to about 50 micometers (µm) in at least one dimension. The particles may be at least about 1 nm, 5 nm, 10 nm, 50 nm, 100 nm, 500 nm, 1 µm, 5 µm, 10 µm, 50 µm or more in at least one dimension. The particles may be at most about 50 µm, 10 µm, 5 µm, 1 µm, 500 nm, 100 nm, 50 nm, 10 nm, 5 nm, 1 nm or less in at least one dimension. The particles may be nanoparticles, microparticles, nanospheres, micropsheres, nanorods, microrods, nanofibers, nanoribbons, etc. Examples of the particles include metal nanoparticles (e.g., gold nanoparticles, silver nanoparticles, and iron nanoparticles), intermetallic nanosemiconductor nanoparticles, core-shell nanoparticles, particles with an inorganic core with a polymer shell, particles with an organic core with a polymer shell, and mixtures thereof. Alternatively, the particles can be organic nanoparticles, such as crosslinked polymers, hydrogel polymers, biodegradable polymers, polylactide (PLA), polyglycolide (PGA), polycaprolactone (PCL), copolymers, polysaccharides, starch, cellulose, chitosan, polyhydroxyalkanoates (PHA), PHB, PHV, lipids, peptides, peptide amphiphiles, polypeptides (e.g., proteins), or combinations thereof. The particles presenting the B cell surface protein on the surface may be introduced *in vitro* to the immune cell comprising the CAR that binds the B cell surface protein. Alternatively or in addition to, the particles presenting the B cell surface protein may be introduced *in vivo* (e.g., by local or systemic injection) along with the immune cell comprising the CAR. Such particles may be used to expand a population of immune cells comprising the CAR *in vitro* or *in vivo.*

The antigen binding domain can comprise any protein or molecule capable of binding to an antigen, e.g., B cell surface protein. Non-limiting examples of the antigen binding domain include, but are not limited to, a monoclonal antibody, a polyclonal antibody, a recombinant antibody, a human antibody, a humanized antibody, a murine antibody, or a functional derivative, variant or fragment thereof, including, but not limited to, Fab, Fab', F(ab')2, Fv, a single-chain Fv (scFv), a minibody, a diabody, and a single-domain antibody such as a heavy chain variable domain (VH), a light chain variable domain (VL) and a variable domain (VHH) of camelid-derived nanobody. In some embodiments, the first antigen binding domain comprises at least one of Fab, Fab', F(ab')2, Fv and scFv. In some embodiments, the antigen binding domain comprises an antibody mimetic. Antibody mimetics refer to molecules which can bind a target molecule with an affinity comparable to an antibody, and include single-chain binding molecules, cytochrome b562-based binding molecules, fibronectin or fibronectin-like protein scaffolds (e.g., adnectins), lipocalin scaffolds, calixarene scaffolds, A-domains and other scaffolds. In some embodiments, the antigen binding domain comprises a transmembrane receptor, or any derivative, variant or fragment thereof. For example, the antigen binding domain can comprise at least a ligand binding domain of the transmembrane receptor.

In some embodiments, the antigen binding domain can comprise scFv. scFv can be derived from an antibody for which the sequences of the variable regions are known. In some embodiments, scFv can be derived from an antibody sequence obtained from an available mouse hybridoma. scFv can be obtained from whole-exomic sequencing of a tumor cell or primary cell. In some embodiments, scFv can be altered. For instance, scFv may be modified in a variety of ways. In some cases, scFv can be mutated, so that the scFv may have higher affinity to its target. In some cases, the affinity of the scFv for its target can be optimized for targets expressed at low levels on normal tissues. This optimization can be performed to minimize potential toxicities, such as hypercytokinemia. In other cases, the cloning of scFv that has higher affinity for the membrane-bound form of a target can be preferable over its soluble form counterpart. This modification can be performed if some targets can also be detected in soluble form at different levels and their targeting can cause unintended toxicity, such as hypercytokinemia.

The antigen binding domain of a CAR of a subject system can be linked to the intracellular signaling domain via a transmembrane domain. The transmembrane domain can be a transmembrane segment. The transmembrane domain of a subject CAR can anchor the CAR to the plasma membrane of a cell, for example an immune cell. In some embodiments, the transmembrane segment comprises a polypeptide. The transmembrane polypeptide linking the antigen binding domain and the intracellular signaling domain of the CAR can have any suitable polypeptide sequence. In some cases, the transmembrane polypeptide comprises a polypeptide sequence of a transmembrane portion of an endogenous or wild-type transmembrane protein. In some embodiments, the transmembrane polypeptide comprises a polypeptide sequence having at least 1 (e.g., at least 2, 3, 4, 5, 6, 7, 8, 9, 10 or more) amino acid substitutions, deletions and insertions compared to a transmembrane portion of an endogenous or wild-type transmembrane protein. In some embodiments, the transmembrane polypeptide comprises a non-natural polypeptide sequence, such as the sequence of a polypeptide linker. The polypeptide linker may be flexible or rigid. The polypeptide linker can be structured or unstructured. In some embodiments, the transmembrane polypeptide transmits a signal from an extracellular region of a cell to an intracellular region via the antigen binding domain. A native transmembrane portion of CD28 can be used in a CAR. In other cases, a native transmembrane portion of CD8α can also be used in a CAR.

The CAR of the present disclosure can comprise a signaling domain involved in immune cell signaling, or any derivative, variant or fragment thereof. The intracellular signaling domain of a CAR can induce activity of an immune cell comprising the CAR. The intracellular signaling domain can transduce the effector function signal and direct the cell to perform a specialized function. The signaling domain can comprise signaling domains of other molecules. In some cases, a truncated portion of the signaling domain is used in a CAR.

In some embodiments, the intracellular signaling domain comprises multiple signaling domains involved in immune cell signaling, or any derivatives, variants or fragments thereof. For example, the intracellular signaling domain can comprise at least 2 immune cell signaling domains, e.g., at least 2, 3, 4, 5, 7, 8, 9 or 10 immune cell signaling domains. An immune cell signaling domain can be involved in regulating primary activation of the TCR complex in either a stimulatory way or an inhibitory way. The intracellular signaling domain may be that of a T cell receptor (TCR) complex. The intracellular signaling domain of a subject CAR can comprise a signaling domain of an Fcγ receptor (FcγR), an Fcε receptor (FcεR), an Fcα receptor (FcαR), neonatal Fc receptor (FcRn), CD3, CD3ζ, CD3γ, CD36, CD3ε, CD4, CD5, CD8, CD21, CD22, CD28, CD32, CD40L (CD154), CD45, CD66d, CD79a, CD79b, CD80, CD86, CD278 (also known as ICOS), CD247ζ, CD247η, DAP10, DAP12, FYN, LAT, Lck, MAPK, an MHC complex, NFAT, NF-κB, PLC-γ, iC3b, C3dg, C3d and Zap70. In some embodiments, the signaling domain comprises an immunoreceptor tyrosine-based activation motif or ITAM. A signaling domain comprising an ITAM can comprise two repeats of the amino acid sequence YxxL/I separated by 6-8 amino acids, wherein each x is independently any amino acid, producing the conserved motif YxxL/Ix(6-8)YxxL/I. A signaling domain comprising an ITAM can be modified, for example, by phosphorylation when the antigen binding domain is bound to an epitope. A phosphorylated ITAM can function as a docking site for other proteins, for example proteins involved in various signaling pathways. In some embodiments, the primary signaling domain comprises a modified ITAM domain, e.g., a mutated, truncated, and/or optimized ITAM domain, which has altered (e.g., increased or decreased) activity compared to the native ITAM domain.

In some embodiments, the intracellular signaling domain of a subject CAR comprises an FcγR signaling domain (e.g., ITAM). The FcγR signaling domain can be selected from FcγRI (CD64), FcyRIIA (CD32), FcyRIIB (CD32), FcγRIIIA (CD16a) and FcyRIIIB (CD16b). In some embodiments, the intracellular signaling domain comprises an FcεR signaling domain (e.g., ITAM). The FcεR signaling domain can be selected from FcεRI and FcεRII (CD23). In some embodiments, the intracellular signaling domain comprises an FcαR signaling domain (e.g., ITAM). The FcαR signaling domain can be selected from FcαRI (CD89) and Fcα/µR. In some embodiments, the intracellular signaling domain comprises a CD3ζ signaling domain. In some embodiments, the primary signaling domain comprises an ITAM of CD3ζ.

In some embodiments, the intracellular signaling domain of a subject CAR comprises an immunoreceptor tyrosine-based inhibition motif or ITIM. A signaling domain comprising an ITIM can comprise a conserved sequence of amino acids (S/I/V/LxYxxI/V/L) that is found in the cytoplasmic tails of some inhibitory receptors of the immune system. A primary signaling domain comprising an ITIM can be modified, for example phosphorylated, by enzymes such as an Src kinase family member (e.g., Lck). Following phosphorylation, other proteins, including enzymes, can be recruited to the ITIM. These other proteins include, but are not limited to, enzymes such as the phosphotyrosine phosphatases SHP-1 and SHP-2, the inositol-phosphatase called SHIP, and proteins having one or more SH2 domains (e.g., ZAP70). The intracellular signaling domain can comprise a signaling domain (e.g., ITIM) of BTLA, CD5, CD31, CD66a, CD72, CMRF35H, DCIR, EPO-R, FcγRIIB (CD32), Fc receptor-like protein 2 (FCRL2), Fc receptor-like protein 3 (FCRL3), Fc receptor-like protein 4 (FCRL4), Fc receptor-like protein 5 (FCRL5), Fc receptor-like protein 6 (FCRL6), protein G6b (G6B), interleukin 4 receptor (IL4R), immunoglobulin superfamily receptor translocation-associated 1 (IRTA1), immunoglobulin superfamily receptor translocation-associated 2 (IRTA2), killer cell immunoglobulin-like receptor 2DL1 (KIR2DL1), killer cell immunoglobulin-like receptor 2DL2 (KIR2DL2), killer cell immunoglobulin-like receptor 2DL3 (KIR2DL3), killer cell immunoglobulin-like receptor 2DL4 (KIR2DL4), killer cell immunoglobulin-like receptor 2DL5 (KIR2DL5), killer cell immunoglobulin-like receptor 3DL1 (KIR3DL1), killer cell immunoglobulin-like receptor 3DL2 (KIR3DL2), leukocyte immunoglobulin-like receptor subfamily B member 1 (LIR1), leukocyte immunoglobulin-like receptor subfamily B member 2 (LIR2), leukocyte immunoglobulin-like receptor subfamily B member 3 (LIR3), leukocyte immunoglobulin-like receptor subfamily B member 5 (LIR5), leukocyte immunoglobulin-like receptor subfamily B member 8 (LIR8), leukocyte-associated immunoglobulin-like receptor 1 (LAIR-1), mast cell function-associated antigen (MAFA), NKG2A, natural cytotoxicity triggering receptor 2 (NKp44), NTB-A, programmed cell death protein 1 (PD-1), PILR, SIGLECL1, sialic acid binding Ig like lectin 2 (SIGLEC2 or CD22), sialic acid binding Ig like lectin 3 (SIGLEC3 or CD33), sialic acid binding Ig like lectin 5 (SIGLEC5 or CD170), sialic acid binding Ig like lectin 6 (SIGLEC6), sialic acid binding Ig like lectin 7 (SIGLEC7), sialic acid binding Ig like lectin 10 (SIGLEC10), sialic acid binding Ig like lectin 11 (SIGLEC11), sialic acid binding Ig like lectin 4 (SIGLEC4), sialic acid binding Ig like lectin 8 (SIGLEC8), sialic acid binding Ig like lectin 9 (SIGLEC9), platelet and endothelial cell adhesion molecule 1 (PECAM-1), signal regulatory protein (SIRP2), and signaling threshold regulating transmembrane adaptor 1 (SIT). In some embodiments, the intracellular signaling domain comprises a modified ITIM domain, e.g., a mutated, truncated, and/or optimized ITIM domain, which has altered (e.g., increased or decreased) activity compared to the native ITIM domain.

In some embodiments, the intracellular signaling domain comprises at least 2 ITAM domains (e.g., at least 3, 4, 5, 6, 7, 8, 9, or 10 ITAM domains). In some embodiments, the intracellular signaling domain comprises at least 2 ITIM domains (e.g., at least 3, 4, 5, 6, 7, 8, 9, or 10 ITIM domains) (e.g., at least 2 primary signaling domains). In some embodiments, the intracellular signaling domain comprises both ITAM and ITIM domains.

In some cases, the intracellular signaling domain of a subject CAR can comprise a costimulatory domain. In some embodiments, a costimulatory domain, for example from a costimulatory molecule, can provide costimulatory signals for immune cell signaling, such as signaling from ITAM and/or ITIM domains, e.g., for the activation and/or deactivation of immune cell activity. In some embodiments, the costimulatory domain can be used to regulate a proliferative and/or survival signal in the immune cell. In some embodiments, the costimulatory signaling domain comprises a signaling domain of a MHC class I protein, MHC class II protein, TNF receptor protein, immunoglobulin-like protein, cytokine receptor, integrin, signaling lymphocytic activation molecule (SLAM protein), activating NK cell receptor, BTLA, or a Toll ligand receptor. In some embodiments, the costimulatory domain comprises a signaling domain of a molecule selected from the group consisting of: 2B4/CD244/SLAMF4, 4-1BB/TNFSF9/CD137, B7-1/CD80, B7-2/CD86, B7-H1/PD-L1, B7-H2, B7-H3, B7-H4, B7-H6, B7-H7, BAFF R/TNFRSF13C, BAFF/BLyS/TNFSF13B, BLAME/SLAMF8, BTLA/CD272, CD100 (SEMA4D), CD103, CD11a, CD11b, CD11c, CD11d, CD150, CD160 (BY55), CD18, CD19, CD2, CD200, CD229/SLAMF3, CD27 Ligand/TNFSF7, CD27/TNFRSF7, CD28, CD29, CD2F-10/SLAMF9, CD30 Ligand/TNFSF8, CD30/TNFRSF8, CD300a/LMIR1, CD4, CD40 Ligand/TNFSF5, CD40/TNFRSF5, CD48/SLAMF2, CD49a, CD49D, CD49f, CD5, CD53, CD58/LFA-3, CD69, CD7, CD8α, CD8β, CD82/Kai-1, CD84/SLAMF5, CD90/Thy1, CD96, CDS, CEACAM1, CRACC/SLAMF7, CRTAM, CTLA-4, DAP12, Dectin-1/CLEC7A, DNAM1 (CD226), DPPIV/CD26, DR3/TNFRSF25, EphB6, GADS, Gi24/VISTA/B7-H5, GITR Ligand/TNFSF18, GITR/TNFRSF18, HLA Class I, HLA-DR, HVEM/TNFRSF14, IA4, ICAM-1, ICOS/CD278, Ikaros gene, IL2Rβ, IL2Rγ, IL7Rα, Integrin α4/CD49d, Integrin α4β1, Integrin α4β7/LPAM-1, IPO-3, ITGA4, ITGA6, ITGAD, ITGAE, ITGAL, ITGAM, ITGAX, ITGB1, ITGB2, ITGB7, KIRDS2, LAG-3, LAT, LIGHT/TNFSF14, LTBR, Ly108, Ly9 (CD229), lymphocyte function associated antigen-1 (LFA-1), Lymphotoxin-α/TNF-β, NKG2C, NKG2D, NKp30, NKp44, NKp46, NKp80 (KLRF1), NTB-A/SLAMF6, OX40 Ligand/TNFSF4, OX40/TNFRSF4, PAG/Cbp, PD-1, PDCD6, PD-L2/B7-DC, PSGL1, RELT/TNFRSF19L, SELPLG (CD162), SLAM (SLAMF1), SLAM/CD150, SLAMF4 (CD244), SLAMF6 (NTB-A), SLAMF7, SLP-76, TACI/TNFRSF13B, TCL1A, TCL1B, TIM-1/KIM-1/HAVCR, TIM-4, TL1A/TNFSF15, TNF RII/TNFRSF1B, TNF-α, TRANCE/RANKL, TSLP, TSLP R, VLA1 and VLA-6. In some embodiments, the intracellular signaling domain comprises multiple costimulatory domains, for example at least two, e.g., at least 3, 4, or 5 costimulatory domains. Costimulatory signaling regions may provide a signal synergistic with the primary effector activating signal and can complete the requirements for activation of a T cell. In some embodiments, the addition of costimulatory domains to the CAR can enhance the efficacy and persistence of the immune cells provided herein.

Binding of the CAR to the B cell surface protein can enhance immune cell proliferation as compared to an immune cell lacking the CAR. Proliferation of the immune cell can refer to expansion of the immune cell. Proliferation of the immune cell can refer to phenotypic changes of the immune cell. Proliferation of an immune cell comprising the CAR provided herein can be greater than that of an immune cell lacking the CAR which exhibits binding to a B cell surface protein. Proliferation of an immune cell comprising the CAR can be about 5 fold to about 10 fold, about 10 fold to about 20 fold, about 20 fold to about 30 fold, about 30 fold to about 40 fold, about 40 fold to about 50 fold, about 50 fold to about 60 fold, about 60 fold to about 70 fold, about 70 fold to about 80 fold, about 80 fold to about 90 fold, about 90 fold to about 100 fold, about 100 fold to about 200 fold, about 200 fold to about 300 fold, about 300 fold to about 400 fold, about 400 fold to about 500 fold, about 500 fold to about 600 fold, about 600 fold to about 700 fold greater than the proliferation of a comparable immune cell lacking the CAR. Proliferation of an immune cell comprising the CAR can be about 5 fold to about 10 fold, about 10 fold to about 20 fold, about 20 fold to about 30 fold, about 30 fold to about 40 fold, about 40 fold to about 50 fold, about 50 fold to about 60 fold, about 60 fold to about 70 fold, about 70 fold to about 80 fold, about 80 fold to about 90 fold, about 90 fold to about 100 fold, about 100 fold to about 200 fold, about 200 fold to about 300 fold, about 300 fold to about 400 fold, about 400 fold to about 500 fold, about 500 fold to about 600 fold, about 600 fold to about 700 fold greater than the proliferation of a comparable immune cell lacking the CAR, and wherein the proliferation is ascertained at least about 12, 24, 36, 48, 60, 72, 84, or 96 hours after contacting the B cell to the B cell surface protein. The enhanced proliferation can be ascertained either *in vitro* or *in vivo.* In some embodiments, proliferation can comprise quantifying the number of immune cells. Quantifying a number of immune cells can comprise flow cytometry, Trypan Blue exclusion and/or hemocytometry. Proliferation can also be determined by phenotypic analysis of the immune cells.

In an aspect, the present disclosure provides a modified immune cell that specifically binds to a neoantigen, wherein the modified immune cell comprises: (a) a chimeric stimulating molecule comprising a polypeptide extracellular domain (PED) that binds to the neoantigen, wherein the PED is fused to an intracellular domain (ICD) of a costimulatory molecule that mediates an immune cell activation signal, and wherein binding of the chimeric stimulating molecule to the neoantigen yields the immune cell activation signal in the modified immune cell; and (b) a chimeric antigen receptor comprising (i) an antigen interacting domain capable of binding a B cell surface protein; (ii) a transmembrane domain; and (iii) an intracellular signaling domain. In some embodiments, the PED can be an extracellular domain of a surface protein of an unmodified TIL. In some embodiments, examples of the PED include antibodies, as well as derivatives, variants, and fragments thereof.

In one embodiment, the present disclosure provides a modified immune cell that specifically binds to a neoantigen, wherein the modified immune cell comprises: (a) a natural TCR or a genetically engineered exogenous TCR that can specifically bind to the neoantigen; (b) an enhanced receptor comprising an extracellular domain (ECD) of a protein, wherein the ECD is fused to an intracellular domain (ICD) of a costimulatory molecule that mediates an immune cell activating signal, and wherein binding of the enhanced receptor to its ligand can yield the immune cell activation signal in the modified immune cell instead of an immune cell inactivation signal; and (c) a chimeric antigen receptor comprising (i) an antigen interacting domain capable of binding a B cell surface protein; (ii) a transmembrane domain; and (iii) an intracellular signaling domain. Among the above-mentioned a, b and c, a is indispensable; in some embodiments, only one of b or c may exist; and in some embodiments, a, b and c coexist.

In one embodiment, the present disclosure provides a modified tumor-infiltrating lymphocyte (TIL) that specifically binds to a neoantigen, wherein the modified immune cell comprises: (a) a natural TCR that can specifically bind to the neoantigen; (b) an enhanced receptor comprising an extracellular domain (ECD) of a protein, wherein the ECD is fused to an intracellular domain (ICD) of a costimulatory molecule that mediates an immune cell activation signal, and wherein binding of the switch molecule to its ligand can yield the immune cell activation signal in the modified TIL instead of an immune cell inactivation signal; and (c) a chimeric antigen receptor comprising (i) an antigen interacting domain capable of binding a B cell surface protein; (ii) a transmembrane domain; and (iii) an intracellular signaling domain. Among the above-mentioned a, b and c, a is indispensable; in some embodiments, only one of b or c may exist; and in some embodiments, a, b and c coexist.

In an aspect, the present disclosure provides a modified immune cell overexpressing a cytokine, for example a chemokine, wherein the immune cell is (i) a tumor-infiltrating lymphocyte (TIL); (ii) a stromal tumor-infiltrating lymphocyte (sTIL); or (iii) a T cell exhibiting specific binding to an antigen. The modified immune cell overexpressing the chemokine can be any modified immune cell provided herein.

Cytokines refer to proteins (e.g., chemokines, interferons, lymphokines, interleukins, and tumor necrosis factors) released by cells which can affect cell behavior. Cytokines are produced by a broad range of cells, including immune cells such as macrophages, B lymphocytes, T lymphocytes and mast cells, as well as endothelial cells, fibroblasts, and various stromal cells. A given cytokine can be produced by more than one type of cell. Cytokines can be involved in producing systemic or local immunomodulatory effects.

Certain cytokines can function as pro-inflammatory cytokines. Pro-inflammatory cytokines refer to cytokines involved in inducing or amplifying an inflammatory reaction. Pro-inflammatory cytokines can work with various cells of the immune system, such as neutrophils and leukocytes, to generate an immune response. Certain cytokines can function as anti-inflammatory cytokines. Anti-inflammatory cytokines refer to cytokines involved in the reduction of an inflammatory reaction. Anti-inflammatory cytokines, in some cases, can regulate a pro-inflammatory cytokine response. Some cytokines can function as both pro- and anti-inflammatory cytokines. Certain cytokines, e.g., chemokines, can function in chemotaxis. Chemokines can induce directed chemotaxis in nearby responsive cells.

In some embodiments, the expression of a cytokine having pro-inflammatory and/or chemotactic functions can be up-regulated in an immune cell. Up-regulating the expression of a cytokine having pro-inflammatory and/or chemotactic functions can be useful, for example, to stimulate an immune response against a target cell in immunotherapy.

Examples of cytokines that can be overexpressed by the immune cells provided herein include, but are not limited to, lymphokines, monokines, and traditional polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-alpha; mullerian-inhibiting substance; mouse gonadotropin-associated peptide; inhibin; activin; vascular endothelial growth factor; integrin; thrombopoietin (TPO); nerve growth factors such as NGF-α; platelet growth factor; transforming growth factors (TGFs) such as TGF-α, TGF-β, TGF-β1, TGF-β2 and TGF-β3; insulin-like growth factor-I and -II; erythropoietin (EPO); Flt-3L; stem cell factor (SCF); osteoinductive factors; interferons (IFNs) such as IFN-α, IFN-β and IFN-γ; colony stimulating factors (CSFs) such as macrophage-CSF (M-CSF); granulocyte-macrophage-CSF (GM-CSF); granulocyte-CSF (G-CSF); macrophage stimulating factor (MSP); interleukins (ILs) such as IL-1, IL-1a, IL-1b, IL-1RA, IL-18, IL-2, IL-3, IL-4, IL-5, IL-6, IL-7, IL-8, IL-9, IL-10, IL-11, IL-12, IL-13, IL-14, IL-15, IL-16, IL-17 and IL-20; tumor necrosis factors such as CD154, LT-β, TNF-α, TNF-β, 4-1BBL, APRIL, CD70, CD153, CD178, GITRL, LIGHT, OX40L, TALL-1, TRAIL, TWEAK and TRANCE; and other polypeptide factors including LIF, oncostatin M (OSM) and kit ligand (KL). Cytokine receptors refer to the receptor proteins which bind cytokines. Cytokine receptors may be both membrane-bound and soluble.

In some embodiments, the overexpressed cytokine is an interleukin (IL-1) family member (e.g., ligand), an IL-1 receptor family member, an interleukin-6 (IL-6) family member (e.g., ligand), an IL-6 receptor, an interleukin-10 (IL-10) family member (e.g., ligand), an IL-10 receptor, an interleukin-12 (IL-12) family member (e.g., ligand), an IL-12 receptor, an interleukin-17 (IL-17) family member (e.g., ligand), or an IL-17 receptor.

In some embodiments, the overexpressed cytokine is an interleukin-1 (IL-1) family member or related protein; a tumor necrosis factor (TNF) family member or related protein; an interferon (IFN) family member or related protein; an interleukin-6 (IL-6) family member or related protein; or a chemokine or related protein. In some embodiments, the cytokine is selected from IL18, IL18BP, IL1A, IL1B, IL1F10, IL1F3/IL1RA, IL1F5, IL1F6, IL1F7, IL1F8, IL1RL2, IL1F9, IL33, BAFF/BLyS/TNFSF138, 4-1BBL, CD153/CD30L/TNFSF8, CD40LG, CD70, Fas Ligand/FASLG/CD95L/CD178, EDA-A1, TNFSF14/LIGHT/CD258, TNFA, LTA/TNFB/TNFSF1, LTB/TNFC, CD70/CD27L/TNFSF7, TNFSF10/TRAIL/APO-2L (CD253), RANKL/OPGL/TNFSF11 (CD254), TNFSF12, TNF-α/TNFA, TNFSF13, TL1A/TNFSF15, OX-40L/TNFSF4/CD252, CD40L/CD154/TNFSF5, IFNA1, IFNA10, IFNA13, IFNA14, IFNA2, IFNA4, IFNA7, IFNB1, IFNE, IFNG, IFNZ, IFNA8, IFNA5/IFNaG, IFNω/IFNW1, CLCF1, CNTF, IL11, IL31, IL6, Leptin, LIF, OSM, CCL1/TCA3, CCL11, CCL12/MCP-5, CCL13/MCP-4, CCL14, CCL15, CCL16, CCL17/TARC, CCL18, CCL19, CCL2/MCP-1, CCL20, CCL21, CCL22/MDC, CCL23, CCL24, CCL25, CCL26, CCL27, CCL28, CCL3, CCL3L3, CCL4, CCL4L1/LAG-1, CCL5, CCL6, CCL7, CCL8, CCL9, CX3CL1, CXCL1, CXCL10, CXCL11, CXCL12, CXCL13, CXCL14, CXCL15, CXCL16, CXCL17, CXCL2/MIP-2, CXCL3, CXCL4, CXCL5, CXCL6, CXCL7/Ppbp, CXCL9, IL8/CXCL8, XCL1, XCL2, FAM19A1, FAM19A2, FAM19A3, FAM19A4 and FAM19A5.

Cytokine expression can be evaluated using a variety of methods. Cytokine expression can be evaluated by assaying cell culture media (e.g., *in vitro* production) in which the modified immune cells are grown or sera (e.g., *in vivo* production) obtained from a subject having the modified immune cells for the presence of one or more cytokines. Cytokine levels can be quantified in various suitable units, including concentration, using any suitable assay. In some embodiments, cytokine proteins are detected. In some embodiments, mRNA transcripts of cytokines are detected. Examples of cytokine assays include enzyme-linked immunosorbent assays (ELISA), immunoblot, immunofluorescence assays, radioimmunoassays, antibody arrays which allow various cytokines in a sample to be detected in parallel, bead-based arrays, quantitative PCR, microarrays, etc. Other suitable methods may include proteomics approaches (2-D gels, MS analysis, etc.).

In some embodiments, the cytokine overexpressed by the modified immune cell provided herein is a chemokine. The chemokine can be, for example, a CC chemokine, a CXC chemokine, a C chemokine, and a CX3C chemokine. In some embodiments, the chemokine overexpressed by the modified immune cell is a CC chemokine selected from CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL23, CCL24, CCL25, CCL26, CCL27 and CCL28. Said chemokine is a CXC chemokine selected from CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16 and CXCL17. In some embodiments, the chemokine overexpressed by the modified immune cell is a C chemokine selected from XCL1 and XCL2. In some embodiments, the chemokine overexpressed by the immune cell is a CX3C chemokine, and the CX3C chemokine is CX3CL1.

In an aspect, the present disclosure provides a method of treating a cancer in a subject, comprising: (a) administering to the subject the modified TIL, the modified T cell positively screened with one or more of PD1, TIM3, CD137 and CD39 in peripheral blood PBMCs, the modified T cell, or the modified immune cell of any one of the various embodiments of the aspects herein; and (b) contacting a target cell of the cancer expressing a neoantigen with the modified TIL, modified and selected T cell, modified T cell, or modified immune cell under conditions that induce cytotoxicity of the modified TIL, modified and selected T cell, modified T cell, or modified immune cell against the target cell, thereby inducing death of the target cell of the cancer.

In an aspect, the present disclosure provides a method of expanding a population of T cells, the method comprising: (a) providing a population of T cells comprising at least the modified immune cell of any one of the various embodiments of the aspects; and (b) exposing the population of T cells to a B cell surface protein to effect expansion of the population of T cells. In some embodiments, in (b), the population of T cells is exposed to a B cell comprising the B cell surface protein.

In an aspect, the present disclosure provides a method of expanding a population of T cells, comprising: (a) introducing a nucleic acid encoding a chimeric antigen receptor (CAR) into the population of T cells, thereby producing a CAR-expressing cell or population of cells, wherein the CAR comprises (i) a domain that interacts with the antigen specifically recognized by the CAR; (ii) a transmembrane domain; and (iii) an intracellular signaling domain; and (b) contacting the CAR-expressing population of cells with an antigen, thereby producing an expanded and/or activated population of immune cells. The antigen is not specifically expressed by a tumor target cell. The antigen may be a B cell surface protein such as CD19, BCMA, or other blood cell surface proteins, or a mutant of a natural protein such as EGFRviii, or an artificially modified protein.

In an aspect, the present disclosure provides a composition comprising one or more polynucleotides that encodes one or more of: (a) an enhanced receptor, wherein the enhanced receptor comprises an extracellular domain (ECD) of a protein, wherein the ECD is fused to an intracellular domain (ICD) of a costimulatory protein that mediates an immune cell activating signal; and (b) an antigen-specific T cell receptor complex, or one or more components thereof.

In an aspect, the present disclosure provides a composition comprising one or more polynucleotides that encodes one or more of: (a) an antigen-specific T cell receptor complex, or one or more components thereof; and (b) a chimeric antigen receptor comprising (i) an antigen interacting domain capable of binding a B cell surface protein; (ii) a transmembrane domain; and (iii) an intracellular signaling domain.

In an aspect, the present disclosure provides a composition comprising one or more polynucleotides that encodes one or more of: (a) an enhanced receptor, wherein the enhanced receptor comprises an extracellular domain (ECD) of a protein, wherein the ECD is fused to an intracellular domain (ICD) of a costimulatory protein that mediates an immune cell activating signal; (b) an antigen-specific T cell receptor complex, or one or more components thereof; and (c) a chimeric antigen receptor comprising (i) an antigen interacting domain capable of binding a B cell surface protein; (ii) a transmembrane domain; and (iii) an intracellular signaling domain.

In various embodiments of the aspects herein, promoters that can be used with the compositions of the present disclosure include those active in a eukaryotic, mammalian, non-human mammalian or human cell. The promoter can be an inducible or constitutively active promoter. Alternatively or additionally, the promoter can be tissue or cell specific.

Non-limiting examples of suitable eukaryotic promoters (i.e., promoters functional in a eukaryotic cell) can include those from cytomegalovirus (CMV) immediate early, herpes simplex virus (HSV) thymidine kinase, early and late SV40, long terminal repeats (LTRs) from retrovirus, human elongation factor-1 promoter (EF1), a hybrid construct comprising the cytomegalovirus (CMV) enhancer fused to the chicken beta-active promoter (CAG), murine stem cell virus promoter (MSCV), phosphoglycerate kinase-1 locus promoter (PGK) and mouse metallothionein-I. The promoter can be a fungi promoter. The promoter can be a plant promoter. A database of plant promoters can be found (e.g., PlantProm). The expression vector may also contain a ribosome binding site for translation initiation and a transcription terminator. The expression vector may also comprise suitable sequences for amplifying expression.

In various embodiments of the aspects herein, modified immune cells can specifically bind a neoantigen and/or a neoepitope. Neoantigens and neoepitopes generally refer to tumor-specific mutations that in some cases trigger an antitumor T cell response. For example, these endogenous mutations can be identified using a whole-exomic sequencing approach. Tran E, et al., "Cancer immunotherapy based on mutation-specific CD4+ T cells in a patient with epithelial cancer," Science 344: 641-644 (2014). A modified immune cell (e.g., modified TIL or modified T cell) comprising an enhanced receptor can exhibit specific binding to a tumor-specific neoantigen. Neoantigens bound by the immune cell can be expressed on a target cell, and for example, are encoded by mutations in an endogenous gene. In some cases, a neoantigen or neoepitope specifically bound by the immune cell can be encoded by a mutated gene. The gene can be selected from the group consisting of: ABL1, ACOl 1997, ACVR2A, AFP, AKT1, ALK, ALPPL2, ANAPC1, APC, ARID1A, AR, AR-v7, ASCL2, β2M, BRAF, BTK, C15ORF40, CDH1, CLDN6, CNOT1, CT45A5, CTAG1B (encoding NY-ESO-1), DCT, DKK4, EEF1B2, EEF1DP3, EGFR, EIF2B3, env, EPHB2, ERBB3, ESR1, ESRP1, FAM11 IB, FGFR3, FRG1B, GAGE1, GAGE 10, GATA3, GBP3, HER2, IDH1, JAK1, KIT, KRAS, LMAN1, MABEB 16, MAGEA1, MAGEA10, MAGEA4, MAGEA8, MAGEB 17, MAGEB4, MAGEC1, MEK, MLANA, MLL2, MMP13, MSH3, MSH6, MYC, NDUFC2, NRAS, NY-ESO, PAGE2, PAGE5, PDGFRa, PIK3CA, PMEL, pol protein, POLE, PTEN, RAC1, RBM27, RNF43, RPL22, RUNX1, SEC31A, SEC63, SF3B 1, SLC35F5, SLC45A2, SMAP1, SMAP1, SPOP, TFAM, TGFBR2, THAP5, TP53, TTK, TYR, UBR5, VHL and XPOT. In some embodiments, the neoantigen is selected based on a genetic profile of a tumor sample from an individual. In some embodiments, the neoantigen may be selected based on a somatic mutation profile of a tumor sample from an individual.

In various embodiments of the aspects herein, the modified immune cell further comprises a kill switch (or called suicide switch). A kill switch can be activated to eliminate the immune cell in cases of severe toxicity, such as hypercytokinemia. This can happen when the immune system has such a strong response that many inflammatory cytokines are released, triggering mild to severe symptoms including fever, headache, rash, rapid heartbeat, low blood pressure, and trouble breathing. The kill switch can be a drug-inducible kill switch. The kill switch can comprise an inducible caspase 9.

Various embodiments of the aspects herein comprise a cell, for example a modified immune cell. Cells, for example immune cells (e.g., lymphocytes including T cells and NK cells), can be obtained from a subject. Non-limiting examples of subjects include humans, dogs, cats, mice, rats, and transgenic species thereof. Examples of samples from a subject from which cells can be derived include, without limitation, skin, heart, lung, kidney, bone marrow, breast, pancreas, liver, muscle, smooth muscle, bladder, gall bladder, colon, intestine, brain, prostate, esophagus, thyroid, serum, saliva, urine, gastric and digestive fluid, tears, stool, semen, vaginal fluid, interstitial fluids derived from tumorous tissue, ocular fluids, sweat, mucus, earwax, oil, glandular secretions, spinal fluid, hair, fingernails, plasma, nasal swab or nasopharyngeal wash, spinal fluid, cerebral spinal fluid, tissue, throat swab, biopsy, placental fluid, amniotic fluid, cord blood, emphatic fluids, cavity fluids, sputum, pus, microbiota, meconium, breast milk, and/or other excretions or body tissues.

In some cases, the cell can be a population of T cells, NK cells, B cells, and the like obtained from a subject. T cells can be obtained from a number of sources, including PBMCs, bone marrow, lymph node tissue, cord blood, thymus tissue, and tissue from a site of infection, ascites, pleural effusion, spleen tissue and tumors. In some embodiments, T cells can be obtained from a unit of blood collected from a subject using any number of techniques, such as FicollTM separation. In one embodiment, cells from the circulating blood of an individual are obtained by apheresis. The apheresis product typically contains lymphocytes, including T cells, monocytes, granulocytes, B cells, other nucleated white blood cells, red blood cells and platelets. The cells collected by apheresis may be washed to remove the plasma fraction and to place the cells in a suitable buffer or medium for subsequent processing steps.

Any of a variety of immune cells can be utilized in the aspects herein. In some embodiments, immune cells comprise granulocytes such as basophils, eosinophils and neutrophils; mast cells; monocytes which can develop into macrophages; antigen-presenting cells such as dendritic cells; and lymphocytes such as natural killer cells (NK cells), B cells and T cells. In some embodiments, the immune cell is an immune effector cell. An immune effector cell refers to an immune cell that can perform a specific function in response to a stimulus. In some embodiments, the immune cell is an immune effector cell which can induce cell death. In some embodiments, the immune cell is a lymphocyte. In some embodiments, the lymphocyte is an NK cell. In some embodiments, the lymphocyte is a T cell. In some embodiments, the T cell is an activated T cell. T cells include both naive and memory cells (e.g., central memory or TCM, effector memory or TEM and effector memory RA or TEMRA), effector cells (e.g., cytotoxic T cells or CTLs or Tc cells), helper cells (e.g., Th1, Th2, Th3, Th9, Th7 and TFH), regulatory cells (e.g., Treg and Trl cells), natural killer T cells (NKT cells), tumor-infiltrating lymphocytes (TILs), lymphocyte-activated killer cells (LAKs), αβ T cells, γδ T cells, and similar unique classes of the T cell lineage. T cells can be divided into two broad categories: CD8+ T cells and CD4+ T cells, based on which protein is present on the cell's surface. T cells expressing a subject system can carry out multiple functions, including killing infected cells and activating or recruiting other immune cells. CD8+ T cells are referred to as cytotoxic T cells or cytotoxic T lymphocytes (CTLs). CTLs expressing a subject system can be involved in recognizing and removing virus-infected cells and cancer cells. CTLs have specialized compartments or granules, containing cytotoxins that cause apoptosis, e.g., programmed cell death. CD4+ T cells can be subdivided into four subsets - Th1, Th2, Th17 and Treg, with "Th" referring to "T helper cell," although additional subsets may exist. Th1 cells can coordinate immune responses against intracellular microbes, especially bacteria. They can produce and secrete molecules that alert and activate other immune cells, like bacteria-ingesting macrophages. Th2 cells are involved in coordinating immune responses against extracellular pathogens, like helminths (parasitic worms), by alerting B cells, granulocytes, and mast cells. Th17 cells can produce interleukin 17 (IL-17), a signaling molecule that activates immune and non-immune cells. Th17 cells are important for recruiting neutrophils.

In some embodiments, the population of immune cells provided herein can be heterogeneous. In some embodiments, the cells used can be composed of a heterogeneous mixture of CD4 and CD8 T cells. The CD4 and CD8 cells can have phenotypic characteristics of circulating effector T cells. Said CD4 and CD8 cells can also have a phenotypic characteristic of effector memory cells. In some embodiment, the cells can be central memory cells.

In some embodiments, the cells include peripheral blood mononuclear cells (PBMCs), peripheral blood lymphocytes (PBLs), and other blood cell subsets such as, but not limited to, T cells, natural killer cells, monocytes, natural killer T cells, monocyte-precursor cells, hematopoietic stem cells or non-pluripotent stem cells. In some cases, the cell can be any immune cell, including any T cell such as tumor-infiltrating cells (TILs), such as CD3+ T cells, CD4+ T cells, CD8+ T cells, or any other type of T cell. The T cells can also include memory T cells, memory stem T cells, or effector T cells. The T cells can also be selected from a bulk population, for example, selecting T cells from whole blood. The T cells can also be expanded from a bulk population. The T cells can also be skewed towards particular populations and phenotypes. For example, the T cells can be skewed to phenotypically comprise, CD45RO (-), CCR7 (+), CD45RA (+), CD62L (+), CD27 (+), CD28 (+) and/or IL-7Rα (+). Suitable cells can be selected that comprise one or more markers selected from a list comprising: CD45RO (- ), CCR7 (+), CD45RA (+), CD62L (+), CD27 (+), CD28 (+) and/or IL-7Rα (+). Cells also include stem cells such as, by way of example, embryonic stem cells, induced pluripotent stem cells, hematopoietic stem cells, neuronal stem cells and mesenchymal stem cells. Cells can comprise any number of primary cells, such as human cells, non-human cells, and/or mouse cells. Cells can be progenitor cells. Cells can be derived from a subject to be treated (e.g., patient). Cells can be derived from a human donor. Host cells can be stem memory TSCM cells comprised of CD45RO (-), CCR7 (+), CD45RA (+), CD62L+ (L-selectin), CD27+, CD28+ and IL-7Rα+, and said stem memory cells can also express CD95, IL-2Rβ, CXCR3 and LFA-1, and show numerous functional attributes distinctive of said stem memory cells. Host cells can be central memory TCM cells comprising L-selectin and CCR7, and said central memory cells can secrete, for example, IL-2, but not IFNγ or IL-4. Cells can also be effector memory TEM cells comprising L-selectin or CCR7 and produce, for example, effector cytokines such as IFNγ and IL-4.

In various embodiments of the aspects herein, the immune cell comprises a lymphocyte. In some embodiments, the lymphocyte is a natural killer cell (NK cell). In some embodiments, the lymphocyte is a T cell. T cells can be obtained from a number of sources, including peripheral blood mononuclear cells, bone marrow, lymph node tissue, spleen tissue, umbilical cord and tumors. In some embodiments, any number of T cell lines available can be used. Immune cells such as lymphocytes (e.g., cytotoxic lymphocytes) can preferably be autologous cells, although heterologous cells can also be used. T cells can be obtained from a unit of blood collected from a subject using any number of techniques, such as Ficoll separation. Cells from the circulating blood of an individual can be obtained by apheresis or leukapheresis. The apheresis product typically contains lymphocytes, including T cells, monocytes, granulocytes, B cells, other nucleated white blood cells, red blood cells and platelets. The cells collected by apheresis can be washed to remove the plasma fraction and to place the cells in a suitable buffer or medium, such as phosphate buffered saline (PBS), for subsequent processing steps. After washing, the cells can be resuspended in a variety of biocompatible buffers, such as Ca-free, Mg-free PBS. Alternatively, the undesirable components of the apheresis sample can be removed and the cells are directly resuspended in culture media. Samples can be provided directly by the subject, or indirectly through one or more intermediaries, such as a sample collection service provider or a medical provider (e.g., a physician or nurse). In some embodiments, isolating T cells from peripheral blood leukocytes can include lysing the red blood cells and separating peripheral blood leukocytes from monocytes by, for example, centrifugation through, e.g., a PERCOL^{™} gradient.

A specific subpopulation of T cells, such as CD4+ or CD8+ T cells, can be further isolated by positive or negative selection techniques. Negative selection of a population of T cells can be accomplished, for example, with a combination of antibodies directed to surface markers unique to the cells negatively selected. One suitable technique includes cell sorting via negative magnetic immunoadherence, which utilizes a cocktail of monoclonal antibodies directed to cell surface markers present on the cells negatively selected. For example, to isolate CD4+ cells, a monoclonal antibody cocktail can include antibodies to CD14, CD20, CD11b, CD16, HLA-DR and CD8. The process of negative selection can be used to produce a desired population of T cells that is primarily homogeneous. In some embodiments, the composition comprises a mixture of two or more (e.g., 2, 3, 4, 5 or more) different kinds of T-cells.

In some embodiments, the immune cell is a member of an enriched population of cells. One or more desired cell types can be enriched by any suitable method, non-limiting examples of which include treating a population of cells to trigger expansion and/or differentiation to a desired cell type, treatment to stop the growth of undesired cell type(s), treatment to kill or lyse undesired cell type(s), purification of a desired cell type (e.g., purification on an affinity column to retain desired or undesired cell types on the basis of one or more cell surface markers). In some embodiments, the enriched population of cells is a population of cells enriched in cytotoxic lymphocytes selected from cytotoxic T cells (also variously known as cytotoxic T lymphocytes, CTLs, T killer cells, cytolytic T cells, CD8+ T cells, and killer T cells), natural killer (NK) cells, and lymphokine- activated killer (LAK) cells.

For isolation of a desired population of cells by positive or negative selection, the concentration of cells and surface (e.g., particles such as beads) can be varied. In certain embodiments, it can be desirable to significantly decrease the volume in which beads and cells are mixed together (i.e., increase the concentration of cells), to ensure maximum contact of cells and beads. For example, a concentration of 2 billion cells/mL can be used. In some embodiments, a concentration of 1 billion cells/mL is used. In some embodiments, greater than 100 million cells/mL are used. A concentration of cells of 10, 15, 20, 25, 30, 35, 40, 45, or 50 million cells/mL can be used. In another embodiment, a concentration of cells of 75, 80, 85, 90, 95, or 100 million cells/mL can be used. In further embodiments, a concentration of 125 or 150 million cells/mL can be used. Using high concentrations can result in increased cell yield, cell activation, and cell expansion.

A variety of target cells can be killed using the systems and methods of the subject disclosure. A target cell to which this method can be applied includes a wide variety of cell types. The target cell can be *in vitro.* The target cell can be *in vivo.* The target cell can be *ex vivo.* The target cell can be an isolated cell. The target cell can be a cell inside of an organism. The target cell can be an organism. The target cell can be a cell in a cell culture. The target cell can be one of a collection of cells. The target cell can be a mammalian cell or derived from a mammalian cell. The target cell can be a rodent cell or derived from a rodent cell. The target cell can be a human cell or derived from a human cell. The target cell can be a prokaryotic cell or derived from a prokaryotic cell. The target cell can be a bacterial cell or can be derived from a bacterial cell. The target cell can be an archaeal cell or derived from an archaeal cell. The target cell can be a eukaryotic cell or derived from a eukaryotic cell. The target cell can be a pluripotent stem cell. The target cell can be a plant cell or derived from a plant cell. The target cell can be an animal cell or derived from an animal cell. The target cell can be an invertebrate cell or derived from an invertebrate cell. The target cell can be a vertebrate cell or derived from a vertebrate cell. The target cell can be a microbe cell or derived from a microbe cell. The target cell can be a fungi cell or derived from a fungi cell. The target cell can be from a specific organ or tissue.

The target cell can be a stem cell or progenitor cell. Target cells can include stem cells (e.g., adult stem cells, embryonic stem cells, and induced pluripotent stem (iPS) cells) and progenitor cells (e.g., cardiac progenitor cells, neural progenitor cells, etc.). Target cells can include mammalian stem cells and progenitor cells, including rodent stem cells, rodent progenitor cells, human stem cells, human progenitor cells, etc. Clonal cells can comprise the progeny of a cell. The target cell can comprise a target nucleic acid. The target cell can be in a living organism. The target cell can be a genetically modified cell. The target cell can be a host cell.

The target cell can be a primary cell. For example, cultures of primary cells can be passaged 0 times, 1 time, 2 times, 4 times, 5 times, 10 times, 15 times or more. Cells can be unicellular organisms. Cells can be grown in culture.

The target cell can be a diseased cell. A diseased cell can have altered metabolic, gene expression, and/or morphologic features. The diseased cell can be a cancer cell, a diabetic cell, and an apoptotic cell. The diseased cell can be a cell from a diseased subject. Exemplary diseases can include blood disorders, cancers, metabolic disorders, eye disorders, organ disorders, musculoskeletal disorders, cardiac disease, etc.

If the target cells are primary cells, they may be harvested from an individual by any method. For example, leukocytes may be harvested by apheresis, leukocytapheresis, density gradient separation, etc. Cells from tissues such as skin, muscle, bone marrow, spleen, liver, pancreas, lung, intestine, stomach, and the like can be harvested by biopsy. A suitable solution may be used for dispersion or suspension of the harvested cells. Such solution can generally be a balanced salt solution (e.g., normal saline, phosphate-buffered saline (PBS), Hank's balanced salt solution, etc.), conveniently supplemented with fetal calf serum or other naturally occurring factors, in conjunction with an acceptable buffer at a low concentration. Buffers can include HEPES, phosphate buffers, lactate buffers, etc. Cells may be used immediately, or they may be stored (e.g., by freezing). Frozen cells can be thawed and can be capable of being reused. Cells can be frozen in DMSO, serum, medium buffer (e.g., 10% DMSO, 50% serum, 40% buffered medium), and/or some other such common solutions used to preserve cells at freezing temperatures.

Non-limiting examples of cells which can be target cells include, but are not limited to, lymphoid cells, such as B cell, T cell (Cytotoxic T cell, Natural Killer T cell, Regulatory T cell, T helper cell), Natural killer cell, cytokine induced killer (CIK) cells (see e.g. US20080241194); myeloid cells, such as granulocytes (Basophil granulocyte, Eosinophil granulocyte, Neutrophil granulocyte/Hypersegmented neutrophil), Monocyte/Macrophage, Red blood cell (Reticulocyte), Mast cell, Thrombocyte/Megakaryocyte, Dendritic cell; cells from the endocrine system, including thyroid (Thyroid epithelial cell, Parafollicular cell), parathyroid (Parathyroid chief cell, Oxyphil cell), adrenal (Chromaffin cell), pineal (Pinealocyte) cells; cells of the nervous system, including glial cells (Astrocyte, Microglia), Magnocellular neurosecretory cell, Stellate cell, Boettcher cell, and pituitary (Gonadotrope, Corticotrope, Thyrotrope, Somatotrope, Lactotroph); cells of the Respiratory system, including Pneumocyte (Type I pneumocyte, Type II pneumocyte), Clara cell, Goblet cell, Dust cell; cells of the circulatory system, including Myocardiocyte, Pericyte; cells of the digestive system, including stomach (Gastric chief cell, Parietal cell), Goblet cell, Paneth cell, G cells, D cells, ECL cells, I cells, K cells, S cells; enteroendocrine cells, including enterochromaffin cell, APUD cell, liver (Hepatocyte, Kupffer cell), Cartilage/bone/muscle; bone cells, including Osteoblast, Osteocyte, Osteoclast, teeth (Cementoblast, Ameloblast); cartilage cells, including Chondroblast, Chondrocyte; skin cells, including Trichocyte, Keratinocyte, Melanocyte (Nevus cell); muscle cells, including Myocyte; urinary system cells, including Podocyte, Juxtaglomerular cell, Intraglomerular mesangial cell/Extraglomerular mesangial cell, Kidney proximal tubule brush border cell, Macula densa cell; reproductive system cells, including Spermatozoon, Sertoli cell, Leydig cell, Ovum; and other cells, including Adipocyte, Fibroblast, Tendon cell, Epidermal keratinocyte (differentiating epidermal cell), Epidermal basal cell (stem cell), Keratinocyte of fingernails and toenails, Nail bed basal cell (stem cell), Medullary hair shaft cell, Cortical hair shaft cell, Cuticular hair shaft cell, Cuticular hair root sheath cell, Hair root sheath cell of Huxley's layer, Hair root sheath cell of Henle's layer, External hair root sheath cell, Hair matrix cell (stem cell), Wet stratified barrier epithelial cells, Surface epithelial cell of stratified squamous epithelium of cornea, tongue, oral cavity, esophagus, anal canal, distal urethra and vagina, basal cell (stem cell) of epithelia of cornea, tongue, oral cavity, esophagus, anal canal, distal urethra and vagina, Urinary epithelium cell (lining urinary bladder and urinary ducts), Exocrine secretory epithelial cells, Salivary gland mucous cell (polysaccharide-rich secretion), Salivary gland serous cell (glycoprotein enzyme-rich secretion), Von Ebner's gland cell in tongue (washes taste buds), Mammary gland cell (milk secretion), Lacrimal gland cell (tear secretion), Ceruminous gland cell in ear (wax secretion), Eccrine sweat gland dark cell (glycoprotein secretion), Eccrine sweat gland clear cell (small molecule secretion). Apocrine sweat gland cell (odoriferous secretion, sex-hormone sensitive), Gland of Moll cell in eyelid (specialized sweat gland), Sebaceous gland cell (lipid-rich sebum secretion), Bowman's gland cell in nose (washes olfactory epithelium), Brunner's gland cell in duodenum (enzymes and alkaline mucus), Seminal vesicle cell (secreting seminal fluid components, including fructose for swimming sperm), Prostate gland cell (secreting seminal fluid components), Bulbourethral gland cell (mucus secretion), Bartholin's gland cell (vaginal lubricant secretion), Gland of Littre cell (mucus secretion), Uterus endometrium cell (carbohydrate secretion), Isolated goblet cell of respiratory and digestive tracts (mucus secretion), Stomach lining mucous cell (mucus secretion), Gastric gland zymogenic cell (pepsinogen secretion), Gastric gland oxyntic cell (hydrochloric acid secretion), Pancreatic acinar cell (bicarbonate and digestive enzyme secretion), Paneth cell of small intestine (lysozyme secretion), Type II pneumocyte of lung (surfactant secretion), Clara cell of lung, Hormone secreting cells, Anterior pituitary cells, Somatotropes, Lactotropes, Thyrotropes, Gonadotropes, Corticotropes, Intermediate pituitary cell, Magnocellular neurosecretory cells, Gut and respiratory tract cells, Thyroid gland cells, thyroid epithelial cell, parafollicular cell, Parathyroid gland cells, Parathyroid chief cell, Oxyphil cell, Adrenal gland cells, chromaffin cells, Ley dig cell of testes, Theca interna cell of ovarian follicle, Corpus luteum cell of ruptured ovarian follicle, Granulosa lutein cells, Theca lutein cells, Juxtaglomerular cell (renin secretion), Macula densa cell of kidney, Metabolism and storage cells, Barrier function cells (Lung, Gut, Exocrine Glands and Urogenital Tract), Kidney, Type I pneumocyte (lining air space of lung), Pancreatic duct cell (centroacinar cell), Nonstriated duct cell (of sweat gland, salivary gland, mammary gland, etc.), Duct cell (of seminal vesicle, prostate gland, etc.), Epithelial cells lining closed internal body cavities, Ciliated cells with propulsive function, Extracellular matrix secretion cells, Contractile cells; Skeletal muscle cells, stem cell, Heart muscle cells, Blood and immune system cells, Erythrocyte (red blood cell), Megakaryocyte (platelet precursor), Monocyte, Connective tissue macrophage (various types), Epidermal Langerhans cell, Osteoclast (in bone), Dendritic cell (in lymphoid tissues), Microglial cell (in central nervous system), Neutrophil granulocyte, Eosinophil granulocyte, Basophil granulocyte, Mast cell, Helper T cell, Suppressor T cell, Cytotoxic T cell, Natural Killer T cell, B cell, Natural killer cell, Reticulocyte, Stem cells and committed progenitors for the blood and immune system (various types), Pluripotent stem cells, Totipotent stem cells, Induced pluripotent stem cells, adult stem cells, Sensory transducer cells, Autonomic neuron cells, Sense organ and peripheral neuron supporting cells, Central nervous system neurons and glial cells, Lens cells, Pigment cells, Melanocyte, Retinal pigmented epithelial cell, Germ cells, Oogonium/Oocyte, Spermatid, Spermatocyte, Spermatogonium cell (stem cell for spermatocyte), Spermatozoon, Nurse cells, Ovarian follicle cell, Sertoli cell (in testis), Thymus epithelial cell, Interstitial cells, and Interstitial kidney cells, or processed or irradiated non-human cells, such as K562, NK92, etc. The target cell can be a natural cell or a modified cell.

Of particular interest are cancer cells. In some embodiments, the target cell is a cancer cell. Non-limiting examples of cancer cells include cells of cancers including Acanthoma, Acinic cell carcinoma, Acoustic neuroma, Acral lentiginous melanoma, Acrospiroma, Acute eosinophilic leukemia, Acute lymphoblastic leukemia, Acute megakaryoblastic leukemia, Acute monocytic leukemia, Acute myeloblastic leukemia with maturation, Acute myeloid dendritic cell leukemia, Acute myeloid leukemia, Acute promyelocytic leukemia, Adamantinoma, Adenocarcinoma, Adenoid cystic carcinoma, Adenoma, Adenomatoid odontogenic tumor, Adrenocortical carcinoma, Adult T-cell leukemia, Aggressive NK-cell leukemia, AIDS-Related Cancers, AIDS-related lymphoma, Alveolar soft part sarcoma, Ameloblastic fibroma, Anal cancer, Anaplastic large cell lymphoma, Anaplastic thyroid cancer, Angioimmunoblastic T-cell lymphoma, Angiomyolipoma, Angiosarcoma, Appendix cancer, Astrocytoma, Atypical teratoid rhabdoid tumor, Basal cell carcinoma, Basal-like carcinoma, B-cell leukemia, B-cell lymphoma, Bellini duct carcinoma, Biliary tract cancer, Bladder cancer, Blastoma, Bone Cancer, Bone tumor, Brain Stem Glioma, Brain Tumor, Breast Cancer, Brenner tumor, Bronchial Tumor, Bronchioloalveolar carcinoma, Brown tumor, Burkitt's lymphoma, Cancer of Unknown Primary Site, Carcinoid Tumor, Carcinoma, Carcinoma in situ, Carcinoma of the penis, Carcinoma of Unknown Primary Site, Carcinosarcoma, Castleman's Disease, Central Nervous System Embryonal Tumor, Cerebellar Astrocytoma, Cerebral Astrocytoma, Cervical Cancer, Cholangiocarcinoma, Chondroma, Chondrosarcoma, Chordoma, Choriocarcinoma, Choroid plexus papilloma, Chronic Lymphocytic Leukemia, Chronic monocytic leukemia, Chronic myelogenous leukemia, Chronic Myeloproliferative Disorder, Chronic neutrophilic leukemia, Clear-cell tumor, Colon Cancer, Colorectal cancer, Craniopharyngioma, Cutaneous T-cell lymphoma, Degos disease, Dermatofibrosarcoma protuberans, Dermoid cyst, Desmoplastic small round cell tumor, Diffuse large B cell lymphoma, Dysembryoplastic neuroepithelial tumor, Embryonal carcinoma, Endodermal sinus tumor, Endometrial cancer, Endometrial Uterine Cancer, Endometrioid tumor, Enteropathy-associated T-cell lymphoma, Ependymoblastoma, Ependymoma, Epithelioid sarcoma, Erythroleukemia, Esophageal cancer, Esthesioneuroblastoma, Ewing Family of Tumor, Ewing Family Sarcoma, Ewing's sarcoma, Extracranial Germ Cell Tumor, Extragonadal Germ Cell Tumor, Extrahepatic Bile Duct Cancer, Extramammary Paget's disease, Fallopian tube cancer, Fetus in fetu, Fibroma, Fibrosarcoma, Follicular lymphoma, Follicular thyroid cancer, Gallbladder Cancer, Gallbladder cancer, Ganglioglioma, Ganglioneuroma, Gastric Cancer, Gastric lymphoma, Gastrointestinal cancer, Gastrointestinal Carcinoid Tumor, Gastrointestinal Stromal Tumor, Gastrointestinal stromal tumor, Germ cell tumor, Germinoma, Gestational choriocarcinoma, Gestational Trophoblastic Tumor, Giant cell tumor of bone, Glioblastoma multiforme, Glioma, Gliomatosis cerebri, Glomus tumor, Glucagonoma, Gonadoblastoma, Granulosa cell tumor, Hairy Cell Leukemia, Hairy cell leukemia, Head and Neck Cancer, Head and neck cancer, Heart cancer, Hemangioblastoma, Hemangiopericytoma, Hemangiosarcoma, Heart cancer, Hemangioblastoma, Hemangiopericytoma, Hemangiosarcoma, Hematological malignancy, Hepatocellular carcinoma, Hepatosplenic T-cell lymphoma, Hereditary breast-ovarian cancer syndrome, Hodgkin Lymphoma, Hodgkin's lymphoma, Hypopharyngeal Cancer, Hypothalamic Glioma, Inflammatory breast cancer, Intraocular Melanoma, Islet cell carcinoma, Islet Cell Tumor, Juvenile myelomonocytic leukemia, Kaposi Sarcoma, Kaposi's sarcoma, Kidney Cancer, Klatskin tumor, Krukenberg tumor, Laryngeal Cancer, Laryngeal cancer, Lentigo maligna melanoma, Leukemia, Leukemia, Lip and Oral Cavity Cancer, Liposarcoma, Lung cancer, Luteoma, Lymphangioma, Lymphangiosarcoma, Lymphoepithelioma, Lymphoid leukemia, Lymphoma, Macroglobulinemia, Malignant Fibrous Histiocytoma, Malignant fibrous histiocytoma, Malignant Fibrous Histiocytoma of Bone, Malignant Glioma, Malignant Mesothelioma, Malignant peripheral nerve sheath tumor, Malignant rhabdoid tumor, Malignant triton tumor, MALT lymphoma, Mantle cell lymphoma, Mast cell leukemia, Mediastinal germ cell tumor, Mediastinal tumor, Medullary thyroid cancer, Medulloblastoma, Medulloblastoma, Medulloepithelioma, Melanoma, Melanoma, Meningioma, Merkel Cell Carcinoma, Mesothelioma, Mesothelioma, Metastatic Squamous Neck Cancer with Occult Primary, Metastatic urothelial carcinoma, Mixed Mullerian tumor, Monocytic leukemia, Mouth Cancer, Mucinous tumor, Multiple Endocrine Neoplasia Syndrome, Multiple Myeloma, Multiple myeloma, Mycosis Fungoides, Mycosis fungoides, Myelodysplastic Disease, Myelodysplastic Syndromes, Myeloid leukemia, Myeloid sarcoma, Myeloproliferative Disease, Myxoma, Nasal Cavity Cancer, Nasopharyngeal Cancer, Nasopharyngeal carcinoma, Neoplasm, Neurinoma, Neuroblastoma, Neuroblastoma, Neurofibroma, Neuroma, Nodular melanoma, Non-Hodgkin Lymphoma, Non-Hodgkin lymphoma, Nonmelanoma Skin Cancer, Non-Small Cell Lung Cancer, Ocular oncology, Oligoastrocytoma, Oligodendroglioma, Oncocytoma, Optic nerve sheath meningioma, Oral Cancer, Oral cancer, Oropharyngeal Cancer, Osteosarcoma, Osteosarcoma, Ovarian Cancer, Ovarian cancer, Ovarian Epithelial Cancer, Ovarian Germ Cell Tumor, Ovarian Low Malignant Potential Tumor, Paget's disease of the breast, Pancoast tumor, Pancreatic Cancer, Pancreatic cancer, Papillary thyroid cancer, Papillomatosis, Paraganglioma, Paranasal Sinus Cancer, Parathyroid Cancer, Penile Cancer, Perivascular epithelioid cell tumor, Pharyngeal Cancer, Pheochromocytoma, Pineal Parenchymal Tumor of Intermediate Differentiation, Pineoblastoma, Pituicytoma, Pituitary adenoma, Pituitary tumor, Plasma Cell Neoplasm, Pleuropulmonary blastoma, Polyembryoma, Precursor T-lymphoblastic lymphoma, Primary central nervous system lymphoma, Primary effusion lymphoma, Primary Hepatocellular Cancer, Primary Liver Cancer, Primary peritoneal cancer, Primitive neuroectodermal tumor, Prostate cancer, Pseudomyxoma peritonei, Rectal Cancer, Renal cell carcinoma, Respiratory Tract Carcinoma Involving the NUT Gene on Chromosome 15, Retinoblastoma, Rhabdomyoma, Rhabdomyosarcoma, Richter's transformation, Sacrococcygeal teratoma, Salivary Gland Cancer, Sarcoma, Schwannomatosis, Sebaceous gland carcinoma, Secondary neoplasm, Seminoma, Serous tumor, Sertoli-Leydig cell tumor, Sex cord-stromal tumor, Sezary Syndrome, Signet ring cell carcinoma, Skin Cancer, Small blue round cell tumor, Small cell carcinoma, Small Cell Lung Cancer, Small cell lymphoma, Small intestine cancer, Soft tissue sarcoma, Somatostatinoma, Soot wart, Spinal Cord Tumor, Spinal tumor, Splenic marginal zone lymphoma, Squamous cell carcinoma, Stomach cancer, Superficial spreading melanoma, Supratentorial Primitive Neuroectodermal Tumor, Surface epithelial-stromal tumor, Synovial sarcoma, T-cell acute lymphoblastic leukemia, T-cell large granular lymphocyte leukemia, T-cell leukemia, T-cell lymphoma, T-cell prolymphocytic leukemia, Teratoma, Terminal lymphatic cancer, Testicular cancer, Thecoma, Throat Cancer, Thymic Carcinoma, Thymoma, Thyroid cancer, Transitional Cell Cancer of Renal Pelvis and Ureter, Transitional cell carcinoma, Urachal cancer, Urethral cancer, Urogenital neoplasm, Uterine sarcoma, Uveal melanoma, Vaginal Cancer, Verner Morrison syndrome, Verrucous carcinoma, Visual Pathway Glioma, Vulvar Cancer, Waldenstrom's macroglobulinemia, Warthin's tumor, Wilms' tumor, and combinations thereof. In some embodiments, the targeted cancer cell represents a subpopulation within a population of cancer cells, such as cancer stem cells. In some embodiments, the cancer is of a hematopoietic lineage, such as a lymphoma. The antigen can be a tumor-associated antigen.

In some embodiments, the target cells form a tumor. A tumor treated with the methods herein can result in stabilized tumor growth (e.g., one or more tumors do not increase more than 1%, 5%, 10%, 15%, or 20% in size, and/or do not metastasize). In some embodiments, the tumor is stabilized for at least about 1, 2, 3, 4, 5, 6, 7, 8, 9, 10, 11, 12, or more weeks. In some embodiments, the tumor is stabilized for at least about 1, 2, 3, 4, 5, 6, 7, 8, 9, 10, 11, 12, or more months. In some embodiments, the tumor is stabilized for at least about 1, 2, 3, 4, 5, 6, 7, 8, 9, 10, or more years. In some embodiments, the size of the tumor or the number of tumor cells is reduced by at least about 5%, 10%, 15%, 20%, 25%, 30%, 35%, 40%, 45%, 50%, 55%, 60%, 65%, 70%, 75%, 80%, 85%, 90%, 95% or more. In some embodiments, the tumor is completely eliminated, or reduced below a level of detection. In some embodiments, the subject remains tumor free (e.g., in remission) for at least about 1, 2, 3, 4, 5, 6, 7, 8, 9, 10, 11, 12, or more weeks following treatment. In some embodiments, the subject remains tumor free for at least about 1, 2, 3, 4, 5, 6, 7, 8, 9, 10, 11, 12, or more months following treatment. In some embodiments, the subject remains tumor free for at least about 1, 2, 3, 4, 5, 6, 7, 8, 9, 10, or more years after treatment.

Death of target cells can be determined by any suitable method, including, but not limited to, counting cells before and after treatment, or measuring the level of a marker associated with live or dead cells (e.g., live or dead target cells). Degree of cell death can be determined by any suitable method. In some embodiments, degree of cell death is determined with respect to the starting condition. For example, an individual can have a known starting amount of target cells, such as a starting cell mass of known size or circulating target cells at a known concentration. In such cases, degree of cell death can be expressed as a ratio of surviving cells after treatment to the starting population of cells. In some embodiments, degree of cell death can be determined by a suitable cell death assay. A variety of cell death assays are available, and can utilize a variety of detection methodologies. Examples of detection methodologies include, without limitation, the use of cell staining, microscopy, flow cytometry, cell sorting, and combinations of these.

When a tumor is subject to surgical resection following completion of a therapeutic period, the efficacy of treatment in reducing tumor size can be determined by measuring the percentage of resected tissue that is necrotic (i.e., dead). In some embodiments, the treatment is therapeutically effective if the necrosis percentage of the resected tissue is greater than about 20% (e.g., at least about 30%, 40%, 50%, 60%, 70%, 80%, 90% or 100%). In some embodiments, the necrosis percentage of the resected tissue is 100%, that is, no living tumor tissue is present or detectable.

Exposing a target cell to the immune cell or population of immune cells disclosed herein can be conducted either *in vitro* or *in vivo.* Exposing a target cell to an immune cell or population of immune cells generally refers to bringing the target cell in contact with the immune cell and/or in sufficient proximity such that an antigen of the target cell (e.g., membrane-bound or non-membrane-bound) can bind to the enhanced receptor expressed in the immune cell. Exposing a target cell to an immune cell or population of immune cells also generally refers to bringing the target cell in contact with the immune cell and/or in sufficient proximity such that an antigen of the target cell (e.g., membrane-bound or non-membrane-bound) can bind to the CAR expressed in the immune cell. Exposing a target cell to an immune cell or population of immune cells *in vitro* can be accomplished by co-culturing the target cells and the immune cells. Target cells and immune cells can be co-cultured, for example, as adherent cells or alternatively in suspension. Target cells and immune cells can be co-cultured in various suitable types of cell culture media, for example with supplements, growth factors, ions, etc. Exposing a target cell to an immune cell or population of immune cells *in vivo* can be accomplished, in some cases, by administering the immune cell to a subject, for example a human subject, and allowing the immune cell to localize to the target cell via the circulatory system. In some cases, an immune cell can be delivered to the immediate area where a target cell is localized, for example, by direct injection.

Exposing can be performed for any suitable length of time, for example at least 1 minute, at least 5 minutes, at least 10 minutes, at least 30 minutes, at least 1 hour, at least 2 hours, at least 3 hours, at least 4 hours, at least 5 hours, at least 6 hours, at least 7 hours, at least 8 hours, at least 12 hours, at least 16 hours, at least 20 hours, at least 24 hours, at least 2 days, at least 3 days, at least 4 days, at least 5 days, at least 6 days, at least 1 week, at least 2 weeks, at least 3 weeks, at least 1 month or longer.

The various domains of enhanced receptors and CARs provided herein can be linked by means of a chemical bond, e.g., an amide bond or a disulfide bond; a small, organic molecule (e.g., a hydrocarbon chain); an amino acid sequence such as a peptide linker (e.g., an amino acid sequence about 3-200 amino acids in length), or a combination of a small, organic molecule and a peptide linker. Peptide linkers can provide desirable flexibility to permit the desired expression, activity and/or conformational positioning of the chimeric polypeptide. The peptide linker can be of any suitable length to connect at least two domains of interest and is preferably designed to be sufficiently flexible so as to allow the proper folding and/or function and/or activity of one or both of the domains it connects. The peptide linker can have a length of at least 3, 5, 10, 15, 20, 25, 30, 35, 40, 45, 50, 55, 60, 65, 70, 75, 80, 85, 90, 95, or 100 amino acids. In some embodiments, the peptide linker has a length of about 0 to 200 amino acids, about 10 to 190 amino acids, about 20 to 180 amino acids, about 30 to 170 amino acids, about 40 to 160 amino acids, about 50 to 150 amino acids, about 60 to 140 amino acids, about 70 to 130 amino acids, about 80 to 120 amino acids, or about 90 to 110 amino acids. In some embodiments, the linker sequence can comprise an endogenous protein sequence. In some embodiments, the linker sequence comprises glycine, alanine and/or serine amino acid residues. In some embodiments, the linker can contain motifs, e.g., multiple or repeating motifs, of GS, GGS, GGGGS, GGSG or SGGG. The linker sequence can comprise any naturally occurring amino acids, non-naturally occurring amino acids, or combinations thereof.

Any suitable delivery method can be used for introducing compositions and molecules (e.g., polypeptides and/or nucleic acids encoding polypeptides) of the present disclosure into a host cell, such as an immune cell. The various components can be delivered simultaneously or temporally separated. The choice of method can be dependent on the type of cell being transformed and/or the circumstances under which the transformation is taking place (e.g., *in vitro*, *ex vivo*, or *in vivo*).

A method of delivery can involve contacting a target polynucleotide or introducing into a cell (or a population of cells such as immune cells) one or more nucleic acids comprising nucleotide sequences encoding the compositions of the present invention. Suitable nucleic acids comprising nucleotide sequences encoding the compositions of the present disclosure can include expression vectors, where an expression vector comprising a nucleotide sequence encoding one or more compositions of the present disclosure is a recombinant expression vector.

Non-limiting examples of delivery methods or transformation include, for example, viral or bacteriophage infection, transfection, conjugation, protoplast fusion, lipofection, electroporation, calcium phosphate precipitation, polyethyleneimine (PEI)-mediated transfection, DEAE-dextran-mediated transfection, liposome-mediated transfection, particle gun technology, calcium phosphate precipitation, direct micro injection, and nanoparticle-mediated nucleic acid delivery.

In some aspects, the present disclosure provides methods comprising delivering one or more polynucleotides, or one or more vectors as described herein, or one or more transcripts thereof, and/or one or more proteins transcribed therefrom, to a host cell. In some aspects, the present disclosure further provides cells produced by such methods, and organisms (such as animals, plants or fungi) comprising or produced from such cells.

Conventional viral and non-viral based gene transfer methods can be used to introduce nucleic acids in mammalian cells or target tissues. Such methods can be used to administer nucleic acids encoding compositions of the present disclosure to cells in culture, or in a host organism. Non-viral vector delivery systems can include DNA plasmids, RNA (e.g., a transcript of the vector described herein), naked nucleic acid, and nucleic acid complexed with a delivery vehicle, such as a liposome. Viral vector delivery systems can include DNA and RNA viruses, which can have either episomal or integrated genomes after delivery to a cell.

Methods of non-viral delivery of nucleic acids can include lipofection, nucleofection, microinjection, biolistics, virosomes, liposomes, immunoliposomes, polycation or lipid:nucleic acid conjugates, naked DNA, artificial virions, and agent-enhanced uptake of DNA. Cationic and neutral lipids that are suitable for efficient receptor-recognition lipofection of polynucleotides can be used. Delivery can be to cells (e.g., *in vitro* or *ex vivo* administration) or target tissues (e.g., *in vivo* administration). The preparation of lipid:nucleic acid complexes, including targeted liposomes such as immunolipid complexes, can be used.

RNA or DNA viral based systems can be used to target specific cells in the body and trafficking the viral payload to the nucleus of the cell. Viral vectors can be administered directly (*in vivo*) or they can be used to treat cells *in vitro,* and the modified cells can optionally be administered (*ex vivo*). Viral based systems can include retroviral, lentivirus, adenoviral, adeno-associated and herpes simplex virus vectors for gene transfer. Integration in the host genome can occur with the retrovirus, lentivirus, and adeno-associated virus gene transfer methods, which can result in long term expression of the inserted transgene. High transduction efficiencies can be observed in many different cell types and target tissues.

Lentiviruses can integrate their genomes into host cells (such as 293 cells, or T cells). Lentiviruses can use a three-plasmid system, or a four-plasmid system.

The tropism of a retrovirus can be altered by incorporating foreign envelope proteins, expanding a potential target population of target cells. Lentiviral vectors are retroviral vectors that can transduce or infect non-dividing cells and produce high viral titers. Selection of a retroviral gene transfer system can depend on the target tissue. Retroviral vectors can comprise cis-acting long terminal repeats with packaging capacity for up to 6-10 kb of foreign sequence. The minimum cis-acting LTRs can be sufficient for replication and packaging of the vectors, which can be used to integrate the therapeutic gene into the target cell to provide permanent transgene expression. Retroviral vectors can include those based upon murine leukemia virus (MuLV), gibbon ape leukemia virus (GaLV), Simian Immuno deficiency virus (SIV), human immuno deficiency virus (HIV), and combinations thereof.

Adenoviral based systems can be used. Adenoviral based systems can lead to transient expression of the transgene. Adenoviral based vectors can have high transduction efficiency in cells and may not require cell division. High titers and levels of expression can be obtained with adenoviral based vectors. Adeno-associated virus ("AAV") vectors can be used to transduce cells with target nucleic acids, e.g., in the *in vitro* production of nucleic acids and peptides, and for *in vivo* and *ex vivo* gene therapy procedures.

Packaging cells can be used to form virus particles capable of infecting a host cell. Such cells can include 293 cells (e.g., for packaging lentivirus or adenovirus), and Psi2 cells or PA317 cells (e.g., for packaging retrovirus). Viral vectors can be generated by producing a cell line that packages a nucleic acid vector into a viral particle. The vectors can contain the minimal viral sequences required for packaging and subsequent integration into a host. The vectors can contain other viral sequences being replaced by an expression cassette for the polynucleotide(s) to be expressed. The missing viral functions can be supplied in trans by the packaging cell line. For example, AAV vectors can comprise ITR sequences from the AAV genome which are required for packaging and integration into the host genome. Viral DNA can be packaged in a cell line, which can contain a helper plasmid encoding other AAV genes, namely rep and cap, while lacking ITR sequences. The cell line can also be infected with adenovirus as a helper. The helper virus can promote replication of the AAV vector and expression of AAV genes from the helper plasmid. Contamination with adenovirus can be reduced by, e.g., heat treatment to which adenovirus is more sensitive than AAV. Additional methods for the delivery of nucleic acids to cells can be used, for example, as described in US20030087817, incorporated herein by reference.

A host cell can be transiently or non-transiently transfected with one or more vectors described herein. A cell can be transfected as it naturally occurs in a subject. A cell can be taken or derived from a subject and transfected. A cell can be derived from cells taken from a subject, such as a cell line. In some embodiments, a cell transfected with one or more vectors described herein is used to establish a new cell line comprising one or more vector-derived sequences. In some embodiments, a cell transiently transfected with the compositions of the present disclosure (such as by transient transfection of one or more vectors, or transfection with RNA) is used to establish a new cell line comprising cells containing the modification but lacking any other exogenous sequence.

Any suitable vector compatible with the host cell can be used with the methods of the present disclosure. Non-limiting examples of vectors for eukaryotic host cells include pXT1, pSG5 (StratageneTM), pSVK3, pBPV, pMSG, and pSVLSV40 (PharmaciaTM).

Contacting the cells with a composition can occur in any culture media and under any culture conditions that promote the survival of the cells. For example, cells may be suspended in any appropriate nutrient medium that is convenient, such as Iscove's modified DMEM or RPMI 1640, supplemented with fetal calf serum or heat inactivated goat serum (about 5-10%), L-glutamine, a thiol, particularly 2-mercaptoethanol, and antibiotics, e.g. penicillin and streptomycin. The culture may contain growth factors to which the cells are responsive. Growth factors, as defined herein, are molecules capable of promoting survival, growth and/or differentiation of cells, either in culture or in the intact tissue, through specific effects on a transmembrane receptor. Growth factors can include polypeptides and non-polypeptide factors.

In numerous embodiments, the chosen delivery system is targeted to specific tissue or cell types. In some cases, tissue- or cell-targeting of the delivery system is achieved by binding the delivery system to tissue- or cell-specific markers, such as cell surface proteins. Viral and non-viral delivery systems can be customized to target tissue or cell types of interest.

Pharmaceutical compositions containing molecules (e.g., polypeptides and/or nucleic acids encoding polypeptides or proteins) or immune cells described herein can be administered for prophylactic and/or therapeutic treatments. In therapeutic applications, the compositions can be administered to a subject already suffering from a disease or condition, in an amount sufficient to cure or at least partially arrest the symptoms of the disease or condition, or to cure, heal, improve, or ameliorate the condition. Amounts effective for this use can vary based on the severity and course of the disease or condition, previous therapy, the subject's health status, weight, and response to the drugs, and the judgment of the treating physician.

Multiple therapeutic agents can be administered in any order or simultaneously. If simultaneously, the multiple therapeutic agents can be provided in a single, unified form, or in multiple forms, for example, as multiple separate pills or cell solutions. The molecules or cell solutions can be packed together or separately, in a single package or in a plurality of packages. One or all of the therapeutic agents can be given in multiple doses. If not simultaneous, the timing between the multiple doses may vary to as much as about 1 to 24 months.

Molecules or cells described herein can be administered before, during, or after the occurrence of a disease or condition, and the timing of administering the composition containing a compound can vary. For example, the pharmaceutical composition can be used as a prophylactic and can be administered continuously to subjects with a propensity to a condition or disease in order to prevent the occurrence of the disease or condition. The molecules, cells, and pharmaceutical compositions can be administered to the subject during the onset of symptoms or as quickly as possible. Administration of the molecules can be started within the first 48 hours of the onset of symptoms, within the first 24 hours of the onset of symptoms, within the first 6 hours of the onset of symptoms, or within 3 hours of the onset of symptoms. Initial administration can be done by any practical route, for example by any of the routes described herein, using any of the formulations described herein. The molecules can be administered as soon as is practicable after the onset of a disease or condition is detected or suspected, and for a length of time necessary for the treatment of the disease, such as, for example, about 1 month to about 3 months. The length of treatment can vary for each subject.

A molecule can be packaged into a biological compartment. A biological compartment comprising the molecule can be administered to a subject. Biological compartments can include, but are not limited to, viruses (lentivirus and adenovirus), nanospheres, liposomes, quantum dots, nanoparticles, microparticles, nanocapsules, vesicles, polyethylene glycol particles, hydrogels and micelles.

For example, the biological compartment can comprise a liposome. A liposome can be a self-assembling structure comprising one or more lipid bilayers, each of which can comprise two monolayers containing oppositely oriented amphipathic lipid molecules. Amphipathic lipids can comprise a polar (hydrophilic) headgroup covalently linked to one or two or more non-polar (hydrophobic) acyl or alkyl chains. Energetically unfavorable contacts between the hydrophobic acyl chains and a surrounding aqueous medium induce amphipathic lipid molecules to arrange themselves such that polar headgroups can be oriented towards the bilayer's surface and acyl chains are oriented towards the interior of the bilayer, effectively shielding the acyl chains from contact with the aqueous environment.

Examples of preferred amphipathic compounds used in liposomes can include phosphoglycerides and sphingolipids, representative examples of which include phosphatidylcholine, phosphatidylethanolamine, phosphatidylserine, phosphatidylinositol, phosphatidic acid, phoasphatidylglycerol, palmitoyloleoyl phosphatidylcholine, lysophosphatidylcholine, lysophosphatidylethanolamine, dimyristoylphosphatidylcholine (DMPC), dipalmitoylphosphatidylcholine (DPPC), dioleoylphosphatidylcholine, distearoylphosphatidylcholine (DSPC), dilinoleoylphosphatidylcholine and egg sphingomyelin, or any combination thereof.

The biological compartment can comprise a nanoparticle. The nanoparticle can comprise a diameter of about 40 nanometers to about 1.5 micrometers, about 50 nanometers to about 1.2 micrometers, about 60 nanometers to about 1 micrometer, about 70 nanometers to about 800 nanometers, about 80 nanometers to about 600 nanometers, about 90 nanometers to about 400 nanometers, or about 100 nanometers to about 200 nanometers.

In some instances, as the size of the nanoparticle increases, the release rate can be slowed or prolonged and as the size of the nanoparticle decreases, the release rate can be increased.

The amount of albumin in the nanoparticle can range from about 5% to about 85% albumin (v/v), from about 10% to about 80%, from about 15% to about 80%, from about 20% to about 70% albumin (v/v), from about 25% to about 60%, from about 30% to about 50%, or from about 35% to about 40%. The pharmaceutical composition can comprise up to 30, 40, 50, 60, 70 or 80% or more of the nanoparticle. In some instances, the nucleic acid molecules of the present disclosure can be bound to the surface of the nanoparticle.

The biological compartment can comprise a virus. The virus can be a delivery system for the pharmaceutical composition of the present disclosure. Exemplary viruses can include lentivirus, retrovirus, adenovirus, herpes simplex virus I or II, parvovirus, reticuloendotheliosis virus, and adeno-associated virus (AAV). Pharmaceutical compositions of the present disclosure can be delivered to a cell using a virus. The virus can infect and transduce the cell *in vivo*, *ex vivo*, or *in vitro.* In *ex vivo* and *in vitro* delivery, the transduced cells can be administered to a subject in need of therapy.

Pharmaceutical compositions can be packaged into viral delivery systems. For example, the compositions can be packaged into virions by a HSV-1 helper virus-free packaging system.

Viral delivery systems (e.g., viruses comprising the pharmaceutical compositions of the present disclosure) can be administered by direct injection, stereotaxic injection, intracerebroventricularly, by minipump infusion systems, by convection, catheters, intravenous, parenteral, intraperitoneal, and/or subcutaenous injection, to a cell, tissue, or organ of a subject in need. In some instances, cells can be transduced *in vitro* or *ex vivo* with viral delivery systems. The transduced cells can be administered to a subject having a disease. For example, a stem cell can be transduced with a viral delivery system comprising a pharmaceutical composition and the stem cell can be implanted in the patient to treat a disease.

In some instances, the dose of cells given to the subject can be less than 1×10⁴ cells/kg, or about 1×10⁴ cells/kg, about 2×10⁴ cells/kg, about 3×10⁴ cells/kg, about 4×10⁴ cells/kg, about 5×10⁴ cells/kg, about 6×10⁴ cells/kg, about 7×10⁴ cells/kg, about 8×10⁴ cells/kg, about 9×10⁴ cells, about 1×10⁵ cells/kg, about 2×10⁵ cells/kg, about 3×10⁵ cells/kg, about 4×10⁵ cells/kg, about 5×10⁵ cells/kg, about 6×10⁵ cells/kg, about 7×10⁵ cells/kg, about 8×10⁵ cells/kg, about 9×10⁵ cells, about 1×10⁶ cells/kg, about 2×10⁶ cells/kg, about 3×10⁶ cells/kg, about 4×10⁶ cells/kg, about 5×10⁶ cells/kg, about 6×10⁶ cells/kg, about 7×10⁶ cells/kg, about 8×10⁶ cells/kg, about 9×10⁶ cells, about 1×10⁷ cells/kg, about 5×10⁷ cells/kg, about 1×10⁸ cells/kg or more. The cell dose here can be calculated according to the number of effectively modified cells that are successfully transfected, or according to the total number of cells.

Introduction of the biological compartments into cells can occur by viral or bacteriophage infection, transfection, conjugation, protoplast fusion, lipofection, electroporation, calcium phosphate precipitation, polyethyleneimine (PEI)-mediated transfection, DEAE-dextran-mediated transfection, liposome-mediated transfection, particle gun technology, calcium phosphate precipitation, direct micro-injection, nanoparticle-mediated nucleic acid delivery, etc.

In some embodiments, immune cells expressing a subject system are administered. Immune cells expressing a subject system can be administered before, during, or after the occurrence of a disease or condition, and the timing of administering the immune cells can vary. For example, immune cells expressing a subject system can be used as a prophylactic and can be administered continuously to subjects with a propensity to a condition or disease in order to prevent the occurrence of the disease or condition. The immune cells can be administered to the subject during the onset of symptoms or as quickly as possible. Administration can be started within the first 48 hours of the onset of symptoms, within the first 24 hours of the onset of symptoms, within the first 6 hours of the onset of symptoms, or within 3 hours of the onset of symptoms. Initial administration can be done by any suitable route, for example by any of the routes described herein, using any of the formulations described herein. The immune cells can be administered as soon as is practicable after the onset of a disease or condition is detected or suspected, and for a length of time necessary for the treatment of the disease, such as, for example, about 1 month to about 3 months. The length of treatment can vary for each subject.

The molecule described herein (e.g., polypeptide and/or nucleic acid) can be present in the composition in a range of from about 1 mg to about 2000 mg; from about 5 mg to about 1000 mg, from about 10 mg to about 25 mg to 500 mg, from about 50 mg to about 250 mg, from about 100 mg to about 200 mg, from about 1 mg to about 50 mg, from about 50 mg to about 100 mg, from about 100 mg to about 150 mg, from about 150 mg to about 200 mg, from about 200 mg to about 250 mg, from about 250 mg to about 300 mg, from about 300 mg to about 350 mg, from about 350 mg to about 400 mg, from about 400 mg to about 450 mg, from about 450 mg to about 500 mg, from about 500 mg to about 550 mg, from about 550 mg to about 600 mg, from about 600 mg to about 650 mg, from about 650 mg to about 700 mg, from about 700 mg to about 750 mg, from about 750 mg to about 800 mg, from about 800 mg to about 850 mg, from about 850 mg to about 900 mg, from about 900 mg to about 950 mg, or from about 950 mg to about 1000 mg.

The molecule (e.g., polypeptide and/or nucleic acid) described herein can be present in the composition in an amount of about 1 mg, about 2 mg, about 3 mg, about 4 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 125 mg, about 150 mg, about 175 mg, about 200 mg, about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, or about 2000 mg.

The molecule (e.g., polypeptide and/or nucleic acid) described herein can be present in the composition that provides at least 0.1, 0.5, 1, 1.5, 2, 2.5, 3, 3.5, 4, 4.5, 5, 5.5, 6, 6.5, 10 or more units of activity/mg molecule. The activity can be regulation of gene expression. In some embodiments, the total number of units of activity of the molecule delivered to a subject is at least 25,000, 30,000, 35,000, 40,000, 45,000, 50,000, 60,000, 70,000, 80,000, 90,000, 110,000, 120,000, 130,000, 140,000, 150,000, 160,000, 170,000, 180,000, 190,000, 200,000, 210,000, 220,000, 230,000, or 250,000 or more units. In some embodiments, the total number of units of activity of the molecule delivered to a subject is at most 25,000, 30,000, 35,000, 40,000, 45,000, 50,000, 60,000, 70,000, 80,000, 90,000, 110,000, 120,000, 130,000, 140,000, 150,000, 160,000, 170,000, 180,000, 190,000, 200,000, 210,000, 220,000, 230,000, or 250,000 or more units.

### Brief Description of the Drawings

Fig. 1 shows an *in vitro* experiment comparing the improvement on T cell function (IFNγ secretion) of various enhanced receptors.
Fig. 2 shows an *in vitro* experiment comparing the improvement on T cell function (IL2 secretion) of various enhanced receptors.
Fig. 3 shows an animal experiment comparing the improvement on tumor inhibition by T cells of various enhanced receptors.
The present invention further relates to the following items:
1. An enhanced receptor for improving the function of an immune cell, wherein the enhanced receptor is a transmembrane protein expressed on the surface of the immune cell, and consists of an extracellular domain (ECD) and an intracellular domain (ICD).
2. The enhanced receptor of item 1, wherein the immune cell is any one of T cells, NK cells, B cells, macrophages, etc.
3. The enhanced receptor of item 1, wherein the intracellular domain (ICD) comprises a costimulatory molecule that elicits an immune cell activating signal, and is capable of eliciting an immune cell activation signal.
4. The enhanced receptor of item 1, wherein the intracellular domain (ICD) is a costimulatory molecule, such as any one of CD28 or 41BBz or ICOS.
5. The enhanced receptor of item 1, wherein the extracellular domain (ECD) is capable of binding to a target cell of the immune cell; is a receptor, a ligand, or an antibody of a membrane protein of the target cell of the immune cell; or is a complete sequence structure of any substance that is capable of binding to the target cell, or a portion comprising a binding domain thereof.
6. The enhanced receptor of item 1, wherein the extracellular domain (ECD) is any one of PD1, CTLA4, NKG2D, SIRPα, LAG3, or other T cell surface proteins.
7. The enhanced receptor of item 1, wherein the extracellular domain (ECD) is an anti-PDL1 monoclonal antibody, an anti-B7 monoclonal antibody, an anti-HER2 monoclonal antibody, an anti-EGFR monoclonal antibody, an anti-CD47 monoclonal antibody, or other antibodies capable of binding to a membrane protein of the target cell, or a partial structure of said antibodies.
8. The enhanced receptor of item 1, wherein:
   1) the ECD is PD1, and the ICD is CD28 or 41BBz or ICOS, in which the sequences of the corresponding enhanced receptors are as set forth in SEQ ID NO: 1 (PD1/CD28), SEQ ID NO: 2 (PD1/41BBz), and SEQ ID NO: 3 (PD1/ICOS), respectively;
   2) the ECD is an anti-PDL1 monoclonal antibody, and the ICD is CD28 or 41BBz or ICOS, in which the sequences of the corresponding enhanced receptors are as set forth in SEQ ID NO: 4, SEQ ID NO: 5, and SEQ ID NO: 6, respectively;
   3) the ECD is an anti-HER2 monoclonal antibody K4D5-628 or K4D5-728 or K4D5-828, and the ICD is CD28 or 41BBz or ICOS, in which the sequences of the corresponding enhanced receptors are as set forth in SEQ ID NO: 7 (K4D5-628/CD28), SEQ ID NO: 8 (K4D5-728/CD28), SEQ ID NO: 9 (K4D5728/CD28), SEQ ID NO: 10 (K4D5-628/41BBz), SEQ ID NO: 11 (K4D5-728/41BBz), SEQ ID NO: 12 (K4D5728/41BBz), SEQ ID NO: 13 (K4D5-628/ICOS), SEQ ID NO: 14 (K4D5-728/ICOS), and SEQ ID NO: 15 (K4D5728/ICOS), respectively;
   4) the ECD is NKG2D, and the ICD is CD28 or 41BBz or ICOS, in which the sequences of the corresponding enhanced receptors are as set forth in SEQ ID NO: 16 (NKG2D/CD28), SEQ ID NO: 17 (NKG2D/41BBz), and SEQ ID NO: 18 (NKG2D/ICOS), respectively;
   5) the ECD is an anti-CD47 monoclonal antibody, and the ICD is CD28 or 41BBz or ICOS, in which the sequences of the corresponding enhanced receptors are as set forth in SEQ ID NO: 19 (CD47 monoclonal antibody/CD28), SEQ ID NO: 20 (CD47 monoclonal antibody/41BBz), and SEQ ID NO: 21 (CD47 monoclonal antibody/ICOS), respectively; or
   6) the ECD is SIRPα, and the ICD is CD28 or 41BBz or ICOS, in which the sequences of the corresponding enhanced receptors are as set forth in SEQ ID NO: 22 (SIRPα/CD28), SEQ ID NO: 23 (SIRPα/41BBz), and SEQ ID NO: 24 (SIRPα/ICOS), respectively.
9. The enhanced receptor of any one of items 1-8, wherein the target cell of the immune cell is a cell harmful to the human body, such as a tumor cell; or a non-tumor cell, such as a virus, a pathogen, etc.
10. The enhanced receptor of any one of items 1-8, wherein the target cell of the immune cell is a cell harmless or harmless in a short period of time to the human body, such as any one of natural human cells like B cells, T cells, NK cells, macrophages, etc.; or a processed human cell; or a processed or irradiated non-human cell, such as K562, NK92, KHYG-1, etc.
11. A composition, comprising the enhanced receptor of any one of items 1-10, and a T cell receptor (TCR), or a CAR (chimeric antigen receptor), wherein the TCR is a naturally unmodified or artificially modified engineered TCR.
12. An engineered T cell, loaded with the composition of item 11, that is, the T cell expresses a natural TCR or an artificial TCR or a CAR, and transmembrane expresses the enhanced receptor of any one of items 1-10 at the same time.
13. The T cell of item 12, wherein the immune cell activation signal in item 3 is activated by the binding of an ECD of the enhanced receptor of any one of items 1-10 to a membrane protein of a target cell, thereby activating the function of the T cell, so that the T cell is capable of resisting the inhibition of the target cell microenvironment on the T cell.
14. The T cell of item 12 or 13, wherein the inhibitory signal of the target cell microenvironment on the T cell is derived from any one or more of PDL1/2, CTLA4, TIM3, TIGIT, PDGFβ, etc.
15. The T cell of item 12 or 13 or 14, wherein the T cell is a single clone characterized by a natural TCR or an artificial TCR or a CAR capable of recognizing only a single target, or is a mixture of a plurality of T cell clones capable of recognizing multiple targets.
16. The T cell of any one of items 12-15, wherein the T cell is derived from tumor-infiltrating T lymphocytes (TILs), and is positive or negative for one or more markers, such as PD1, TIM3, CD137, CD39, etc.
17. The T cell of any one of items 12-15, wherein the T cell is derived from T cells that are from peripheral blood mononuclear cells (PBMCs) and positive or negative for one or more markers, such as PD1, TIM3, CD137, CD39, etc.
18. The T cell of any one of items 12-17, wherein the T cell has dual recognition functions, that is, the target cell in item 9 is recognized by a first natural TCR or artificial TCR or CAR, and the target cell in item 10 is recognized by a second natural TCR or artificial TCR or CAR at the same time.
19. The T cell of item 18, wherein the T cell has dual recognition functions, that is, on the one hand, the T cell is capable of recognizing and killing the target cell in item 9 to achieve the purpose of disease treatment, and on the other hand, the T cell is capable of being activated by the target cell in item 10 to expand to enhance efficacy.
20. The T cell of any one of items 12-19, wherein in addition to the composition of item 11, the T cell is further loaded with other gene modifications such as a suicide switch.
21. A cell drug for treating a tumor, comprising the T cell of any one of items 12-20.

### Detailed Description of Embodiments

For a more complete understanding and application of the present invention, the present invention will be described in detail hereinafter with reference to the examples and the accompanying drawings, which examples are intended only to illustrate the present invention and are not intended to limit the scope of the present invention. The scope of the present invention is specifically defined by the appended claims.

### Example 1. Related Example of Enhanced Receptor-PD1/CD28

In this example, an enhanced receptor consisting of PD1 as the extracellular segment and CD28 as the intracellular segment, represented by PD1/CD28 or ER (Enhanced Receptor), was constructed and tested.

### Preparation of lentiviral vector for PD1/CD28 enhanced receptor

The lentiviral vector for PD1/CD28 enhanced receptor (or referred to as Switch for short hereinafter) employed a safer fourth-generation lentiviral vector system. The main vector PD1/CD28, packaging vector pMDL-gag, Rev, and envelope vector pMD2.G were co-transfected with calcium phosphate or liposome PEI into 293T cells, the supernatant was collected after 48 hours, and the lentiviruses were concentrated by ultracentrifugation.

Titer detection of the lentiviral vector for PD1/CD28 enhanced receptor: Using 3-fold dilution, 293T cells were infected with 50 ul dilution for 48 hours to 72 hours, 293T cells were collected for PD1 staining, the ratio of PD1+ cells was analysed by flow cytometry, and the titer was calculated according to the following formula: Titer (TU/ml) = 40000-45000 (the number of starting 293T cells) * % PD1+ cells * fold of dilution * 20 (calculated by taking the first ratio of PD1+ cells < 20%).

Similarly, lentiviral vectors comprising the following gene sequences were constructed:
1) non-fusion expression of PD1/CD28 and an artificial TCR targeting tumor target NY-ESO-1, referred to as ER-TCR for short; and
2) non-fusion expression of PD1/CD28 and a CAR targeting CD19, referred to as ER-CAR for short.

The titer of each lentiviral vector was calculated, and those with a titer of > 3×10⁷ can be used for the next step.

### Construction of other groups of lentiviral expression vectors

Similar to the above steps, lentiviral vectors loaded with the following gene sequences were constructed, respectively:
1) an artificial TCR targeting tumor target NY-ESO-1;
2) a CAR targeting CD19;
4) non-fusion expression of PD1/CD28 and an artificial TCR targeting tumor target NY-ESO-1, referred to as ER-TCR for short; and
5) non-fusion expression of PD1/CD28 and a CAR targeting CD19, referred to as ER-CAR for short.

### Preparation of several groups of tumor-recognizing T cells

Four kinds of tumor-recognizing T cells were prepared as follows.

### Preparation of TCR-T cells (NY-ESO-1-TCR-T or TCR-T) targeting tumor target NY-ESO-1

The patient's leukocytes with tumor cells being NY-ESO-1 positive and of HLA-A:0201 type were collected by apheresis, PBMCs were separated with Ficoll lymphocyte separation solution, and allowed to grow adherently for 2 hours, and the suspension cells-mainly T cells-were obtained. TCR lentiviral vectors recognizing NY-ESO-1 were added, lentiviruses were added at MOI (number of lentiviruses/number of cells) = 1, and TCR-T cells were cultured and expanded. The expression rate of TCR was 68.7% by flow cytometry.

At MOI = 1, ER lentiviruses were added to the TCR-T to obtain ER-TCR-T.

### Preparation of CAR-T cells (CD19 CAR-T or CAR-T) targeting CD19 and CAR-T (ER-CAR-T) transfected with enhanced receptor

The patient's leukocytes were collected by apheresis, PBMCs were separated with Ficoll lymphocyte separation solution, and allowed to grow adherently for 2 hours, and the suspension cells-mainly T cells-were obtained. CAR lentiviral vectors recognizing CD19 were added, lentiviruses were added at MOI (number of lentiviruses/number of cells) = 1, and CAR-T cells were cultured and expanded.

At MOI = 1, ER lentiviruses were added to the CAR-T to obtain ER-CAR-T.

### Preparation of TIL cells from fresh tumor tissues and TIL (ER-TIL) transfected with enhanced receptor

Cells of tumor tissues were isolated from 10 g or more of the patient's tumor tissues by enzymatic digestion, CD3-positive T cells-TIL-were separated and purified with CD3 magnetic beads, and the remaining cells were cultured adherently to obtain tumor cells autologous to the patient.

At MOI = 1, ER lentiviruses were added to the TIL to obtain ER-TIL.

### Preparation of neoantigen-reactive T cells and neoT (ER-neoT) transfected with enhanced receptor

Firstly, whole-exomic sequencing or RNA transcriptome sequencing was performed on the patient's peripheral blood or surgically removed tumor tissues, the mutation sites were selected, affinity peptide prediction was made in combination with the HLA typing of the patient, and about 20 mutations were selected as Neoantigen. The Neoantigen was genetically synthesized, and was transcribed into RNA *in vitro.* Again, the patient's peripheral blood was collected by apheresis or directly drawn, PBMCs were isolated and grown adherently for 2 hours. To the adherent mononuclear cells, related cytokines were added to promote the differentiation and maturation of DC cells, and the RNA was transferred into the cells by electroporation. The suspension cells-mainly T cells-were co-cultured with the DC cells transferred by electroporation, CD137+-positive T cells were isolated to obtain neoantigen-reactive T cells (or referred to as neoT for short hereinafter), and the cells were expanded.

At MOI = 1, ER lentiviruses were added to the neoT to obtain ER-neoT.

### In vitro functional experiment of PD1/CD28 enhanced receptor in TCR-T targeting NY-ESO-1

In order to verify the role of PD1/CD28 enhanced receptor in the TCR-T targeting NY-ESO-1, a tumor cell line, J82-NY-ESO-1-PDL1, with its HLA typing as A:0201 was firstly constructed. Lentiviruses carrying PDL1 and NY-ESO-1 were added to J82 cells at MOI = 5. After 72 h, G418 and Puromycin were added to screen positive cells. After about 2 weeks, the expression of PDL1 and NY-ESO-1 was detected by flow cytometry. The flow cytometry results showed that up to 95% of J82 cells expressed PDL1 and NY-ESO-1 at the same time, indicating that the cell construction was successful.

In order to verify the function of ER, the expression of CD107a and cytokine secretion of T cells were detected by co-culturing tumor cells and T cells. As shown in Fig. 6a, the expression of CD107a in the ER-TCR-T cells was higher than that in ordinary TCR-T. The secretion of IFN-γ and IL-2 shown in the ER-TCR-T was also higher than that in TCR-T. The results of the *in vitro* experiment showed that the ability of TCR-T to kill tumors is significantly improved after transfection with ER.

### In vitro functional experiment of TIL expressing PD1/CD28 enhanced receptor

In order to verify the role of Switch in the TIL, tumor cells isolated from the patient's fresh tumor tissues were co-cultured with the TIL and cells for 24 hours, respectively. The secretion of IFN-γ and IL-2 was detected by ELISA. The results showed that the killing ability of the ER-TIL was significantly higher than that of TIL not transfected with the enhanced receptor. The results of the *in vitro* experiment showed that the ability of TIL to kill tumors is significantly improved after transfection with ER.

### In vitro functional experiment of neoantigen-reactive T cells expressing PD1/CD28 enhanced receptor

In order to verify the role of Switch in the neoT, tumor cells isolated from the patient's fresh tumor tissues were co-cultured with the neoT and Switch-neoT cells for 24 hours, respectively. The secretion of IFN-γ and IL-2 was detected by ELISA. The results showed that the killing ability of the ER-neoT was significantly higher than that of neoT not transfected with the enhanced receptor. The results of the *in vitro* experiment showed that the ability of neoT to kill tumors is significantly improved after transfection with ER.

### In vitro functional experiment of CAR-T cells expressing PD1/CD28 enhanced receptor

A tumor cell line expressing CD19 was firstly constructed. The tumor cells were co-cultured with ordinary CD19CAR-T cells not transfected with the enhanced receptor and the ER-CAR-T for 24 hours, respectively. The results of the secretion of IFN-γ and IL-2 by ELISA showed that the killing ability of the ER-CAR-T is significantly higher than that of ordinary CAR-T not transfected with the enhanced receptor.

### Animal experiment of NY-ESO-1 TCR-T cells expressing PD1/CD28 enhanced receptor

Inoculation: 1×10⁶ J82-NY-ESO-1-PDL1 tumor cells were subcutaneously inoculated into NSG mice. The blank control group was subcutaneously injected with PBS (A0). The mice injected with tumor cells began to form tumors about two weeks later. The tumor size was measured on Day 23, and 30 mice were selected for recruitment.

Administration: The blank control group (A0) was injected with PBS via tail vein. The mice inoculated with tumor cells were divided into 5 groups: no drug-PBS group (A1); T cell group (A2); ER-T cell group (A3); TCR-T cell group (A4); and 5. ER-TCR-T cell group (A5), respectively. The administrations were all performed by intravenous infusion of 1×10⁷ cells via tail vein.

After administration, the tumor size was measured every 2-3 days and the mouse state was observed. Tumor volume = 1/2 * long diameter * short diameter * short diameter. The observation was continued for 30 days.

### Change in tumor burden:

The tumor volume of tumor-bearing mice continued to increase one week after administration. On Day 7-12, the tumors in A4 and A5 groups were detected to begin to shrink, but the tumors in A4 group began to rebound on Day 15-25, while the tumors in A5 group continued to shrink to almost disappear. The other three groups of tumors maintained natural growth.

### Change in total amount of infused T cells in vivo:

In the three groups of administration, on Day 10 after administration, one mouse was randomly selected from groups 3, 4 and 5, tumor tissues were removed, T cells (i.e., TIL) were isolated therefrom and counted, and the TIL density, that is, the total number of TIL per unit weight of tumor tissue, was calculated. The results showed that the TIL density in the tumors of A5 group was far higher than that of other groups.

### Animal experiment of CAR-T cells expressing PD1/CD28 enhanced receptor

Inoculation: 1×10⁶ CD19-expressing tumor cells were subcutaneously inoculated into NSG mice. The blank control group was subcutaneously injected with PBS (A0). The mice injected with tumor cells began to form tumors about two weeks later. The tumor size was measured on Day 23, and 30 mice were selected for recruitment.

Administration: The blank control group (A0) was injected with PBS via tail vein. The mice inoculated with tumor cells were divided into 5 groups: no drug-PBS group (A1); T cell group (A2); ER-T cell group (A3); CAR-T cell group (A4); and ER-CAR-T cell group (A5), respectively. The administrations were all performed by intravenous infusion of 1×10⁷ cells via tail vein.

After administration, the tumor size was measured every 2-3 days and the mouse state was observed. Tumor volume = 1/2 * long diameter * short diameter * short diameter. The observation was continued for 30 days.

### Change in tumor burden:

The tumor volume of tumor-bearing mice in each group continued to increase one week after administration. The trend of A1-3 groups was basically the same, and the tumor volume continued to increase, until death occurred on Day 30-40. The tumors in A4 and A5 groups were detected to begin to shrink from Week 2, but the tumors in A4 group began to rebound in Week 3-4, while the tumors in A5 group continued to shrink to almost disappear.

### Change in total amount of infused T cells in vivo:

In the three groups of administration, on Day 10 after administration, one mouse was randomly selected from groups 3, 4 and 5, tumor tissues were removed, T cells (i.e., TIL) were isolated therefrom and counted, and the TIL density, that is, the total number of TIL per unit weight of tumor tissue, was calculated. The results showed that the TIL density in the tumors of A5 group was far higher than that of other groups.

### Example 2. Related Example of Enhanced Receptor-PD1/41BBz

In this example, an enhanced receptor consisting of PD1 as the extracellular segment and 41BBz as the intracellular segment, represented by PD1/41BBz or ER (Enhanced Receptor), was constructed and tested. The difference between the enhanced receptor of this example and the enhanced receptor of Example 1 was that the costimulatory molecules of the intracellular segment were different.

The experimental design was the same as that of Example 1, and the only difference was that the enhanced receptors were different.

### In vitro functional experiment of NY-ESO-1-targeting TCR-T expressing PD1/41BBz enhanced receptor

The experimental results showed that the expression of CD107a, and the secretion of IFN-γ and IL-2 in the ER-TCR-T cells were both higher than those in ordinary TCR-T cells not transfected with the enhanced receptor.

### In vitro functional experiment of TIL expressing PD1/41BBz enhanced receptor

The results of the *in vitro* experiment showed that the ability of TIL to kill tumors is significantly improved after transfection with ER.

### In vitro functional experiment of neoantigen-reactive T cells expressing PD1/41BBz enhanced receptor

The results showed that the killing ability of the ER-neoT is significantly higher than that of neoT.

### In vitro functional experiment of CAR-T cells expressing PD1/41BBz enhanced receptor

The results showed that the killing ability of the ER-CAR-T is significantly higher than that of ordinary CAR-T not transfected with the enhanced receptor.

### Animal experiment of NY-ESO-1 TCR-T cells expressing PD1/41BBz enhanced receptor

The experimental design was exactly the same as that of the TCR-T animal experiment of Example 1, and the only difference was that the enhanced receptors used were different. The experimental conclusions were also similar to those of the TCR-T animal experiment of Example 1, that is, in the ER-TCR-T group, the tumors were eliminated more thoroughly than those in the TCR-T group not transfected with the enhanced receptor (reflecting the property of the ER to enhance activation), the TIL density in the tumor tissue was higher than that of other groups (reflecting that the ER can help TCR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

### Animal experiment of CD19 CAR-T cells expressing PD1/41BBz enhanced receptor

The experimental design was exactly the same as that of the CAR-T animal experiment of Example 1, and the only difference was that the enhanced receptors used were different. The experimental conclusions were also similar to those of the CAR-T animal experiment of Example 1, that is, in the ER-CAR-T group, the tumors were eliminated more thoroughly than those in the CAR-T group not transfected with the enhanced receptor (reflecting the property of the ER to enhance activation), the TIL density in the tumor tissue was higher than that of other groups (reflecting that the ER can help CAR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

### Example 3. Related Example of Enhanced Receptor-PD1/ICOS

In this example, an enhanced receptor consisting of PD1 as the extracellular segment and ICOS as the intracellular segment, represented by PD1/ICOS or ER (Enhanced Receptor), was constructed and tested. The difference between the enhanced receptor of this example and the enhanced receptor of Example 1 was that the costimulatory molecules of the intracellular segment were different.

The experimental design was the same as that of Example 1, and the only difference was that the enhanced receptors were different.

In the *in vitro* experiment, the experimental conclusion was also similar to that of Example 1, that is, as compared to natural unmodified TCR-T, CAR-T, TIL and neoT, all of the TCR-T, CAR-T, TIL and neoT expressing the ER showed a stronger killing response to target cells, respectively.

In the animal experiment, the experimental conclusions were also similar to those of Example 1, that is, in the TCR-T and CAR-T groups expressing the ER, the tumors were eliminated more thoroughly than those in the TCR-T and CAR-T groups not transfected with the enhanced receptor (reflecting the property of the ER to enhance activation), the TIL density in the tumor tissue was higher than that of other groups (reflecting that the ER can help TCR-T and CAR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

### Example 4. Related Example of Enhanced Receptor-Anti-HER2 Monoclonal Antibody/CD28

In this example, an enhanced receptor consisting of an anti-HER2 monoclonal antibody (referred to as aHER2mAb for short) as the extracellular segment and CD28 as the intracellular segment was constructed and tested. K4D5 was usually used to represent the antibody Fab segment of HER2. This first enhanced receptor was represented by aHER2mAb/CD28 or K4D5/CD28.

### Amino acid sequences of three anti-HER2 antibodies K4D5 with different affinities

K4D5-6.28, K4D5-7.28 and K4D5-8.28 were three antibody Fab segments that specifically recognize HER2 from low to high affinity. K4D5-7.28 and K4D5-6.28 only differed in the amino acid residue at position 76, while K4D5-8.28 and K4D5-6.28 only differed in the amino acid residue at position 250.

### Construction of three K4D5/CD28 and K4D5/CD28-CD19 CAR lentiviral expression plasmids

Three K4D5/CD28 and K4D5/CD28-CD19 CAR lentiviral expression plasmids were constructed. K4D5/CD28 and CD19 CAR were non-fusion expression.

### Preparation of K4D5/CD28 and K4D5/CD28-CD19 CAR lentiviral vectors

The three K4D5/CD28 and K4D5/CD28-CD19 CAR lentiviral vectors employed a safer fourth-generation lentiviral vector system. The main vector K4D5/CD28 or K4D5/CD28-CD19 CAR, packaging vector pMDL-gag, Rev, and envelope vector pMD2.G in each group were co-transfected with lipofectamin 2000 into 293T cells, the supernatant was collected after 48 hours, and the lentiviruses were concentrated by ultracentrifugation.

Titer detection of the three K4D5/CD28 and K4D5/CD28-CD19 CAR lentiviral vectors: Using 3-fold dilution, 293T cells were infected with 50 ul dilution for 48 hours to 72 hours, 293T cells were collected and labeled and stained with HER2 recombinant protein, the ratio of HER2-positive cells was analysed by flow cytometry, and the titer was calculated according to the following formula:
Titer (TU/ml) = number of starting 293T cells * % HER2⁺ cells * fold of dilution * 20 (calculated by taking the first ratio of HER2⁺ cells < 20%). Taking K4D5-8.28/CD28 as an example, the titer of each lentiviral vector in each group was calculated, and those with a titer of > 1×10⁷ can be used for the next step.

### Preparation of CAR-T cells transfected with K4D5/CD28 enhanced receptor

30 ml peripheral blood from a healthy individual was collected via vein, PBMCs were separated with Ficoll lymphocyte separation solution, and allowed to grow adherently for 2 hours, and the suspension cells-mainly T cells-were obtained. Three groups of K4D5/CD28-CD19 CAR lentiviral vectors with different affinities were added, respectively; and unmodified CD19 CAR without the enhanced receptor was added as control to another group of T cells, respectively. Lentiviruses were added at MOI (number of lentiviruses/number of cells) = 2, and enhanced CAR-T cells were cultured and expanded.

### Detection of K4D5/CD28 function by OKT3/HER2 protein costimulation

In order to verify the function of K4D5/CD28, T cells were firstly activated by OKT3 and HER2 protein costimulation, and the secretion of cytokines was detected by ELISA. A 96-well plate was co-coated with HER2 protein and OKT3 protein, and then T cells in each group were inoculated into the 96-well plate at a density of 1×10^5 cells/well. After 48 hours of culture, the culture supernatant was collected for ELISA. The results showed that IFN-γ secreted by the three groups of K4D5/CD28-T cells and K4D5/CD28-CAR-T cells with different affinities was far higher than that secreted by CAR-T cells not transfected with the enhanced receptor. According to the comparison among K4D5/CD28-CAR-T cells with different affinities, the stronger the affinity, the more IFN-γ secreted. The same results were obtained for the detection of IL-2. The results of the OKT3/HER2 protein costimulation experiment showed that T cells expressing K4D5/CD28 can effectively respond to the activating signal of CD3. When the signal of CD3 is weak, K4D5 can effectively enhance the activating signal by binding to HER2 protein, thereby further enhancing the function of T cells, and this enhancement effect is positively correlated with the affinity for HER2 protein, that is, the stronger the affinity, the stronger the enhancement effect.

### Detection of activation function of CAR-T cells transfected with K4D5/CD28 enhanced receptor

In order to verify the role of K4D5/CD28 in the CAR-T cells, a tumor cell line, J82-CD19, expressing CD19 was firstly constructed. Lentiviruses carrying CD19 were added to J82 cells at MOI = 5. After 72 h, the expression of CD19 was detected by flow cytometry. The flow cytometry results showed that 90% of J82 cells expressed CD19, indicating that the cell construction was successful.

In order to verify the function of K4D5/CD28, the cytokine secretion of T cells were detected by co-culturing tumor cells and T cells. The results showed that IFN-γ secreted by the three groups of K4D5/CD28-CAR-T cells with different affinities after co-culturing with J82-CD19 tumor cells was each higher than that of CAR-T cells not transfected with the enhanced receptor. According to the comparison among the three groups of K4D5/CD28-CAR-T cells with different affinities, the weaker the affinity, the more IFN-γ secreted. The basically consistent results were obtained for the detection of IL-2. The results of the *in vitro* experiment showed that K4D5/CD28 can effectively enhance the tumor killing effect of CAR-T cells, and this enhancement effect is negatively correlated with the affinity for K4D5.

### Example 5. Related Example of Enhanced Receptor-Anti-HER2 Monoclonal Antibody/41BBz

In this example, an enhanced receptor consisting of an anti-HER2 monoclonal antibody (referred to as aHER2mAb for short) as the extracellular segment and 41BBz as the intracellular segment was constructed and tested. K4D5 was usually used to represent the antibody Fab segment of HER2. In this example, the enhanced receptor was represented by aHER2mAb/41BBZ or K4D5/41BBZ or ER (Enhanced Receptor). The difference between the enhanced receptor of this example and the enhanced receptor of Example 3 was that the costimulatory molecules of the intracellular segment were different.

### Amino acid sequences of three anti-HER2 antibodies K4D5 with different affinities

See Example 3.

### Construction of three K4D5/41BBZ and K4D5/41BBZ-CD19 CAR lentiviral expression plasmids

Three K4D5/41BBZ and K4D5/41BBZ-CD19 CAR lentiviral expression plasmids were constructed. K4D5/41BBZ and CD19 CAR were non-fusion expression.

### Preparation of K4D5/41BBZ and K4D5/41BBZ-CD19 CAR lentiviral vectors

See Example 3 for the preparation process.

### Preparation of CAR-T cells transfected with K4D5/41BBZ enhanced receptor

See Example 3 for the preparation process.

### Detection of K4D5/41BBZ function by OKT3/HER2 protein costimulation

See Example 3 for the experimental design. The experimental results showed that T cells expressing K4D5/41BBZ can effectively respond to the activating signal of CD3. When the signal of CD3 is weak, K4D5 can effectively enhance the activating signal by binding to HER2 protein, thereby further enhancing the function of T cells, and this enhancement effect is positively correlated with the affinity for HER2 protein, that is, the stronger the affinity, the stronger the enhancement effect.

### Detection of activation function of CAR-T cells transfected with K4D5/41BBZ enhanced receptor

See Example 3 for the experimental design. The results of the *in vitro* experiment showed that K4D5/41BBZ can effectively enhance the tumor killing effect of CAR-T cells, and this enhancement effect is negatively correlated with the affinity for K4D5.

### Example 6. Related Example of Enhanced Receptor-Anti-HER2 Monoclonal Antibody/ICOS

In this example, an enhanced receptor consisting of an anti-HER2 monoclonal antibody (referred to as aHER2mAb for short) as the extracellular segment and ICOS as the intracellular segment was constructed and tested. K4D5 was used to represent the antibody Fab segment of HER2. In this example, the enhanced receptor was represented by aHER2mAb/ICOS or K4D5/ICOS or ER (Enhanced Receptor). The difference between the enhanced receptor of this example and the enhanced receptor of Example 3 was that the costimulatory molecules of the intracellular segment were different.

The experimental design was the same as that of Example 3, and the only difference was that the enhanced receptors were different.

In the *in vitro* experiment, the experimental conclusion was also similar to that of Example 1, that is, as compared to natural unmodified CAR-T, the CAR-T expressing the ER showed a stronger killing response to target cells.

In the animal experiment, the experimental conclusions were also similar to those of Example 1, that is, in the CAR-T group expressing the ER, the tumors were eliminated more thoroughly than those in the CAR-T group not transfected with the enhanced receptor (reflecting the property of the ER to enhance activation), the TIL density in the tumor tissue was higher than that of other groups (reflecting that the ER can help CAR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

### Example 7. Related Example of Enhanced Receptor-Anti-PDL1 Monoclonal Antibody/CD28

In this example, an enhanced receptor consisting of an anti-PDL1 monoclonal antibody (referred to as aPDLlmAb for short) as the extracellular segment and CD28 as the intracellular segment was constructed and tested. In this example, the enhanced receptor was represented by aPDL1mAb/CD28 or ER (Enhanced Receptor). Moreover, aPDLlmAb protein transmembrane expressed was used as a control for the enhanced receptor.

### Preparation of lentiviral vectors for aPDL1mAb/CD28 enhanced receptor and transmembrane aPDL1mAb

1. Lentiviral vectors for transmembrane expressed aPDLlmAb and without CD28 were constructed.
2. Lentiviral vectors for aPDL1mAb/CD28 enhanced receptor were constructed. See Example 1 for the construction process.

### Construction of other groups of lentiviral expression vectors

Similar to the above steps, lentiviral vectors loaded with the following gene sequences were constructed, respectively:
1) an artificial TCR targeting tumor target NY-ESO-1;
2) a CAR targeting CD19;
4) non-fusion expression of PD1/CD28 and an artificial TCR targeting tumor target NY-ESO-1, referred to as ER-TCR for short; and
5) non-fusion expression of PD1/CD28 and a CAR targeting CD19, referred to as ER-CAR for short.

### Preparation of several groups of tumor-recognizing T cells

Four kinds of tumor-recognizing T cells were prepared as follows.

### Preparation of TCR-T cells (NY-ESO-1-TCR-T or TCR-T) targeting tumor target NY-ESO-1

The patient's leukocytes with tumor cells being NY-ESO-1 positive and of HLA-A:0201 type were collected by apheresis, PBMCs were separated with Ficoll lymphocyte separation solution, and allowed to grow adherently for 2 hours, and the suspension cells-mainly T cells-were obtained. TCR lentiviral vectors recognizing NY-ESO-1 were added, lentiviruses were added at MOI (number of lentiviruses/number of cells) = 1, and TCR-T cells were cultured and expanded. The expression rate of TCR was 68.7% by flow cytometry.

At MOI = 1, ER lentiviruses were added to the TCR-T to obtain ER-TCR-T.

### Preparation of CAR-T cells (CD19 CAR-T or CAR-T) targeting CD19 and CAR-T (ER-CAR-T) transfected with enhanced receptor

The patient's leukocytes were collected by apheresis, PBMCs were separated with Ficoll lymphocyte separation solution, and allowed to grow adherently for 2 hours, and the suspension cells-mainly T cells-were obtained. CAR lentiviral vectors recognizing CD19 were added, lentiviruses were added at MOI (number of lentiviruses/number of cells) = 1, and CAR-T cells were cultured and expanded.

At MOI = 1, ER lentiviruses were added to the CAR-T to obtain ER-CAR-T.

### Preparation of TIL cells from fresh tumor tissues and TIL (ER-TIL) transfected with enhanced receptor

Cells of tumor tissues were isolated from 10 g or more of the patient's tumor tissues by enzymatic digestion, CD3-positive T cells-TIL-were separated and purified with CD3 magnetic beads, and the remaining cells were cultured adherently to obtain tumor cells autologous to the patient.

At MOI = 1, ER lentiviruses were added to the TIL to obtain ER-TIL.

### Preparation of neoantigen-reactive T cells and neoT (ER-neoT) transfected with enhanced receptor

Firstly, whole-exomic sequencing or RNA transcriptome sequencing was performed on the patient's peripheral blood or surgically removed tumor tissues, the mutation sites were selected, affinity peptide prediction was made in combination with the HLA typing of the patient, and about 20 mutations were selected as Neoantigen. The Neoantigen was genetically synthesized, and was transcribed into RNA *in vitro.* Again, the patient's peripheral blood was collected by apheresis or directly drawn, PBMCs were isolated and grown adherently for 2 hours. To the adherent mononuclear cells, related cytokines were added to promote the differentiation and maturation of DC cells, and the RNA was transferred into the cells by electroporation. The suspension cells-mainly T cells-were co-cultured with the DC cells transferred by electroporation, CD137+-positive T cells were isolated to obtain neoantigen-reactive T cells (or referred to as neoT for short hereinafter), and the cells were expanded.

At MOI = 1, ER lentiviruses were added to the neoT to obtain ER-neoT.

### In vitro functional experiment of NY-ESO-1-targeting TCR-T expressing aPDL1mAb/CD28 enhanced receptor

### 1) ELISA plate experiment

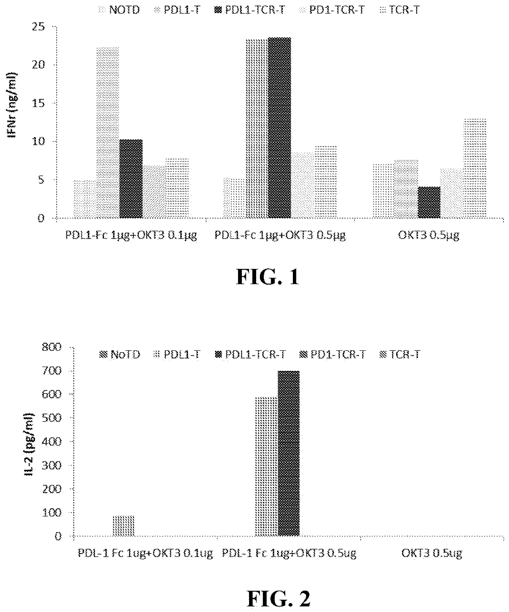

In order to verify the role of aPDL1mAb/CD28 enhanced receptor in ordinary T cells and NY-ESO-1-targeting TCR-T, ELISA plates respectively coated with 0.5 ug OKT3 (an anti-CD3 antibody) protein, 1 ug PDL1-Fc segment + 0.5 ug OKT3 protein, and 1 ug PDL1-Fc segment + 0.1 ug OKT3 protein were firstly prepared. Then, the following T cells were respectively added, and IFNγ (Fig. 1) and IL2 (Fig. 2) were detected after 48 hours:
NOTD, referring to ordinary T cells that were not engineered;
PDL1-T, referring to T cells with aPDL1mAb-CD28;
PD1-T, referring to T cells with PD1-CD28;
PDL1-TCR-T, referring to TCR-T cells with aPDL1mAb-CD28; and
PD1-TCR-T, referring to TCR-T cells with PD1-CD28,
wherein PDL1 referred to aPDL1mAb-CD28; and PD1 referred to PD1-CD28.

As can be seen in Figs. 1 and 2, after encountering PDL1 protein (both 1 ug PDL1-Fc segment + 0.5 ug OKT3 protein, and 1 ug PDL1-Fc segment + 0.1 ug OKT3 protein groups) that inhibited T cell function, IFNγ and IL2 secreted by the T cells and TCR-T cells with aPDLlmAb enhanced receptor were both higher than those secreted by T cells and TCR-T cells without aPDLlmAb enhanced receptor.

### 2) Cell experiment

In order to verify the role of aPDL1mAb/CD28 enhanced receptor in the TCR-T targeting NY-ESO-1, a tumor cell line, J82-NY-ESO-1-PDL1, with its HLA typing as A:0201 was firstly constructed. Lentiviruses carrying PDL1 and NY-ESO-1 were added to J82 cells at MOI = 5. After 72 h, G418 and Puromycin were added to screen positive cells. After about 2 weeks, the expression of PDL1 and NY-ESO-1 was detected by flow cytometry. The flow cytometry results showed that most J82 cells expressed PDL1 and NY-ESO-1 at the same time, indicating that the cell construction was successful.

In order to verify the function of ER, the expression of CD107a and cytokine secretion of T cells were detected by co-culturing tumor cells and T cells. The results showed that the expression of CD107a in the ER-TCR-T cells was higher than that in ordinary TCR-T. The secretion of IFN-γ and IL-2 shown in the ER-TCR-T was also higher than that in TCR-T. The results of the *in vitro* experiment showed that the ability of TCR-T to kill tumors is significantly improved after transfection with ER switch.

In order to compare the function of the transmembrane aPDL1mAb, the expression of CD107a and cytokine secretion of T cells were detected by co-culturing tumor cells and T cells. The results showed that the expression of CD107a, IFN-γ and IL-2 in the aPDL1mAb-NY-ESO-1-TCR-T cells was similar to that in ordinary TCR-T, neither promoting activation nor inhibiting activity.

### In vitro functional experiment of TIL expressing aPDL1mAb/CD28 enhanced receptor

In order to verify the role of ER in the TIL, tumor cells isolated from the patient's fresh tumor tissues were co-cultured with the TIL and cells for 24 hours, respectively. The secretion of IFN-γ and IL-2 was detected by ELISA. The experimental results showed that the killing ability of the ER-TIL was significantly higher than that of TIL. The results of the *in vitro* experiment showed that the ability of TIL to kill tumors is significantly improved after transfection with ER switch.

In order to compare the function of the transmembrane aPDL1mAb, the expression of CD107a and cytokine secretion of T cells were detected by co-culturing tumor cells and T cells. The results showed that the expression of CD107a, IFN-γ and IL-2 in the aPDL1mAb-TIL cells was similar to that in ordinary TIL, neither promoting activation nor inhibiting activity.

### In vitro functional experiment of neoantigen-reactive T cells expressing aPDL1mAb/CD28 enhanced receptor

In order to verify the role of ER in the neoT, tumor cells isolated from the patient's fresh tumor tissues were co-cultured with the neoT and ER-neoT cells for 24 hours, respectively. The secretion of IFN-γ and IL-2 was detected by ELISA. The results showed that the killing ability of the ER-neoT was significantly higher than that of neoT. The results of the *in vitro* experiment showed that the ability of neoT to kill tumors is significantly improved after transfection with ER switch.

In order to compare the function of the transmembrane aPDL1mAb, the expression of CD107a and cytokine secretion of T cells were detected by co-culturing tumor cells and T cells. The results showed that the expression of CD107a, IFN-γ and IL-2 in the aPDL1mAb-neoT cells was similar to that in ordinary neoT, neither promoting activation nor inhibiting activity.

### In vitro functional experiment of CAR-T cells expressing aPDL1mAb/CD28 enhanced receptor

A tumor cell line expressing CD19 was firstly constructed. The tumor cells were co-cultured with ordinary CD19CAR-T cells not transfected with the enhanced receptor and the ER-CAR-T for 24 hours, respectively. The results of the secretion of IFN-γ and IL-2 by ELISA showed that the killing ability of the ER-CAR-T is significantly higher than that of ordinary CAR-T not transfected with the enhanced receptor.

In order to compare the function of the transmembrane aPDL1mAb, the expression of CD107a and cytokine secretion of T cells were detected by co-culturing tumor cells and T cells. The results showed that the expression of CD107a, IFN-γ and IL-2 in the aPDL1mAb-CAR-T cells was similar to that in ordinary CAR-T, neither promoting activation nor inhibiting activity.

### Animal experiment of NY-ESO-1 TCR-T cells expressing aPDL1mAb/CD28 enhanced receptor

Inoculation: 1×10⁶ J82-NY-ESO-1-PDL1 tumor cells were subcutaneously inoculated into NSG mice. The blank control group was subcutaneously injected with PBS (A0). The mice injected with tumor cells began to form tumors about two weeks later. The tumor size was measured on Day 23, and 30 mice were selected for recruitment.

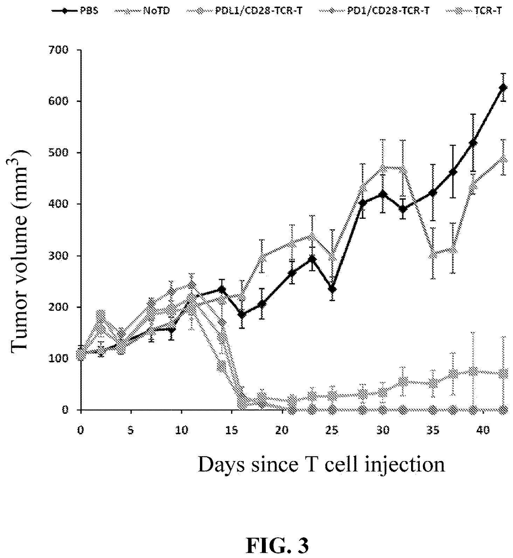

The results of the animal experiment of the combination of ER and TCR-T are shown in Fig. 3. In the legend, PDL1/CD28 referred to aPDL1mAb-CD28 enhanced receptor; and PD1/CD28 referred to PD1/CD28 enhanced receptor.

Administration: The blank control group (A0) was injected with PBS via tail vein. The mice inoculated with tumor cells were divided into 5 groups: no drug-PBS group (A1); non-engineered ordinary T cell group (NOTD, A2); TCR-T cell group (A3); ER-TCRT cell group (A4); and PD1/CD28-TCRT group (A5), respectively. The administrations were all performed by intravenous infusion of 1×10⁷ cells via tail vein.

After administration, the tumor size was measured every 2-3 days and the mouse state was observed. Tumor volume = 1/2 * long diameter * short diameter * short diameter. The observation was continued for 30 days.

### Change in tumor burden:

The tumor volume of tumor-bearing mice in each group continued to increase one week after administration. The trend of A1-2 groups was basically the same, and the tumor volume continued to increase, until death occurred on Day 40. The tumors in A3, A4 and A5 groups were detected to begin to shrink from Day 10, but the tumors in A3 group began to rebound on Day 15, while the tumors in A4 and A5 groups continued to shrink to almost disappear.

It can be seen that the TCR-T cells with PD1/CD28 or aPDL1mAb/CD28 enhanced receptor had significantly enhanced inhibition on tumors than TCR-T cells without the enhanced receptor.

### Change in total amount of infused T cells in vivo:

In the three groups of administration, on Day 10 after administration, one mouse was randomly selected from groups 3, 4 and 5, tumor tissues were removed, and TILs were isolated therefrom. The total amount of cells reinfused was detected by flow cytometry. It can be seen that the total amount of T cells reinfused in the tumors of the A5 group far exceeded that of other groups.

### Animal experiment of CAR-T cells expressing aPDL1mAb/CD28 enhanced receptor

Inoculation: 1×10⁶ CD19- and PDL1-expressing tumor cells were subcutaneously inoculated into NSG mice. The blank control group was subcutaneously injected with PBS (A0). The mice injected with tumor cells began to form tumors about two weeks later. The tumor size was measured on Day 23, and 30 mice were selected for recruitment.

Administration: The blank control group (A0) was injected with PBS via tail vein. The mice inoculated with tumor cells were divided into 5 groups: no drug-PBS group (A1); T cell group (A2); ER-T cell group (A3); CAR-T cell group (A4); and ER-CAR-T cell group (A5), respectively. The administrations were all performed by intravenous infusion of 1×10⁷ cells via tail vein.

After administration, the tumor size was measured every 2-3 days and the mouse state was observed. Tumor volume = 1/2 * long diameter * short diameter * short diameter. The observation was continued for 30 days.

### Change in tumor burden:

The tumor volume of tumor-bearing mice in each group continued to increase one week after administration. The trend of A1-3 groups was basically the same, and the tumor volume continued to increase, until death occurred on Day 30-40. The tumors in A4 and A5 groups were detected to begin to shrink from Week 2, but the tumors in A4 group began to rebound in Week 3-4, while the tumors in A5 group continued to shrink to almost disappear.

### Change in total amount of infused T cells in vivo:

In the three groups of administration, on Day 10 after administration, one mouse was randomly selected from groups 3, 4 and 5, tumor tissues were removed, T cells (i.e., TIL) were isolated therefrom and counted, and the TIL density, that is, the total number of TIL per unit weight of tumor tissue, was calculated. The results showed that the TIL density in the tumors of A5 group was far higher than that of other groups.

### Example 8. Related Example of Enhanced Receptor-aPDL1mAb/41BBz

In this example, an enhanced receptor consisting of an anti-PDL1 monoclonal antibody (referred to as aPDLlmAb for short) as the extracellular segment and 41BBz as the intracellular segment was constructed and tested. In this example, the enhanced receptor was represented by aPDL1mAb/41BBz or ER (Enhanced Receptor). Moreover, aPDLlmAb protein transmembrane expressed was used as a control for the enhanced receptor. The difference between the enhanced receptor of this example and the enhanced receptor of Example 7 was that the costimulatory molecules of the intracellular segment were different.

The experimental design was the same as that of Example 7, and the only difference was that the enhanced receptors were different.

In the *in vitro* experiment, the experimental conclusion was also similar to that of Example 7, that is, as compared to natural unmodified TCR-T, CAR-T, TIL and neoT, all of the TCR-T, CAR-T, TIL and neoT expressing the ER showed a stronger killing response to target cells, respectively.

In the animal experiment, the experimental conclusions were also similar to those of Example 7, that is, in the TCR-T and CAR-T groups expressing the ER, the tumors were eliminated more thoroughly than those in the TCR-T and CAR-T groups not transfected with the enhanced receptor (reflecting the property of the ER to enhance activation), the TIL density in the tumor tissue was higher than that of other groups (reflecting that the ER can help TCR-T and CAR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

### Example 9. Related Example of Enhanced Receptor-aPDL1mAb/ICOS

In this example, an enhanced receptor consisting of an anti-PDL1 monoclonal antibody (referred to as aPDLlmAb for short) as the extracellular segment and ICOS as the intracellular segment was constructed and tested. In this example, the enhanced receptor was represented by aPDL1mAb/ICOS or ER (Enhanced Receptor). Moreover, aPDLlmAb protein transmembrane expressed was used as a control for the enhanced receptor. The difference between the enhanced receptor of this example and the enhanced receptor of Example 7 was that the costimulatory molecules of the intracellular segment were different.

The experimental design was the same as that of Example 7, and the only difference was that the enhanced receptors were different.

In the *in vitro* experiment, the experimental conclusion was also similar to that of Example 7, that is, as compared to natural unmodified TCR-T, CAR-T, TIL and neoT, all of the TCR-T, CAR-T, TIL and neoT expressing the ER showed a stronger killing response to target cells, respectively.

In the animal experiment, the experimental conclusions were also similar to those of Example 7, that is, in the TCR-T and CAR-T groups expressing the ER, the tumors were eliminated more thoroughly than those in the TCR-T and CAR-T groups not transfected with the enhanced receptor (reflecting the property of the ER to enhance activation), the TIL density in the tumor tissue was higher than that of other groups (reflecting that the ER can help TCR-T and CAR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

### Example 10. Related Example of Enhanced Receptor-NKG2D/CD28

In this example, an enhanced receptor consisting of NKG2D as the extracellular segment and CD28 as the intracellular segment was constructed and tested. In this example, the enhanced receptor was represented by NKG2D/CD28 or ER (Enhanced Receptor). Moreover, NKG2D protein transmembrane expressed was used as a control for the enhanced receptor.

The experimental design was the same as that of Example 7, and the only difference was that the enhanced receptors were different.

In the *in vitro* experiment, the experimental conclusion was also similar to that of Example 7, that is, as compared to natural unmodified TCR-T, CAR-T, TIL and neoT, all of the TCR-T, CAR-T, TIL and neoT expressing the ER showed a stronger killing response to target cells, respectively.

In the animal experiment, the experimental conclusions were also similar to those of Example 7, that is, in the TCR-T and CAR-T groups expressing the ER, the tumors were eliminated more thoroughly than those in the TCR-T and CAR-T groups not transfected with the enhanced receptor (reflecting the property of the ER to enhance activation), the TIL density in the tumor tissue was higher than that of other groups (reflecting that the ER can help TCR-T and CAR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

### Example 11. Related Example of Enhanced Receptor-NKG2D/41BBz

In this example, an enhanced receptor consisting of NKG2D as the extracellular segment and 41BBz as the intracellular segment was constructed and tested. In this example, the enhanced receptor was represented by NKG2D/41BBz or ER (Enhanced Receptor). Moreover, NKG2D protein transmembrane expressed was used as a control for the enhanced receptor. The difference between the enhanced receptor of this example and the enhanced receptor of Example 10 was that the costimulatory molecules of the intracellular segment were different.

The experimental design was the same as that of Example 7, and the only difference was that the enhanced receptors were different.

In the *in vitro* experiment, the experimental conclusion was also similar to that of Example 7, that is, as compared to natural unmodified TCR-T, CAR-T, TIL and neoT, all of the TCR-T, CAR-T, TIL and neoT expressing the ER showed a stronger killing response to target cells, respectively.

In the animal experiment, the experimental conclusions were also similar to those of Example 7, that is, in the TCR-T and CAR-T groups expressing the ER, the tumors were eliminated more thoroughly than those in the TCR-T and CAR-T groups not transfected with the enhanced receptor (reflecting the property of the ER to enhance activation), the TIL density in the tumor tissue was higher than that of other groups (reflecting that the ER can help TCR-T and CAR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

### Example 12. Related Example of Enhanced Receptor-NKG2D/ICOS

In this example, an enhanced receptor consisting of NKG2D as the extracellular segment and ICOS as the intracellular segment was constructed and tested. In this example, the enhanced receptor was represented by NKG2D/ICOS or ER (Enhanced Receptor). Moreover, NKG2D protein transmembrane expressed was used as a control for the enhanced receptor. The difference between the enhanced receptor of this example and the enhanced receptor of Example 10 was that the costimulatory molecules of the intracellular segment were different.

The experimental design was the same as that of Example 7, and the only difference was that the enhanced receptors were different.

In the *in vitro* experiment, the experimental conclusion was also similar to that of Example 7, that is, as compared to natural unmodified TCR-T, CAR-T, TIL and neoT, all of the TCR-T, CAR-T, TIL and neoT expressing the ER showed a stronger killing response to target cells, respectively.

In the animal experiment, the experimental conclusions were also similar to those of Example 7, that is, in the TCR-T and CAR-T groups expressing the ER, the tumors were eliminated more thoroughly than those in the TCR-T and CAR-T groups not transfected with the enhanced receptor (reflecting the property of the ER to enhance activation), the TIL density in the tumor tissue was higher than that of other groups (reflecting that the ER can help TCR-T and CAR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

### Example 13. Related Example of Enhanced Receptor-Anti-CD47 Monoclonal Antibody/CD28

In this example, an enhanced receptor consisting of an anti-CD47 monoclonal antibody (referred to as aCD47mAb for short) as the extracellular segment and CD28 as the intracellular segment was constructed and tested. In this example, the enhanced receptor was represented by aCD47mAb/CD28 or ER (Enhanced Receptor). Moreover, aCD47mAb protein transmembrane expressed was used as a control for the enhanced receptor.

The experimental design was the same as that of Example 7, and the only difference was that the enhanced receptors were different.

In the *in vitro* experiment, the experimental conclusion was also similar to that of Example 7, that is, as compared to natural unmodified TCR-T, CAR-T, TIL and neoT, all of the TCR-T, CAR-T, TIL and neoT expressing the ER showed a stronger killing response to target cells, respectively.

In the animal experiment, the experimental conclusions were also similar to those of Example 7, that is, in the TCR-T and CAR-T groups expressing the ER, the tumors were eliminated more thoroughly than those in the TCR-T and CAR-T groups not transfected with the enhanced receptor (reflecting the property of the ER to enhance activation), the TIL density in the tumor tissue was higher than that of other groups (reflecting that the ER can help TCR-T and CAR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

### Example 14. Related Example of Enhanced Receptor-aCD47mAb/41BBz

In this example, an enhanced receptor consisting of aCD47mAb as the extracellular segment and 41BBz as the intracellular segment was constructed and tested. In this example, the enhanced receptor was represented by aCD47mAb/41BBz or ER (Enhanced Receptor). Moreover, SIRPα protein transmembrane expressed was used as a control for the enhanced receptor. The difference between the enhanced receptor of this example and the enhanced receptor of Example 13 was that the costimulatory molecules of the intracellular segment were different.

The experimental design was the same as that of Example 7, and the only difference was that the enhanced receptors were different.

In the *in vitro* experiment, the experimental conclusion was also similar to that of Example 7, that is, as compared to natural unmodified TCR-T, CAR-T, TIL and neoT, all of the TCR-T, CAR-T, TIL and neoT expressing the ER showed a stronger killing response to target cells, respectively.

In the animal experiment, the experimental conclusions were also similar to those of Example 7, that is, in the TCR-T and CAR-T groups expressing the ER, the tumors were eliminated more thoroughly than those in the TCR-T and CAR-T groups not transfected with the enhanced receptor (reflecting the property of the ER to enhance activation), the TIL density in the tumor tissue was higher than that of other groups (reflecting that the ER can help TCR-T and CAR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

### Example 15. Related Example of Enhanced Receptor-aCD47mAb/ICOS

In this example, an enhanced receptor consisting of aCD47mAb as the extracellular segment and ICOS as the intracellular segment was constructed and tested. In this example, the enhanced receptor was represented by aCD47mAb/ICOS or ER (Enhanced Receptor). Moreover, SIRPα protein transmembrane expressed was used as a control for the enhanced receptor. The difference between the enhanced receptor of this example and the enhanced receptor of Example 13 was that the costimulatory molecules of the intracellular segment were different.

The experimental design was the same as that of Example 7, and the only difference was that the enhanced receptors were different.

In the *in vitro* experiment, the experimental conclusion was also similar to that of Example 7, that is, as compared to natural unmodified TCR-T, CAR-T, TIL and neoT, all of the TCR-T, CAR-T, TIL and neoT expressing the ER showed a stronger killing response to target cells, respectively.

In the animal experiment, the experimental conclusions were also similar to those of Example 7, that is, in the TCR-T and CAR-T groups expressing the ER, the tumors were eliminated more thoroughly than those in the TCR-T and CAR-T groups not transfected with the enhanced receptor (reflecting the property of the ER to enhance activation), the TIL density in the tumor tissue was higher than that of other groups (reflecting that the ER can help TCR-T and CAR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

### Example 16. Related Example of Enhanced Receptor-SIRPα/CD28

In this example, an enhanced receptor consisting of SIRPα as the extracellular segment and CD28 as the intracellular segment was constructed. In this example, the enhanced receptor was represented by SIRPα/CD28 or ER (Enhanced Receptor). Moreover, SIRPα protein transmembrane expressed was used as a control for the enhanced receptor.

The experimental design was the same as that of Example 7, and the only difference was that the enhanced receptors were different.

In the *in vitro* experiment, the experimental conclusion was also similar to that of Example 7, that is, as compared to natural unmodified TCR-T, CAR-T, TIL and neoT, all of the TCR-T, CAR-T, TIL and neoT expressing the ER showed a stronger killing response to target cells, respectively.

In the animal experiment, the experimental conclusions were also similar to those of Example 7, that is, in the TCR-T and CAR-T groups expressing the ER, the tumors were eliminated more thoroughly than those in the TCR-T and CAR-T groups not transfected with the enhanced receptor (reflecting the property of the ER to enhance activation), the TIL density in the tumor tissue was higher than that of other groups (reflecting that the ER can help TCR-T and CAR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

### Example 17. Related Example of Enhanced Receptor-SIRPα/41BBz

In this example, an enhanced receptor consisting of SIRPα as the extracellular segment and 41BBz as the intracellular segment was constructed and tested. In this example, the enhanced receptor was represented by SIRPα/41BBz or ER (Enhanced Receptor). Moreover, SIRPα protein transmembrane expressed was used as a control for the enhanced receptor. The difference between the enhanced receptor of this example and the enhanced receptor of Example 16 was that the costimulatory molecules of the intracellular segment were different.

The experimental design was the same as that of Example 7, and the only difference was that the enhanced receptors were different.

In the *in vitro* experiment, the experimental conclusion was also similar to that of Example 7, that is, as compared to natural unmodified TCR-T, CAR-T, TIL and neoT, all of the TCR-T, CAR-T, TIL and neoT expressing the ER showed a stronger killing response to target cells, respectively.

In the animal experiment, the experimental conclusions were also similar to those of Example 7, that is, in the TCR-T and CAR-T groups expressing the ER, the tumors were eliminated more thoroughly than those in the TCR-T and CAR-T groups not transfected with the enhanced receptor (reflecting the property of the ER to enhance activation), the TIL density in the tumor tissue was higher than that of other groups (reflecting that the ER can help TCR-T and CAR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

### Example 18. Related Example of Enhanced Receptor-SIRPα/ICOS

In this example, an enhanced receptor consisting of SIRPα as the extracellular segment and ICOS as the intracellular segment was constructed and tested. In this example, the enhanced receptor was represented by SIRPα/ICOS or ER (Enhanced Receptor). Moreover, SIRPα protein transmembrane expressed was used as a control for the enhanced receptor. The difference between the enhanced receptor of this example and the enhanced receptor of Example 16 was that the costimulatory molecules of the intracellular segment were different.

The experimental design was the same as that of Example 7, and the only difference was that the enhanced receptors were different.

In the *in vitro* experiment, the experimental conclusion was also similar to that of Example 7, that is, as compared to natural unmodified TCR-T, CAR-T, TIL and neoT, all of the TCR-T, CAR-T, TIL and neoT expressing the ER showed a stronger killing response to target cells, respectively.

In the animal experiment, the experimental conclusions were also similar to those of Example 7, that is, in the TCR-T and CAR-T groups expressing the ER, the tumors were eliminated more thoroughly than those in the TCR-T and CAR-T groups not transfected with the enhanced receptor (reflecting the property of the ER to enhance activation), the TIL density in the tumor tissue was higher than that of other groups (reflecting that the ER can help TCR-T and CAR-T cells to better recruit into tumors), and the tumors in the non-tumor-recognizing T cell (ER-T) group transfected with the enhanced receptor were not killed (reflecting the safety of the ER, that is, when T cells do not recognize the target cells, the ER will not independently kill PDL1-positive target cells).

## Claims

1. An enhanced receptor for improving the function of an immune cell, wherein the enhanced receptor is a transmembrane protein expressed on the surface of the immune cell, and consists of an extracellular domain (ECD) and an intracellular domain (ICD), wherein the ECD is an anti-PDL1 monoclonal antibody, and the ICD comprises an intracellular domain of a costimulatory molecule that elicits an immune cell activating signal.

2. The enhanced receptor of claim 1, wherein the immune cell is any one of T cells, NK cells, B cells, or macrophages.

3. The enhanced receptor of claim 1, wherein the costimulatory molecule is selected from CD28, 41BBz or ICOS.

4. The enhanced receptor of claim 1, wherein:
1) the costimulatory molecule is CD28, and the sequence of the enhanced receptors is as set forth in SEQ ID NO: 4,
2) the costimulatory molecule is 41BBz, and the sequence of the enhanced receptors is as set forth in SEQ ID NO: 5, or
3) the costimulatory molecule is ICOS, and the sequence of the enhanced receptors is as set forth in SEQ ID NO: 6.

5. The enhanced receptor of any one of claims 1-4, wherein the ECD is capable of binding to the target cell of the immune cell, and the target cell of the immune cel is a cell harmful to the human body, such as a tumor cell; or a non-tumor cell, such as a virus, or a pathogen.

6. The enhanced receptor of any one of claims 1-4, wherein the the ECD is capable of binding to the target cell of the immune cell, and the target cell of the immune cell is a cell harmless or harmless in a short period of time to the human body, such as any one of natural human cells like B cells, T cells, NK cells, macrophages; or a processed human cell; or a processed or irradiated non-human cell, such as K562, NK92, or KHYG-1.

7. A composition, comprising (a) the enhanced receptor of any one of claims 1-6, and (b) a T cell receptor (TCR) or a chimeric antigen receptor (CAR), wherein the TCR is a naturally unmodified TCR or an artificially modified engineered TCR.

8. An engineered T cell, loaded with the composition of claim 7, wherein the T cell expresses a natural TCR or an artificial TCR or a CAR, and transmembranely expresses the enhanced receptor of any one of claims 1-6 at the same time.

9. The engineered T cell of claim 8, wherein the immune cell activating signal is activated by the binding of an ECD of the enhanced receptor of any one of claims 1-6 to a membrane protein of a target cell, thereby activating the function of the T cell, so that the T cell is capable of resisting the inhibition of the target cell microenvironment on the T cell.

10. The engineered T cell of claim 8 or 9, wherein the inhibition of the target cell microenvironment on the T cell is derived from PDL1 or PDL2.

11. The engineered T cell of any one of claims 8-10, wherein the T cell is **characterized in** one or more of the following:
a) the T cell is a single clone **characterized by** a natural TCR or an artificial TCR or a CAR capable of recognizing only a single target, or a mixture of a plurality of T cell clones capable of recognizing multiple targets,
b) the T cell is derived from tumor-infiltrating T lymphocytes (TILs) or T cells that are from peripheral blood mononuclear cells (PBMCs), and positive or negative for one or more markers, such as PD1, TIM3, CD137, and/or CD39,
c) the T cell has dual recognition functions, wherein the first target cell is recognized by a first natural TCR or artificial TCR or CAR, and the second target cell is recognized by a second natural TCR or artificial TCR or CAR at the same time; the first target cell is a cell harmful to the human body, such as a tumor cell, or a non-tumor cell, such as a virus, a pathogen; the second target cell is a cell harmless or harmless in a short period of time to the human body, such as any one of natural human cells like B cells, T cells, NK cells, macrophages; or a processed human cell; or a processed or irradiated non-human cell, such as K562, NK92, KHYG-1.

12. The engineered T cell of claim 11, wherein the T cell has dual recognition functions, that is, on the one hand, the T cell is capable of recognizing and killing the first target cell to achieve the purpose of disease treatment, and on the other hand, the T cell is capable of being activated by the second target cell to expand to enhance efficacy.

13. The engineered T cell of any one of claims 8-12, wherein the engineered T cell is further loaded with other gene modifications such as a suicide switch.

14. A cell drug for treating a tumor, comprising the engineered T cell of any one of claims 8-13.
